# EUROPEAN PATENT APPLICATION

(11) **EP 4 425 181 A1**
(43) Date of publication of application: **04.09.2024**
(21) Application number: 22883018.8
(22) Date of filing: 27.10.2022
(51) Int. Cl.: G01N 33/68, G01N 33/543

(54) **COMPOSITION FOR DETECTING OR MEASURING ANALYTE**

(30) Priority: 27.10.2021 KR 20210144315
(71) Applicant: Bertis Inc, Bundang-gu, Seongnam-si, Gyeonggi-do 13605 (KR)
(72) Inventor: KIM, Sung Soo, Yongin-si Gyeonggi-do 17066 (KR); LEE, Jaehong, Seoul 06776 (KR)
(74) Representative: Müller, Christian Stefan Gerd
(86) International application number: PCT/KR2022/016533
(87) International publication number: WO 2023/075435

(57) **Abstract**

The present disclosure relates to a composition for detecting or measuring an analyte and an analysis method using the composition. In particular, efficiency and performance of sample analysis may be greatly improved through the composition and analysis method of the present disclosure.

## Description

### Technical Field

The present disclosure relates to a composition for detecting or measuring an analyte, a kit comprising the same, and a method for detecting or measuring an analyte using the same.

### Background Art

Methods for detecting or measuring analytes in biological samples include protein chip assay, immunoassay, ligand binding assay, radioimmunoassay, radioimmunodiffusion, Ouchterlony immunodiffusion, rocket immunoelectrophoresis, immunohistochemical staining, complement fixation assay, two-dimensional electrophoresis assay, Western blotting, ELISA (enzyme-linked immunosorbent assay), and mass spectrometry, and methods for quantifying a genetic material include reverse transcription polymerase chain reaction (RT-PCR), competitive RT-PCR, real-time RT-PCR, RNase protection assay (RPA), Northern blotting, and DNA chip assay.

Among them, mass-spectrometry (MS) is an analytical technique that can monitor changes in the concentration of an analyte of interest in a biological sample by selectively separating, detecting and quantifying the analyte based on a specific mass-to-charge ratio (m/z) of the analyte. This type of mass spectrometry is an analytical method with high selectivity and sensitivity that can detect only information about a desired component.

However, in the process of detecting and quantifying substances (such as proteins) consisting of amino acids, even a highly sensitive mass spectrometer cannot analyze trace substances below the detection limit, because amino acids do not have an amplification mechanism. In addition, the speed of analyzing a large number of samples is relatively low.

In addition, in mass spectrometry, when the analyte has a complex three-dimensional structure such as that of a protein, it is fragmented into peptides by a digestion reaction, and only the mass-to-charge ratio (m/z) of a specific peptide among the peptides is measured. In this process, a large number of unnecessary peptides are also absorbed into the analyte, thereby generating noise that reduces the sensitivity.

Therefore, there is a need for a method that reduces the analysis time and increases the convenience of analysis while being capable of quantifying analytes in biological samples with high sensitivity even without the above-described processes.

### DISCLOSURE

### Technical Problem

An object of the present disclosure is to provide a composition for detecting or measuring an analyte and a kit comprising the same.

Another object of the present disclosure is to provide a method for detecting or measuring an analyte.

However, objects to be achieved by the present disclosure are not limited to the objects mentioned above, and other objects not mentioned herein will be clearly understood by those of ordinary skill in the art from the following description.

### Technical Solution

Hereinafter, various embodiments described herein will be described with reference to figures. In the following description, numerous specific details are set forth, such as specific configurations, compositions, and processes, etc., in order to provide a thorough understanding of the present disclosure. However, certain embodiments may be practiced without one or more of these specific details, or in combination with other known methods and configurations. In other instances, known processes and preparation techniques have not been described in particular detail in order to not unnecessarily obscure the present disclosure. Reference throughout this specification to "one embodiment" or "an embodiment" means that a particular feature, configuration, composition, or characteristic described in connection with the embodiment is included in at least one embodiment of the present disclosure. Thus, the appearances of the phrase "in one embodiment" or "an embodiment" in various places throughout this specification are not necessarily referring to the same embodiment of the present disclosure. Additionally, the particular features, configurations, compositions, or characteristics may be combined in any suitable manner in one or more embodiments. Unless otherwise stated in the present disclosure, all the scientific and technical terms used in this specification have the same meanings as commonly understood by those skilled in the technical field to which the present disclosure pertains.

According to one embodiment of the present disclosure, the present disclosure is directed to a composition for detecting or measuring an analyte, the composition comprising a complex compound represented by Formula 1:

[Formula 1] [M]ₙ-L₁-N₁,

wherein
n is an integer ranging from 2 to 100000;
M is a repeatable unit compound;
L₁ is either a direct bond between M and N₁ or a linker; and
N₁ is a first binding moiety that binds directly or indirectly to the analyte.

In the present disclosure, the "analyte" is a substance to be analyzed which is present in a sample or solution. In particular, in the present disclosure, the analyte may be a substance present in a biological sample, and may comprise any one or more selected from the group consisting of proteins, lipoproteins, glycoproteins, DNA, and RNA. However, the analyte may comprise, without limitation, any biomolecule in which organic substances such as amino acids, nucleotides, monosaccharides or lipids are contained as monomers.

In the present disclosure, M is a repeatable unit compound, and is not particularly limited in kind as long as it is a compound that may be detected or measured in place of the analyte. Preferably, M may have a mass-to-charge ratio (m/z) of 30 to 3,000. When the mass-to-charge ratio (m/z) of M is 30 to 3,000, there is an effect that it is easy to analyze M by mass spectrometry.

In the present disclosure, the "unit" or "monomer" is a compound serving as a monomer for synthesizing a polymer, and the kind thereof is not particularly limited. Examples of the monomer include amino acids, amino acid analogs, peptides, peptide analogs, monosaccharides, oligosaccharides, or polysaccharides.

In the present disclosure, the "amino acid" may include, without limitation, any amino acid capable of forming a peptide bond while having a structure in which a basic amino group (-NH₂), an acidic carboxyl group (-COOH) and a side chain (-R group) are bonded to the alpha carbon which is the central carbon. Accordingly, the amino acids include all amino acids derived from organisms or artificially synthesized amino acids, and constituent elements thereof are not limited to carbon, hydrogen, oxygen, nitrogen or sulfur, and may additionally include other elements. The amino acids may include all types of isomers. Among the amino acids, 20 types of amino acids are encoded by the genes of eukaryotes and prokaryotes, but more than 500 types of naturally occurring amino acids are known.

In the present disclosure, the "amino acid analog" may be used instead of an amino acid to crosslink a peptide or protein complex by a peptide bond, and examples thereof include, without limitation, those having an amino group (-NH₂) and a carboxyl group (-COOH) in the molecule.

In the present disclosure, the amino acid may be glycine, alanine, valine, leucine, isoleucine, threonine, serine, cysteine, methionine, aspartic acid, asparagine, glutamic acid, glutamine, lysine, arginine, histidine, phenylalanine, tyrosine, tryptophan, proline, pyrrolysine, theanine, gamma-glutamylmethylamide, beta-aminobutyric acid or gamma-aminobutyric acid; or an isomer thereof. Preferably, the amino acid may be any one or more selected from the group consisting of glycine, alanine, valine, leucine, isoleucine, threonine, serine, cysteine, aspartic acid, asparagine, glutamic acid, glutamine, lysine, arginine, phenylalanine, tyrosine, tryptophan and proline, but is not limited thereto.

Preferably, in Formula 1, n is an integer from 2 to 50000, more preferably an integer from 2 to 10000, even more preferably an integer from 2 to 5000, still more preferably an integer from 2 to 1000 , most preferably an integer from 2 to 100.

According to another embodiment of the present disclosure, in Formula 1, n is an integer from 101 to 100000, preferably from 101 to 50000, more preferably from 101 to 10000, and more preferably from 101 to 5000. is the number of integers, and the most preferably it is an integer from 101 to 1000.

In addition, in the present disclosure, the amino acid analog may be one in which a protecting group is added to a functional group other than the carboxyl group (-COOH) and amino group (NH₂-) of the amino acid, and non-limiting examples thereof can include (Fmoc-Cys-OtBu)2, (H-Cys-OH)2, (H-Cys-OMe)2·2HCl, (H-HoCys-OH)2, (R)-N-Fmoc-2-(7-octenyl)Alanine, (S)-N-Fmoc-α-(4-pentenyl)Alanine, (Z-Cys-OH)2, 3-Cyclopentane-D-Alanine, 3-Methoxy-2-nitropyridine, 5-Ethyltio-1H-Tetrazole, 6-Fmoc-Acp-ol, 8-Aoc-OH·HCl, 9-Fluorenylmethanol, Ac-2-Nal-OH, Ac-Ala-OH, Ac-Ala-OMe, Ac-Arg-OH, Ac-Arg-OH·2H2O, Ac-Asp(OtBu)-OH, Ac-Asp-OH, Ac-Asp-OtBu, Ac-Cys(Me)-OH, Ac-Cys(Trt)-OH, Ac-Cys-OH, Ac-D-2-Nal-OH, Ac-D-Ala-OH, Ac-D-Allo-Ile-OH, Ac-Dap(Boc)-OH, Ac-D-Arg(Pbf)-OH, Ac-D-Arg-OH, Ac-D-Asn(Trt)-OH, Ac-D-Asp(OtBu)-OH, Ac-D-Cys(Trt)-OH, Ac-D-Gln(Trt)-OH, Ac-D-Glu(OtBu)-OH, Ac-D-Glu-OH, Ac-D-His(Trt)-OH, Ac-DL-Abu-OH, Ac-DL-Ala-OH,Ac-D-Phe(2-Br)-OH, Ac-D-Phe(3-F)-OH, Ac-D-Phe(4-Br)-OH, Ac-D-Phe-OH, Ac-D-Pro-OH, Ac-D-Ser(tBu)-OH, Ac-D-Thr(tBu)-OH, Ac-D-Trp(Boc)-OH, Ac-D-Trp-OH, Ac-D-Tyr(tBu)-OH, Ac-D-Val-OH, Ac-Gln-OH, Ac-Gln-OtBu, Ac-Glu(OtBu)-OH, Ac-Gly-Gly-OH, Ac-Gly-OEt, Ac-Gly-OH, Ac-His(Trt)-OH, Ac-His-OH H2O, Ac-HMBA-linker, Ac-HoPhe-OH, Ac-Ile-OH, Ac-Leu-OH, Ac-Lys(Ac)-OH·DCHA, Ac-Lys(Boc)-OH, Ac-Lys(Fmoc)-OH, Ac-Lys(Z)-OH, Ac-Lys-OMe·HCl, Ac-Met-OH, Ac-Nle-OH, Ac-Nva-OH, Ac-Om-OH, Ac-Phe-OH, Ac-Phg(4-OAc)-OH, Ac-Phg(4-OH)-OEt, Ac-Pro-OH, Ac-Ser(tBu)-OH, Ac-Thr(tBu)-OH, Ac-Trp(Boc)-OH, Ac-Trp-NH2, Ac-Trp-OEt, Ac-Trp-OH, Ac-Trp-OMe, Ac-Tyr(3,5-DiNO2)-OH, Ac-Tyr(Ac)-OH, Ac-Tyr(tBu)-OH, Ac-Tyr-OEt·H2O, Ac-Tyr-OH, Ac-Tyr-OMe, Ac-Val-OH, Ac-β-Ala-OH·DCHA, Alloc-D-Met-OH·DCHA, Alloc-Gly-OH, Alloc-Gly-OH·DCHA, Alloc-Leu-OH, Alloc-Leu-OH DCHA, Alloc-Lys(Fmoc)-OH, Allo-Thr-OH, Beta-Ala-Gly-Him, Boc-1-Nal-OH, Boc-2-Abz-OH, Boc-2-Nal-OH, Boc-2-Pal-OH, Boc-3-Pal-OH, Boc-4-Abz-OH, Boc-4-Amb-OH, Boc-4-Amc-OH, Boc-4-oxo-Pro-OH, Boc-4-oxo-Pro-OMe, Boc-4-Pal-OH, Boc-5-Ava-OH, Boc-8-Aoc-OH, Boc-Abu-OH, Boc-Abu-OH DCHA, Boc-Aib-OH, Boc-Aib-ol, Boc-Ala-NH2, Boc-Alaninol, Boc-Ala-OH, Boc-Ala-ONp, Boc-Ala-OSu, Boc-Aoa-OH, Boc-Arg(Mts)-OH, Boc-Arg(Mts)-OH·CHA, Boc-Arg(NO2)-OH, Boc-Arg(Pbf)-OH, Boc-Arg(Pbf)-OH·CHA, Boc-Arg(Tos)-OH, Boc-Arg(Z)-OH, Boc-Arg-OH, Boc-Arg-OH·HCl·H2O, Boc-Arg-pNA·HCl, Boc-Asn(Trt)-OH, Boc-Asn(Xan)-OH, Boc-Asn-OH, Boc-Asn-ONp, Boc-Asp(OBzl)-OH, Boc-Asp(OBzl)-ONp, Boc-Asp(OBzl)-OSu, Boc-Asp(OcHex)-OH, Boc-Asp(OFm)-OH, Boc-Asp(OMe)-OH, Boc-Asp(OMe)-OH·DCHA, Boc-Asp(OtBu)-OH, Boc-Asp(OtBu)-OH·DCHA, Boc-Asp(OtBu)-ONp, Boc-Asp(OtBu)-OSu, Boc-Asparaginol, Boc-Asp-OBzl, Boc-Asp-OMe, Boc-Asp-OtBu, Boc-Bip(44')-OH, Boc-Cha-OH, Boc-Chg-OH, Boc-Cit-OH, Boc-Cyclopropylglycine, Boc-Cys(Acm)-OH, Boc-Cys(Acm)-ONp, Boc-Cys(Bzl)-OH, Boc-Cys(Bzl)-OSu, Boc-Cys(Dpm)-OH, Boc-Cys(MMt)-OH, Boc-Cys(Npys)-OH, Boc-Cys(pMeBzl)-OH, Boc-Cys(pMeOBzl)-OH, Boc-Cys(tBu)-OH, Boc-Cys(Trt)-OH, Boc-Cys(Trt)-OH·DCHA, Boc-Cys(Trt)-OSu, Boc-Cysteinol(Bzl), Boc-Cysteinol(pMeBzl), Boc-D-1-Nal-OH, Boc-D-2-Nal-OH, Boc-D-2-Pal-OH, Boc-D-3-Pal-OH, Boc-D-4-Pal-OH, Boc-Dab(Boc)-OH·DCHA, Boc-Dab(Fmoc)-OH, Boc-Dab(Z)-OH·DCHA, Boc-Dab-OH, Boc-D-Abu-OH, Boc-D-Abu-OH DCHA, Boc-D-Ala(33-diphenyl)-OH, Boc-D-Ala-NH2, Boc-D-Alaninol, Boc-D-Ala-OH, Boc-D-Ala-OMe, Boc-D-Ala-ONp, Boc-D-Ala-OSu, Boc-D-Allo-Ile-OH, Boc-D-Allo-Ile-OH·DCHA, Boc-Dap(Boc)-OH·DCHA, Boc-Dap(Fmoc)-OH, Boc-Dap(Z)-OH, Boc-Dap(Z)-OH·DCHA, Boc-Dap-OH, Boc-D-Arg(Mtr)-OH, Boc-D-Arg(Mts)-OH·CHA, Boc-D-Arg(Pbf)-OH, Boc-D-Arg(Tos)-OH, Boc-D-Arg-OH·HCl·H2O, Boc-D-Asn(Trt)-OH, Boc-D-Asn-OH, Boc-D-Asp(OBzl)-OH, Boc-D-Asp(OcHex)-OH, Boc-D-Asp(OMe)-OH, Boc-D-Asp(OtBu)-OH, Boc-D-Asp(OtBu)-OH·DCHA, Boc-D-Asp-OBzl, Boc-D-Asp-OH, Boc-D-Asp-OMe, Boc-D-Asp-OtBu, Boc-D-Cha-OH, Boc-D-Chg-OH, Boc-D-Cys(Acm)-OH, Boc-D-Cys(Dpm)-OH, Boc-D-Cys(pMeBzl)-OH, Boc-D-Cys(pMeOBzl)-OH, Boc-D-Cys(Trt)-OH, Boc-D-Cysteinol(Bzl), Boc-D-Cysteinol(pMeBzl), Boc-D-Dap(Fmoc)-OH, Boc-D-Dap-OH, Boc-D-Gln(Trt)-OH, Boc-D-Gln(Xan)-OH, Boc-D-Glu(OBzl)-Osu, Boc-D-Glu(OcHex)-OH, Boc-D-Glu(OMe)-OH, Boc-D-Glu(OMe)-OH-DCHA, Boc-D-Glu(OtBu)-OH, Boc-D-Glu-NH2, Boc-D-Glu-OBzI, Boc-D-Glu-OBzl·DCHA, Boc-D-Gly(Allyl)-OH·DCHA, Boc-D-His(Bom)-OH, Boc-D-His(DNp)-OH·IPA, Boc-D-His(Tos)-OH, Boc-D-His(Trt)-OH, Boc-D-His-OH, Boc-D-HoPhe-OH, Boc-D-HoPro-OH, Boc-D-Hyp-OMe, Boc-D-Ile-OH, Boc-DL-Abu-OH, Boc-DL-Ala-OH, Boc-DL-Asp(OBzl)-OH, Boc-D-Leucinol, Boc-D-Leu-OH·H2O, Boc-DL-Leu-OH H2O, Boc-DL-Met-OH, Boc-DL-Phe(4-NO2)-OH, Boc-DL-Phenylalaninol, Boc-DL-Phenylglycinol, Boc-DL-Phe-OH, Boc-DL-Phg-OH, Boc-DL-Prolinol, Boc-DL-Pro-OH, Boc-DL-Ser(Bzl)-OH, Boc-DL-Tle-OH, Boc-DL-Tyr-OH, Boc-D-Lys(2-Cl-Z)-OH, Boc-D-Lys(Boc)-OH, Boc-D-Lys(Boc)-OH DCHA, Boc-D-Lys(Boc)-ONp, Boc-D-Lys(Boc)-OSu, Boc-D-Lys(Fmoc)-OH, Boc-D-Lys(Tfa)-OH, Boc-D-Lys(Z)-OH, Boc-D-Lysinol(Z), Boc-D-Lys-OH, Boc-DL-β-HoPhe-OH, Boc-D-Methioninol, Boc-D-Met-OH, Boc-D-N-Me-Ala-OH, Boc-D-N-Me-Phe-OH DCHA, Boc-D-N-Me-Phg-OH, Boc-D-N-Me-Tyr(Bzl)-OH, Boc-D-Nva-OH·DCHA, Boc-Dopa-OH, Boc-D-Om(Me2)-OH, Boc-D-Om(Z)-OH, Boc-D-Om(Z)-OSu, Boc-D-Om-OH, Boc-D-Pen(pMeBzl)-OH·DCHA, Boc-D-Phe(2-Br)-OH, Boc-D-Phe(3,4-DiF)-OH, Boc-D-Phe(34-Cl2)-OH, Boc-D-Phe(3-CF3)-OH, Boc-D-Phe(3-Cl)-OH, Boc-D-Phe(4-Br)-OH, Boc-D-Phe(4-Cl)-OH, Boc-D-Phe(4-CN)-OH, Boc-D-Phe(4-F)-OH, Boc-D-Phe(4-I)-OH, Boc-D-Phe(4-Me)-OH, Boc-D-Phe(4-NH2)-OH, Boc-D-Phe(4-NO2)-OH, Boc-D-Phenylalaninol, Boc-D-Phenylglycinol, Boc-D-Phe-OH, Boc-D-Phe-ONp, Boc-D-Phg-OH, Boc-D-Pra-OH, Boc-D-Prolinol, Boc-D-Pro-OH, Boc-D-Pro-OSu, Boc-D-Ser(Bzl)-OH, Boc-D-Ser(Me)-OH, Boc-D-Ser(Me)-OH DCHA, Boc-D-Ser(tBu)-OH, Boc-D-Ser(tBu)-OH·DCHA, Boc-D-Serinol(Bzl), Boc-D-Ser-OBzl, Boc-D-Ser-OH, Boc-D-Ser-OMe, Boc-D-Thr(Bzl)-OH, Boc-D-Thr(Me)-OH, Boc-D-Thr(tBu)-OH, Boc-D-Threoninol(Bzl), Boc-D-Thr-OH, Boc-D-Thz-OH, Boc-D-Trp(Boc)-OH, Boc-D-Trp(For)-OH, Boc-D-Trp-OH, Boc-D-Tryptophanol, Boc-D-Tyr(2-Br-Z)-OH, Boc-D-Tyr(3-I)-OH, Boc-D-Tyr(All)-OH, Boc-D-Tyr(All)-OH DCHA, Boc-D-Tyr(Bzl)-OH, Boc-D-Tyr(Et)-OH, Boc-D-Tyr(Me)-OH, Boc-D-Tyr(tBu)-OH, Boc-D-Tyr-OH, Boc-D-Tyr-OMe, Boc-D-Valinol, Boc-D-Val-OH, Boc-Gln(Trt)-OH, Boc-Gln(Xan)-OH, Boc-Gln-OH, Boc-Gln-ONp, Boc-Glu(OBzl)-OH, Boc-Glu(OBzl)-OMe, Boc-Glu(OcHex)-OH, Boc-Glu(OcHex)-OH DCHA, Boc-Glu(OFm)-OH, Boc-Glu(OMe)-OH, Boc-Glu(OMe)-OMe, Boc-Glu(OSu)-OBzl, Boc-Glu(OSu)-OSu, Boc-Glu(OtBu)-OH, Boc-Glu(OtBu)-ONp, Boc-Glu(OtBu)-OSu, Boc-Glu-NH2, Boc-Glu-OBzl·DCHA, Boc-Glu-OH, Boc-Glu-OMe, Boc-Glu-OtBu, Boc-Glutaminol, Boc-Glutamol(OBzl), Boc-Glycinol, Boc-Gly-Gly-Gly-OH, Boc-Gly-Leu-OH, Boc-Gly-N(OMe)Me, Boc-Gly-NH2, Boc-Gly-OEt, Boc-Gly-OH, Boc-Gly-OMe, Boc-Gly-OSu, Boc-Gly-OtBu, Boc-Gly-Pro-OH, Boc-His(1-Me)-OH, Boc-His(3-Bom)-OMe·HCl, Boc-His(Boc)-OH, Boc-His(Boc)-OH-Benzene, Boc-His(Boc)-OH·DCHA, Boc-His(Boc)-OH·DCHA, Boc-His(Bom)-OH, Boc-His(Dnp)-OH, Boc-His(Dnp)-OH IPA, Boc-His(Tos)-OH, Boc-His(Trt)-OH, Boc-His(Z)-OH, Boc-His-Gly-OH, Boc-His-OH, Boc-Histidinol(Tos), Boc-HoArg(NO2)-OH, Boc-HoPhe-OH, Boc-HoPro-OH, Boc-HoSer(Bzl)-OH, Boc-HoTyr-OH, Boc-Hyp(Bzl)-OH-DCHA, Boc-Hyp-OEt, Boc-Hyp-OH, Boc-Hyp-OL, Boc-Hyp-OMe, Boc-Ida-OH, Boc-Ile-OH·1/2H2O, Boc-Ile-OSu, Boc-Inp-OH, Boc-Inp-OSu, Boc-isoleucinol, Boc-Leucinol, Boc-Leu-Gly-OH, Boc-Leu-OH·H2O, Boc-Leu-OMe, Boc-Leu-OSu, Boc-L-M-Tyrosine, Boc-Lys(2-Cl-Z)-OH, Boc-Lys(Ac)-OH, Boc-Lys(Ac)-pNA, Boc-Lys(Boc)-OH, Boc-Lys(Boc)-OH·DCHA, Boc-Lys(Boc)-OMe, Boc-Lys(Boc)-ONp, Boc-Lys(Boc)-OSu, Boc-Lys(Boc)-Pro-OH, Boc-Lys(Fmoc)-OH, Boc-Lys(Fmoc)-OMe, Boc-Lys(For)-OH, Boc-Lys(Tfa)-OH, Boc-Lys(Z)-OH, Boc-Lys(Z)-OSu, Boc-Lys(Z)-pNA, Boc-Lysinol(2-Cl-Z), Boc-Lysinol(Z), Boc-Lys-OH, Boc-Lys-OMe HCl, Boc-Lys-OSu, Boc-Lys-OtBu, Boc-Met(O)-OH, Boc-Met(O2)-OH, Boc-Methioninol, Boc-Met-OH(oil), Boc-Met-OH(powder), Boc-Met-OSu, Boc-Nip-OH, Boc-Nle-OH, Boc-Nle-OH DCHA, Boc-N-Me-Ala-OH, Boc-N-Me-Arg(Mtr)-OH, Boc-N-Me-Glu(OBzl)-OH, Boc-N-Me-Nle-OH, Boc-N-Me-Phe-OH-DCHA, Boc-N-Me-Phg-OH, Boc-N-Me-Ser(tBu)-OH, Boc-N-Me-Ser-OH, Boc-N-Me-Ser-OH·DCHA, Boc-N-Me-Tyr(Bzl)-OH, Boc-N-Me-Tyr-OH·DCHA, Boc-N-Me-Val-OH, Boc-N-Me-Val-OH·DCHA, Boc-Norvalinol, Boc-Nva-OH DCHA, Boc-Nva-OSu, Boc-ON, Boc-Orn(2-Cl-Z)-OH, Boc-Om(Alloc)-OH·DCHA, Boc-Om(Fmoc)-OH, Boc-Orn(Z)-OH, Boc-Om(Z)-OSu, Boc-Om-OH, Boc-Pen(pMeBzl)-OH, Boc-Phe(2-Br)-OH, Boc-Phe(2-F)-OH, Boc-Phe(2-Me)-OH, Boc-Phe(3,4-DiCl)-OH, Boc-Phe(3,4-DiF)-OH, Boc-Phe(345-TriF)-OH, Boc-Phe(3-F)-OH, Boc-Phe(4-Br)-OH, Boc-Phe(4-Cl)-OH, Boc-Phe(4-F)-OH, Boc-Phe(4-I)-OH, Boc-Phe(4-I)-OMe, Boc-Phe(4-NH2)-OH, Boc-Phe(4-NH2)-OMe, Boc-Phe(4-NHFmoc)-OH, Boc-Phe(4-NHZ)-OH, Boc-Phe(4-NO2)-OH, Boc-Phe-Gly-OMe, Boc-Phe-Leu-OH, Boc-Phenylalaninol, Boc-Phenylglycinol, Boc-Phe-OH, Boc-Phe-OMe, Boc-Phe-ONp, Boc-Phe-OSu, Boc-Phe-Phe-OH, Boc-Phg-OH, Boc-Pra-OH, Boc-Pro-N(OMe)Me, Boc-Pro-NHEt, Boc-Pro-OH, Boc-Pro-OMe, Boc-Pro-Phe-OH, Boc-Pyr-OH, Boc-Pyr-OtBu, Boc-Sar-OH, Boc-Sar-OSu, Boc-Ser(Ac)-OH·DCHA, Boc-Ser(Bzl)-OH, Boc-Ser(Fmoc-Leu)-OH, Boc-Ser(Fmoc-Ser(tBu))-OH, Boc-Ser(Me)-OH, Boc-Ser(Me)-OH·DCHA, Boc-Ser(PO3Bzl2)-OH, Boc-Ser(tBu)-OH, Boc-Ser(tBu)-OH·DCHA, Boc-Ser(Tos)-OMe, Boc-Ser(Trt)-OH, Boc-Serinol(Bzl), Boc-Ser-OBzl, Boc-Ser-OEt, Boc-Ser-OH, Boc-Ser-OH DCHA, Boc-Ser-OMe, Boc-Tea-OH·DCHA, Boc-Thr(Bzl)-OH, Boc-Thr(Fmoc-Val)-OH, Boc-Thr(Me)-OH, Boc-Thr(tBu)-OH, Boc-Threoninol(Bzl), Boc-Thr-OBzl, Boc-Thr-OH, Boc-Thr-OMe, Boc-Thr-OSu, Boc-Thz-OH, Boc-Tic-OH, Boc-Tle-OH, Boc-Tos-Ser-OMe, Boc-Trp(Boc)-OH, Boc-Trp(For)-OH, Boc-Trp(Hoc)-OH, Boc-Trp-OBzl, Boc-Trp-OH, Boc-Trp-OMe, Boc-Trp-OSu, Boc-Trp-Phe-OMe, Boc-Tryptophanol, Boc-Tyr(2-Br-Z)-OH, Boc-Tyr(2-CI-Z)-OH, Boc-Tyr(3-Cl)-OH·DCHA, Boc-Tyr(Bzl)-OH, Boc-Tyr(Bzl)-OSu, Boc-Tyr(Me)-OH, Boc-Tyr(Me)-OMe, Boc-Tyr(tBu)-OH, Boc-Tyr-OEt, Boc-Tyr-OH, Boc-Tyr-OMe, Boc-Tyrosinol, Boc-Tyr-OSu, Boc-Tyr-OtBu, Boc-Val-Ala-OH, Boc-Val-Gly-OH, Boc-Valinol, Boc-Val-NH2, Boc-Val-OH, Boc-Val-OMe, Boc-Val-OSu, Boc-β-Ala-NH2, Boc-β-Ala-OH, Boc-β-Ala-OSu, Boc-β-HoAla-OH, Boc-β-HoArg(Tos)-OH, Boc-β-HoAsn-OH, Boc-β-HoAsp(OBzI)-OH, Boc-β-HoGln-OH, Boc-β-HoGlu(OBzl)-OH, Boc-β-HoIle-OH, Boc-β-HoPhe-OH, Boc-β-HoPro-OH, Boc-β-HoSer(Bzl)-OH, Boc-β-HoVal-OH, Boc-β-Iodo-Ala-OMe, Boc-ε-Acp-OH, Bz-Ala-OH, Bz-Arg-NH2·HCl·H2O, Bz-Arg-OEt·HCl, Bz-Arg-OH, Bz-Arg-OMe·HCl, Bz-Arg-pNA·HCl, Bz-DL-Arg-pNA·HCl, Bz-DL-Leu-OH, Bz-D-Phe-OH, Bz-Gln-OH, Bz-Glu-OH, Bzl-Gly-OH·HCl, Bzl-Hyp-OMe, Bzl-Pro-OH, Bz-Lys-OH, Bz-Nle-OH, Bz-Orn-OH, Bz-Phe-OH, Bz-Pro-OMe, Bz-Tyr-OEt, Bz-Tyr-pNA, D-Alaninol, D-Biotin, D-Biotin-EDA, Dde-Lys(Dde)-OH, Dde-Lys(Fmoc)-OH, DEPBT, Di-Bzl-Gly-OEt, D-Leucinol, DL-Methioninol, DL-m-Tyrosine, DL-Penylalaninol, DL-Phenylglycinol, DL-Prolinol, DL-Valinol, D-Methioninol, D-Penylalaninol, D-Phenylglycinol, D-Prolinol(oil), D-Threoninol, D-Tryptophanol, D-Tyrosinol, D-Valinol, Fmoc-Argininol(Pbf), Fmoc-β-(2-thienyl)-D-Alanine, Fmoc-(Dmb)Ala-OH, Fmoc-(Dmb)Gly-OH, Fmoc-(Fmoc-Hmb)-Ala-OH, Fmoc-(Fmoc-Hmb)-Lys(Boc)-OH, Fmoc-(Fmoc-Hmb)-Val-OH, Fmoc-(N-ethyl)-L-Glutamine, Fmoc-13-diaminopropane hydrochloride, Fmoc-1-Nal-OH, Fmoc-2-Abz-OH, Fmoc-2-Nal-OH, Fmoc-2-Pal-OH, Fmoc-3-(4-thiazolyl)-Alanine, Fmoc-3-Abz-OH, Fmoc-3-Pal-OH, Fmoc-4-Abz-OH, Fmoc-4-Amb-OH, Fmoc-4-Amc-OH, Fmoc-4-Pal-OH, Fmoc-5-Ava-OH, Fmoc-7-Ahp-OH, Fmoc-8-Aoc-OH, Fmoc-Abu-OH, Fmoc-Aib-OH, Fmoc-Ala-Cl, Fmoc-Alaninol, Fmoc-Ala-OH, Fmoc-Ala-OMe, Fmoc-Ala-OPfp, Fmoc-Ala-OSu, Fmoc-Ala-Ser[Psi(MeMe)Pro]-OH, Fmoc-Ala-Thr[Psi(MeMe)Pro]-OH, Fmoc-Allo-Thr(tBu)-OH, Fmoc-Aph(Hor)-OH, Fmoc-Arg(Alloc)2-OH, Fmoc-Arg(Boc)2-OH, Fmoc-Arg(Me)2-OH·HCl, Fmoc-Arg(MePbf)-OH, Fmoc-Arg(Mtr)-OH, Fmoc-Arg(Mtr)-Opfp, Fmoc-Arg(Mts)-OH, Fmoc-Arg(NO2)-OH, Fmoc-Arg(Pbf)-Gly-OH, Fmoc-Arg(Pbf)-NH2, Fmoc-Arg(Pbf)-OH, Fmoc-Arg(Pbf)-OPfp, Fmoc-Arg(Tos)-OH, Fmoc-Argininol(Tos), Fmoc-Arg-OH, Fmoc-Arg-OH·HCl, Fmoc-Asn(Trt)-OH, Fmoc-Asn(Trt)-Opfp, Frnoc-Asn(Trt)-Ser[Psi(MeMe)Pro]-OH, Fmoc-Asn(Trt)-Thr[Psi(MeMe)Pro]-OH, Fmoc-Asn-OH, Fmoc-Asn-Opfp, Fmoc-Asp(Edans)-OH, Fmoc-Asp(OAll)-OH, Fmoc-Asp(OBzl)-OH, Fmoc-Asp(OcHex)-OH, Fmoc-Asp(ODMAB)-OH, Fmoc-Asp(OMe)-OH, Fmoc-Asp(OMpe)-OH, Fmoc-Asp(OtBu)-Glu(OtBu)-NH2, Fmoc-Asp(OtBu)-N(Hmb)-Gly-OH, Fmoc-Asp(OtBu)-OH, Fmoc-Asp(OtBu)-OPfp, Fmoc-Asp(OtBu)-OSu, Fmoc-Asp(OtBu)-Ser[Psi(MeMe)Pro]-OH, Fmoc-Asp(OtBu)-Thr[Psi(MeMe)Pro]-OH, Fmoc-Asparaginol, Fmoc-Asparaginol(Trt), Fmoc-Aspartimol(OtBu), Fmoc-Asp-OAll, Fmoc-Asp-OBzl, Fmoc-Asp-OFm, Fmoc-Asp-OH, Fmoc-Asp-OMe, Fmoc-Asp-OtBu, Fmoc-Bip(44')-OH, Fmoc-Bpa-OH, Fmoc-Cha-OH, Fmoc-Chg-OH, Fmoc-Cit-OH, Fmoc-Cl, Fmoc-Cpg-OH, Fmoc-Cycloheptyl-Ala-OH, Fmoc-Cyclopropylglycine, Fmoc-Cys(Ac)-OH, Fmoc-Cys(Acm)-OH, Fmoc-Cys(Acm)-OPfp, Fmoc-Cys(Bzl)-OH, Fmoc-Cys(CAM)-OH, Fmoc-Cys(Dpm)-OH, Fmoc-Cys(Et)-OH, Fmoc-Cys(Me)-OH, Fmoc-Cys(MMt)-OH, Fmoc-Cys(Mtt)-OH, Fmoc-Cys(Pam)2-OH(R), Fmoc-Cys(Pam)2-OH(S), Fmoc-Cys(pMeBzl)-OH, Fmoc-Cys(pMeOBzl)-OH, Fmoc-Cys(SO3H)-OH, Fmoc-Cys(StBu)-OH, Fmoc-Cys(tBu)-OH, Fmoc-Cys(tert-butoxycarnylpropyl)-OH, Fmoc-Cys(Trt)-NH2, Fmoc-Cys(Trt)-OH, Fmoc-Cys(Trt)-Opfp, Fmoc-Cys(Xati)-OH, Fmoc-Cysteinol(Acm), Fmoc-Cysteinol(Trt), Fmoc-D-1-Nal-OH, Fmoc-D-2-Nal-OH, Fmoc-D-3-Pal-OH, Fmoc-D-4-Pal-OH, Fmoc-Dab(Alloc)-OH, Fmoc-Dab(Boc)-OH, Fmoc-Dab(Dde)-OH, Fmoc-Dab(Fmoc)-OH, Fmoc-Dab(ivDde)-OH, Fmoc-Dab(Mtt)-OH, Fmoc-Dab(Z)-OH, Fmoc-Dab-OH, Fmoc-D-Abu-OH, Fmoc-D-Ala-NH2, Fmoc-D-Alaninol, Fmoc-D-Ala-OH, Fmoc-D-Ala-OPfp, Fmoc-D-Allo-Ile-OH, Fmoc-D-Allo-Ile-OPfp, Fmoc-D-Allo-Thr(tBu)-OH, Fmoc-Dap(Alloc)-OH, Fmoc-Dap(Boc)-OH, Fmoc-Dap(Dde)-OH, Fmoc-Dap(Dnp)-OH, Fmoc-Dap(Mtt)-OH, Fmoc-Dap(Z)-OH, Fmoc-D-Aph(Cbm)-OH, Fmoc-D-Aph(L-Hor)-OH, Fmoc-D-Aph(tBuCbm)-OH, Fmoc-Dap-OH, Fmoc-D-Arg(Me)2-OH·HCl, Fmoc-D-Arg(Mtr)-OH, Fmoc-D-Arg(NO2)-OH, Fmoc-D-Arg(Pbf)-OH, Fmoc-D-Arg(Tos)-OH, Fmoc-D-Arg-OH, Fmoc-D-Arg-OH·HCl, Fmoc-D-Asn(Trt)-OH, Fmoc-D-Asn-OH, Fmoc-D-Asp(OAll)-OH, Fmoc-D-Asp(OBzl)-OH, Fmoc-D-Asp(OtBu)-OH, Fmoc-D-Asp(OtBu)-Opfp, Fmoc-D-Aspartimol(OtBu), Fmoc-D-Asp-OAll, Fmoc-D-Asp-OBzl, Fmoc-D-Asp-OH, Fmoc-D-Asp-OMe, Fmoc-D-Asp-OtBu, Fmoc-D-Bip(44')-OH, Fmoc-D-Bpa-OH, Fmoc-D-Cha-OH, Fmoc-D-Chg-OH, Fmoc-D-Cit-OH, Fmoc-D-Cys(Acm)-OH, Fmoc-D-Cys(Dpm)-OH, Fmoc-D-Cys(Mmt)-OH, Fmoc-D-Cys(tBu)-OH, Fmoc-D-Cys(Trt)-OH, Fmoc-D-Cys(Trt)-OPfp, Fmoc-D-Dab(Boc)-OH, Fmoc-D-Dab(Dde)-OH, Fmoc-D-Dab(Z)-OH, Fmoc-D-Dab-OH, Fmoc-D-Dap(Boc)-OH, Fmoc-D-Dap-OH, Fmoc-Deg-OH, Fmoc-D-Gln(Trt)-OH, Fmoc-D-Gln-OH, Fmoc-D-Gln-OPfp, Fmoc-D-Glu(OBzl)-OH, Fmoc-D-Glu(OMe)-OH, Fmoc-D-Glu(OtBu)-OH, Fmoc-D-Glu(OtBu)-OPfp, Fmoc-D-Glu-OAll, Fmoc-D-Glu-OH, Fmoc-D-Glu-OtBu, Fmoc-D-His(Boc)-OR-CHA, Fmoc-D-His(Fmoc)-OH, Fmoc-D-His(Trt)-OH, Fmoc-D-His-OH, Fmoc-D-HoArg-OH, Fmoc-D-HoArg-OH·HCl, Fmoc-D-HoCit-OH, Fmoc-D-HoCys(Trt)-OH, Fmoc-D-HoPhe-OH, Fmoc-D-HoPro-OH, Fmoc-D-Ile-OH, Fmoc-D-isoGln-OH, Fmoc-DL-Ala-OH, Fmoc-DL-Asp(OtBu)-OH, Fmoc-D-Leu-D-Ser(psi(MeMe)-Pro)-OH, Fmoc-D-Leu-OH, Fmoc-D-Leu-OPfp, Fmoc-DL-Gly(allyl)-OH, Fmoc-DL-Phe(4-NO2)-OH, Fmoc-DL-Phe-OH, Fmoc-DL-Pra-OH, Fmoc-DL-Tyr(Me)-OH, Fmoc-D-Lys(2-Cl-Z)-OH, Fmoc-D-Lys(Ac)-OH, Fmoc-D-Lys(Alloc)-OH, Fmoc-D-Lys(Boc)-OH, Fmoc-D-Lys(Boc)-OPfp, Fmoc-D-Lys(Dde)-OH, Fmoc-D-Lys(Fmoc)-OH, Fmoc-D-Lys(Mtt)-OH, Fmoc-D-Lys(Z)-OH, Fmoc-D-Lys-OH HCl, Fmoc-D-Met(O)-OH, Fmoc-D-Met-OH, Fmoc-D-Met-OPfp, Fmoc-D-Nle-OH, Fmoc-D-N-Me-Leu-OH, Fmoc-D-N-Me-Phe-OH, Fmoc-D-N-Me-Val-OH, Fmoc-D-Nva-OH, Fmoc-Dopa(acetonide)-OH, Fmoc-D-Orn(Alloc)-OH, Fmoc-D-Om(Boc)-OH, Fmoc-D-Pen(Trt)-OH, Fmoc-D-Phe(2-Cl)-OH, Fmoc-D-Phe(3,4-DiCl)-OH, Fmoc-D-Phe(3-Cl)-OH, Fmoc-D-Phe(4-Br)-OH, Fmoc-D-Phe(4-Cl)-OH, Fmoc-D-Phe(4-CN)-OH, Fmoc-D-Phe(4-I)-OH, Fmoc-D-Phe(4-Me)-OH, Fmoc-D-Phe(4-NH2)-OH, Fmoc-D-Phe(4-NHBoc)-OH, Fmoc-D-Phe(4-NO2)-OH, Fmoc-D-Phe(F5)-OH, Fmoc-D-Phe-OH, Fmoc-D-Phe-OPfp, Fmoc-D-Phg(4-NO2)-OH, Fmoc-D-Phg-OH, Fmoc-D-Pra-OH, Fmoc-D-Pro-OH, Fmoc-D-Pro-OPfp, Fmoc-D-Ser(Ac)-OH, Fmoc-D-Ser(Bzl)-OH, Fmoc-D-Ser(HPO3Bzl)-OH, Fmoc-D-Ser(Me)-OH, Fmoc-D-Ser(tBu)-OH, Fmoc-D-Ser(tBu)-OPfp, Fmoc-D-Ser(Trt)-OH, Fmoc-D-Ser-OH, Fmoc-D-Ser-OMe, Fmoc-D-Thr(Ac)-OH, Fmoc-D-Thr(tBu)-OH, Fmoc-D-Thr(tBu)-OPfp, Fmoc-D-Threoninol, Fmoc-D-Threoninol(tBu), Fmoc-D-Thr-OH·H2O, Fmoc-D-Thz-OH, Fmoc-D-Tic-OH, Fmoc-D-Tle-OH, Fmoc-D-trans-Hyp(tBu)-OH, Fmoc-D-Trp(Boc)-OH, Fmoc-D-Trp-OH, Fmoc-D-Trp-OPfp, Fmoc-D-Tryptophanol, Fmoc-D-Tyr(3-Cl)-OH, Fmoc-D-Tyr(3-I)-OH, Fmoc-D-Tyr(3-NO2)-OH, Fmoc-D-Tyr(4-Et)-OH, Fmoc-D-Tyr(Ac)-OH, Fmoc-D-Tyr(Bzl)-OH, Fmoc-D-Tyr(HPO3Bzl)-OH, Fmoc-D-Tyr(Me)-OH, Fmoc-D-Tyr(tBu)-OH, Fmoc-D-Tyr(tBu)-OPfp, Fmoc-D-Tyr-OH, Fmoc-D-Val-OH, Fmoc-D-Val-OPfp, Fmoc-Gln(Trt)-OH, Fmoc-Gln(Trt)-OPfp, Fmoc-Gln(Trt)-Ser[Psi(MeMe)Pro]-OH, Fmoc-Gln(Trt)-Thr[Psi(MeMe)Pro]-OH, Fmoc-Gln-OH, Fmoc-Gln-OPfp, Fmoc-Glu(Alloc)-OH, Fmoc-Glu(Edans)-OH, Fmoc-Glu(OAII)-OH, Fmoc-Glu(OBzl)-OBzl, Fmoc-Glu(OBzl)-OH, Fmoc-Glu(OcHex)-OH, Fmoc-Glu(Odmab)-OH, Fmoc-Glu(OMe)-OH, Fmoc-Glu(OSu)-OSu, Fmoc-Glu(OtBu)-Glu(OtBu)-NH2, Fmoc-Glu(OtBu)-Gly-OH, Fmoc-Glu(OtBu)-OH, Fmoc-Glu(OtBu)-OPfp, Fmoc-Glu(OtBu)-Ser[Psi(MeMe)Pro]-OH, Fmoc-Glu(OtBu)-Thr[Psi(MeMe)Pro]-OH, Fmoc-Glu-OAll, Fmoc-Glu-OBzl, Fmoc-Glu-OH, Fmoc-Glu-OMe, Fmoc-Glu-OtBu, Fmoc-Glutaminol, Fmoc-Glutamol(OtBu), Fmoc-Gly(allyl)-OH, Fmoc-Glycinol, Fmoc-Gly-Cl, Fmoc-Gly-D-Ser(psi(MeMe)-Pro)-OH, Fmoc-Gly-Gly-Gly-OH, Fmoc-Gly-Gly-OH, Fmoc-Gly-HMBA-MBHA-Resin, Fmoc-Gly-OH, Fmoc-Gly-OPfp, Fmoc-Gly-OSu, Fmoc-Gly-Ser(Psi(MeMe)Pro)-OH, Fmoc-Gly-Thr[Psi(MeMe)Pro]-OH, Fmoc-His(Boc)-OH·CHA, Fmoc-His(Boc)-OH·DCHA, Fmoc-His(Bzl)-OH, Fmoc-His(Clt)-OH, Fmoc-His(DNP)-OH, Fmoc-His(Fmoc)-OH, Fmoc-His(MMt)-OH, Fmoc-His(Mtt)-OH, Fmoc-His(Trt)-OH, Fmoc-His(Trt)-OPfp, Fmoc-His(Z)-OH, Fmoc-HoArg(Pbf)-OH, Fmoc-HoArg-OH, Fmoc-HoArg-OH HCl, Fmoc-HoCit-OH, Fmoc-HoCys(Trt)-OH, Fmoc-HoLeu-OH, Fmoc-HomoArg(Me)2-OH·HCl, Fmoc-HoPhe-OH, Fmoc-HoPro-OH, Fmoc-HoSer(Trt)-OH, Fmoc-HoTyr-OH DCHA, Fmoc-Hyp(Bom)-OH, Fmoc-Hyp(Bzl)-OH, Fmoc-Hyp(tBu)-OH, Fmoc-Hyp-OBzl, Fmoc-Hyp-OH, Fmoc-Hyp-OMe, Fmoc-Ida-OH, Fmoc-Ile-OH, Fmoc-Ile-OPfp, Fmoc-Ile-Pro-OH, Fmoc-Ile-Ser[Psi(MeMe)Pro]-OH, Fmoc-Ile-Thr[Psi(MeMe)Pro]-OH, Fmoc-Inp-OH, Fmoc-isoGln-OH, Fmoc-isoleucinol, Fmoc-Leucinol, Fmoc-Leu-OH, Fmoc-Leu-OPfp, Fmoc-Leu-OSu, Fmoc-Leu-Ser[Psi(MeMe)Pro]-OH, Fmoc-Leu-Thr[Psi(MeMe)Pro]-OH, Fmoc-Lys(2-Cl-Z)-OH, Fmoc-Lys(Ac)-OH, Fmoc-Lys(Alloc)-OH, Fmoc-Lys(Biotin)-OH, Fmoc-Lys(Boc)-OH, Fmoc-Lys(Boc)-OPfp, Fmoc-Lys(Boc)-OSu, Fmoc-Lys(Boc)-Ser[Psi(MeMe)Pro]-OH, Fmoc-Lys(Boc)-Thr[Psi(MeMe)Pro]-OH, Fmoc-Lys(BocMe)-OH, Fmoc-Lys(Bz)-OH, Fmoc-Lys(Caproyl)-OH, Fmoc-Lys(Dabcyl)-OH, Fmoc-Lys(Dansyl)-OH, Fmoc-Lys(Dde)-OH, Fmoc-Lys(Dnp)-OH, Fmoc-Lys(Fmoc)-OH, Fmoc-Lys(Fmoc)-OPfp, Fmoc-Lys(For)-OH, Fmoc-Lys(ipr)-OH, Fmoc-Lys(iprBoc)-OH, Fmoc-Lys(iprBoc)-OH DCHA, Fmoc-Lys(ivDde)-OH, Fmoc-Lys(Me)2-OH·HCl, Fmoc-Lys(Me)3-OH, Fmoc-Lys(Mtt)-OH, Fmoc-Lys(Nic)-OH, Fmoc-Lys(Palmitoyl)-OH, Fmoc-Lys(Tfa)-OH, Fmoc-Lys(Trt)-OH, Fmoc-Lys(Z)-OH, Fmoc-Lys[Boc-Cys(Trt)]-OH, Fmoc-Lysinol(Boc), Fmoc-Lys-OAll·HCl, Fmoc-Lys-OH, Fmoc-Lys-OH·HCl, Fmoc-Lys-OMe HCl, Fmoc-Met(O)-OH, Fmoc-Met(O2)-OH, Fmoc-Met-OH, Fmoc-Met-OPfp, Fmoc-N-(2-Boc-aminoethyl)-Gly-OH, Fmoc-N(Hmb)-Gly-OH, Fmoc-Nip-OH, Fmoc-Nle-OH, Fmoc-N-Me-Ala-OH, Fmoc-N-Me-Arg(Mtr)-OH, Fmoc-N-Me-Asp(OtBu)-OH, Fmoc-N-Me-Glu(OtBu)-OH, Fmoc-N-Me-Ile-OH, Fmoc-N-Me-Leu-OH, Fmoc-N-Me-Lys(Boc)-OH, Fmoc-N-Me-Met-OH, Fmoc-N-Me-Nle-OH, Fmoc-N-Me-Nva-OH, Fmoc-N-Me-Phe-OH, Fmoc-N-Me-Ser(Me)-OH, Fmoc-N-Me-Ser(tBu)-OH, Fmoc-N-Me-Thr(Bzl)-OH, Fmoc-N-Me-Thr(tBu)-OH, Fmoc-N-Me-Thr-OH, Fmoc-N-Me-Tyr(tBu)-OH, Fmoc-N-Me-Val-OH, Fmoc-Nva-OH, Fmoc-Oic-OH, Fmoc-O-Phospho-Tyrosine, Fmoc-Orn(2-Cl-Z)-OH, Fmoc-Orn(Alloc)-OH, Fmoc-Orn(Boc)-OH, Fmoc-Om(Dde)-OH, Fmoc-Orn(Fmoc)-OH, Fmoc-Om(ivDde)-OH, Fmoc-Om(Mtt)-OH, Fmoc-Orn(Trt)-OH, Fmoc-Orn(Z)-OH, Fmoc-Orn-OH·HCl, Fmoc-OSu, Fmoc-Pen(Trt)-OH, Fmoc-Phe(2,6-DiF)-OH, Fmoc-Phe(2-Br)-OH, Fmoc-Phe(2-Cl)-OH, Fmoc-Phe(2-F)-OH, Fmoc-Phe(3,4-DiF)-OH, Fmoc-Phe(3,5-DiF)-OH, Fmoc-Phe(3-Br)-OH, Fmoc-Phe(3-Cl)-OH, Fmoc-Phe(3-F)-OH, Fmoc-Phe(4-Ac)-OH, Fmoc-Phe(4-Br)-OH, Fmoc-Phe(4-CF3)-OH, Fmoc-Phe(4-Cl)-OH, Fmoc-Phe(4-CN)-OH, Fmoc-Phe(4-F)-OH, Fmoc-Phe(4-I)-OH, Fmoc-Phe(4-Me)-OH, Fmoc-Phe(4-NH2)-OH, Fmoc-Phe(4-NO2)-OH, Fmoc-Phe(F5)-OH, Fmoc-Phenylalaninol, Fmoc-Phe-OH, Fmoc-Phe-OMe, Fmoc-Phe-OPfp, Fmoc-Phe-Ser[Psi(MeMe)Pro]-OH, Fmoc-Phe-Thr[Psi(MeMe)Pro]-OH, Fmoc-Phg-OH, Fmoc-Pra-OH, Fmoc-Pro-Leu-Gly-OH, Fmoc-Prolinol, Fmoc-Pro-OH, Fmoc-Pro-OPfp, Fmoc-Pro-OSu, Fmoc-Sar-OH, Fmoc-Sec(mob)-OH, Fmoc-Ser(Ac)-OH, Fmoc-Ser(Bzl)-OH, Fmoc-Ser(Et)-OH, Fmoc-Ser(HPO3Bzl)-OH, Fmoc-Ser(Me)-OH, Fmoc-Ser(TBDMS)-OH, Fmoc-Ser(tBu)-OH, Fmoc-Ser(tBu)-OPfp, Fmoc-Ser(tBu)-Ser[Psi(MeMe)Pro]-OH, Fmoc-Ser(tBu)-Thr[Psi(MeMe)Pro]-OH, Fmoc-Ser(Trt)-OH, Fmoc-Serinol, Fmoc-Serinol(tBu), Fmoc-Ser-OBzl, Fmoc-Ser-OH, Fmoc-Ser-OMe, Fmoc-Ser-OPAC, Fmoc-Thr(Ac)-OH, Fmoc-Thr(Bzl)-OH, Fmoc-Thr(Et)-OH, Fmoc-Thr(HPO3Bzl)-OH, Fmoc-Thr(Me)-OH, Fmoc-Thr(SO3Na)-OH, Fmoc-Thr(TBDMS)-OH, Fmoc-Thr(tBu)-OH, Fmoc-Thr(tBu)-OPfp, Fmoc-Thr(tBu)-Ser[Psi(MeMe)Pro]-OH, Fmoc-Thr(tBu)-Thr(Psi(MeMe)pro)-OH, Fmoc-Thr(Trt)-OH, Fmoc-Threoninol, Fmoc-Threoninol(tBu)DHP, Fmoc-Thr-OBzl, Fmoc-Thr-OH, Fmoc-Thr-OMe, Fmoc-Thr-OPAC, Fmoc-Thz-OH, Fmoc-Tic-OH, Fmoc-Tle-OH, Fmoc-Trp(5-OH)-OH, Fmoc-Trp(Boc)-OH, Fmoc-Trp(Boc)-Ser[Psi(MeMe)Pro]-OH, Fmoc-Trp(Boc)-Thr[Psi(MeMe)Pro]-OH, Fmoc-Trp-OH, Fmoc-Trp-OPfp, Fmoc-Trp-OSu, Fmoc-Tryptophanol, Fmoc-Tyr(2-Br-Z)-OH, Fmoc-Tyr(3,5-DiI)-OH, Fmoc-Tyr(3-Cl)-OH, Fmoc-Tyr(3-I)-OH, Fmoc-Tyr(3-NO2)-OH, Fmoc-Tyr(Ac)-OH, Fmoc-Tyr(Bzl)-OH, Fmoc-Tyr(HPO3Bzl)-OH, Fmoc-Tyr(Me)-OH, Fmoc-Tyr(PO3Bzl2)-OH, Fmoc-Tyr(SO3H)-OH, Fmoc-Tyr(SO3Na)-OH-H2O, Fmoc-Tyr(tBu)-OH, Fmoc-Tyr(tBu)-OPfp, Fmoc-Tyr(tBu)-pNA, Fmoc-Tyr(tBu)-Ser[Psi(MeMe)Pro]-OH, Fmoc-Tyr(tBu)-Thr[Psi(MeMe)Pro]-OH, Fmoc-Tyr-OBzl, Fmoc-Tyr-OH, Fmoc-Tyr-OMe, Fmoc-Tyrosinol(tBu), Fmoc-Tyr-OtBu, Fmoc-Val-Cl, Fmoc-Val-Gly-OH, Fmoc-Valinol, Fmoc-Val-OH, Fmoc-Val-OPfp, Fmoc-Val-Ser[Psi(MeMe)Pro]-OH, Fmoc-Val-Thr[Psi(MeMe)Pro]-OH, Fmoc-β-Ala-OH, Fmoc-β-Ala-OPfp, Fmoc-β-cyclopropyl-L-Alanine, Fmoc-β-D-HoTyr(tBu)-OH, Fmoc-β-HoAla-OH, Fmoc-β-HoArg(Pbf)-OH, Fmoc-β-HoAsn(Trt)-OH, Fmoc-β-HoAsp(OtBu)-OH, Fmoc-β-HoGln(Trt)-OH, Fmoc-β-HoGlu(OtBu)-OH, Fmoc-β-HoIle-OH, Fmoc-β-HoLeu-OH, Fmoc-β-HoLys(Boc)-OH, Fmoc-β-HoMet-OH, Fmoc-β-HoPhe-OH, Fmoc-P-HoPro-OH, Fmoc-β-HoSer(Bzl)-OH, Fmoc-β-HoSer(tBu)-OH, Fmoc-β-HoThr(tBu)-OH, Fmoc-β-HoTrp(Boc)-OH, Fmoc-β-HoTyr(tBu)-OH, Fmoc-β-HoVal-OH, Fmoc-γ-Abu-OH, Fmoc-ε-Acp-OH, For-Ala-OH, For-DL-Trp-OH, For-Gly-OEt, For-Gly-OH, For-Met-OH, For-Val-OH, H-1-Nal-OH, H-2-Nal-OH-HCI, H-2-Pal-OH·2HCl, H-3-Pal-OH·2HCl, H-3-Pal-OMe·2HCl, H-4-oxo-Pro-OH·HBr, H-4-Pal-OH·2HCl, H-5-Ava-OH, H-Abu-Gly-OH, H-Abu-NH2·HCl, H-Abu-OH, H-Abu-OtBu·HCl, H-Acpc-OEt·HCl, H-Aib-OEt·HCl, H-Aib-OH, H-Aib-OMe·HCl, H-Aib-OtBu·HCl, H-Ala-Ala-OH, H-Ala-Ala-OMe·HCl, H-Ala-AMC·HCl, H-Ala-Glu-OH, H-Ala-NH2·HCl, H-Ala-OBzl·HCl, H-Ala-OBzl·TosOH, H-Ala-OcHex·HCl, H-Ala-OcHex·TosOH, H-Ala-OH, H-Ala-OiPr·HCl, H-Ala-OMe·HCl, H-Ala-OtBu·HCl, H-Ala-Phe-OH, H-Ala-pNA HCl, H-Ala-Pro-OMe·HCl, H-Ala-Trp-OH, H-Ala-Tyr-OH, H-Arg(Mtr)-OH·1/2H2O, H-Arg(NO2)-OBzl·HCl, H-Arg(NO2)-OH, H-Arg(NO2)-OMe·HCl, H-Arg(Pbf)-NH2, H-Arg(Pbf)-OH, H-Arg(Pbf)-OMe·HCl, H-Arg(Tos)-OH, H-Arg-NH2·2HCl, H-Arg-OEt·2HCl, H-Arg-OH, H-Arg-OH·HCl, H-Arg-OMe·2HCl, H-Arg-OtBu·2HCl, H-Arg-pNA·2HCl, H-Asn(Trt)-OH·H2O, H-Asn-OH, H-Asn-OMe·HCl, H-Asn-OtBu, H-Asp(OBzl)-NH2·HCl, H-Asp(OBzl)-OBzl·HCl, H-Asp(OBzl)-OBzl·TosOH, H-Asp(OBzl)-OH, H-Asp(OBzl)-OtBu·HCl, H-Asp(OBzl)-pNA·HCl, H-Asp(OcHex)-OH, H-Asp(OEt)-OEt·HCl, H-Asp(OMe)-OH, H-Asp(OMe)-OH-HCI, H-Asp(OMe)-OMe·HCl, H-Asp(OMe)-OtBu·HCl, H-Asp(OtBu)-OH, H-Asp(OtBu)-OMe·HCl, H-Asp(OtBu)-OtBu·HCl, H-Asp-OBzl, H-Asp-OMe, H-Asp-OtBu, H-Bpa-OH, H-Cha-NH2, H-Cha-OMe·HCl, H-Chg-OH, H-Chg-OMe·HCl, H-Chg-OtBu·HCl, H-Cit-OH, H-Cys(Acm)-NH2·HCl, H-Cys(Acm)-OH·, H-Cys(Acm)-OH-HCl, H-Cys(Boc)-OMe·HCl, H-Cys(Bzl)-OH, H-Cys(Bzl)-OMe·HCl, H-Cys(Dpm)-OH, H-Cys(Me)-OH, H-Cys(pMeOBzl)-OH, H-Cys(tBu)-OH HCl, H-Cys(tBu)-OtBu·HCl, H-Cys(Trt)-NH2, H-Cys(Trt)-OH, H-Cys(Trt)-OMeHCl, H-Cys(Trt)-OtBu·HCl, H-Cys(Z)-OH, H-Cys(Z)-OH HCl, H-Cys-NH2·HCl, H-Cys-OEt·HCl, H-Cys-OH, H-Cys-OMe·HCl, H-D-1-Nal-OH, H-D-1-Nal-OH·HCl, H-D-2-Nal-OH, H-D-2-Nal-OH HCl, H-D-2-Pal-OH·2HCl, H-D-3-Pal-OH·2HCl, H-D-4-Pal-OH·2HCl, H-Dab(Z)-OH, H-Dab-HBr, H-Dab-OH·HCl, H-D-Abu-OEt·HCl, H-D-Abu-OH, H-D-Ala-NH2·HCl, H-D-Ala-OBzl·TosOH, H-D-Ala-OH, H-D-Ala-OiPr·HCl, H-D-Ala-OMe·HCl, H-D-Ala-OtBu·HCl, H-D-Allo-Ile-OH, H-Dap(Boc)-OH, H-Dap-OH-HBr, H-Dap-OH HCl, H-D-Arg(NO2)-OH, H-D-Arg(Pbf)-OH, H-D-Arg-NH2·2HCl, H-D-Arg-OH, H-D-Arg-OH·HCl, H-D-Arg-OMe·2HCl, H-D-Asn-OH·H2O, H-D-Asp(OBzl)-OBzl·HCl, H-D-Asp(OBzl)-OBzl·TosOH, H-D-Asp(OBzl)-OH, H-D-Asp(OEt)-OEt·HCl, H-D-Asp(OMe)-OH·HCl, H-D-Asp(OMe)-OMe·HCl, H-D-Asp(OtBu)-OH, H-D-Asp(OtBu)-OMe HCl, H-D-Asp(OtBu)-OtBu·HCl, H-D-Asp-OBzl, H-D-Asp-OH, H-D-Asp-OMe, H-D-Asp-OtBu, H-D-Asp-OtBu·HCl, H-D-Bip(44')-OH-HCI, H-D-Bpa-OH, H-D-Chg-OH, H-D-Cit-OH, H-D-Cys(Acm)-OH·HCl, H-D-Cys(pMeOBzl)-OBzl·TosOH, H-D-Cys(Trt)-OH, H-D-Cys-OEt·HCl, H-D-Cys-OH·H2O·HCl, H-D-Cys-OMe·HCl, H-D-Dab-OH·2HCl, H-Deg-OH, H-D-Gln(Trt)-OH·H2O, H-D-Gln-OH, H-D-Glu(OBzl)-OBzl·HCl, H-D-Glu(OBzl)-OH, H-D-Glu(OEt)-OEt·HCl, H-D-Glu(OMe)-OH, H-D-Glu(OMe)-OMe·HCl, H-D-Glu(OtBu)-OH, H-D-Glu(OtBu)-OMe·HCl, H-D-Glu(OtBu)-OtBu·HCl, H-D-Glu-OBzl, H-D-Glu-OBzl·HCl, H-D-Glu-OH, H-D-Glu-OtBu, H-D-Gly(Allyl)-OH, H-D-Gly(allyl)-OH·HCl, H-D-His(Trt)-OH, H-D-His-OH, H-D-HoArg-OH, H-D-HoCys-OH, H-D-HoPhe-OH, H-D-HoPro-OH, H-D-HoPro-OMe·HCl, H-D-HoSer-OH, H-DL-2-Nal-OH, H-DL-3-Pal-OH·2HCl, H-DL-Ala-OMe·HCl, H-DL-Arg-OH-HCI, H-DL-Asp(OBzl)-OH, H-DL-Asp(OMe)-OMe·HCl, H-DL-Asp(OtBu)-OMe·HCl, H-DL-Asp-OMe, H-DL-Dab·2HCl, H-D-Leu-Gly-OH, H-D-Leu-Leu-OH, H-D-Leu-NH2·HCl, H-D-Leu-OBzl·TosOH, H-D-Leu-OEt·HCl, H-D-Leu-OH, H-D-Leu-OMe·HCl, H-D-Leu-OtBu·HCl, H-DL-Glu(OMe)-OMe·HCl, H-DL-His-OH, H-DL-HoPhe-OH, H-DL-HoPhe-OMe·HCl, H-DL-HoSer-OH, H-DL-Ile-OH, H-DL-Leu-NH2·HCl, H-DL-Leu-OMe·HCl, H-DL-Lys(Fmoc)-OH, H-DL-Lys-OMe·2HCl, H-DL-Met-OH, H-DL-Met-OMe·HCl, H-DL-Nip-OH, H-DL-Nle-OH, H-DL-N-Me-Val-OH, H-DL-Nva-OH, H-DL-Om-OH HCl, H-DL-Phe(3-Br)-OH, H-DL-Phe(3-CN)-OH, H-DL-Phe(3-F)-OH, H-DL-Phe(4-Cl)-OH, H-DL-Phe(4-Cl)-OH·HCl, H-DL-Phe(4-Cl)-OMe·HCl, H-DL-Phe(4-I)-OH, H-DL-Phe(4-Me)-OH, H-DL-Phe(4-NO2)-OH H2O, H-DL-Phe-OEt·HCl, H-DL-Phe-OMe·HCl, H-DL-Phg(2-Cl)-OH, H-DL-Phg-OH, H-DL-Pra-OH, H-DL-Pro-NH2, H-DL-Pro-OH, H-DL-Ser(Bzl)-OH, H-DL-Ser-OEt·HCl, H-DL-Ser-OMe·HCl, H-DL-Ser-OtBu·HCl, H-DL-Tle-OH, H-DL-Trp-NH2, H-DL-Trp-OMe·HCl, H-DL-Tyr(Me)-OH, H-DL-Tyr-OMe·HCl, H-DL-Val-OEt·HCl, H-DL-Val-OMe·HCl, H-D-Lys(Boc)-OtBu·HCl, H-D-Lys(Fmoc)-OH, H-D-Lys(Tfa)-OH, H-D-Lys(Z)-OMe·HCl, H-D-Lys(Z)-OtBu·HCl, H-D-Lys-OBzl·HCl·TosOH, H-D-Lys-OH HCl, H-D-Lys-OMe·2HCl, H-D-Met-OEt·HCl, H-D-Met-OH, H-D-Met-OMe HCl, H-D-Nle-OH, H-D-Nle-OMe·HCl, H-D-N-Me-Leu-OBzl·TosOH, H-D-N-Me-Pro-OH, H-D-N-Me-Val-OH·HCl, H-D-N-Me-Val-OMe·HCl, H-D-Nva-OEt·HCl, H-D-Orn(Boc)-OH, H-D-Orn(Z)-OH, H-D-Om-OH HCl, H-D-Pen-OH, H-D-Phe(2,4-Dime)-OH, H-D-Phe(2,5-DiCI)-OH, H-D-Phe(2,6-DiCI)-OH, H-D-Phe(2-Br)-OH, H-D-Phe(2-Cl)-OH·HCl, H-D-Phe(2-F)-OH HC1, H-D-Phe(3,4-DiCl)-OH, H-D-Phe(3,4-DiF)-OH, H-D-Phe(3,5-DiF)-OH, H-D-Phe(3-Br)-OH, H-D-Phe(3-Br)-OH HC1, H-D-Phe(3-Cl)-OH, H-D-Phe(4-Br)-OH, H-D-Phe(4-CF3)-OH HC1, H-D-Phe(4-Cl)-OH, H-D-Phe(4-Cl)-OH·HCl, H-D-Phe(4-Cl)-OMe·HCl, H-D-Phe(4-CN)-OH, H-D-Phe(4-F)-OH·HCl, H-D-Phe(4-I)-OH, H-D-Phe(4-Me)-OH, H-D-Phe(4-NO2)-OH·H2O, H-D-Phe(4-NO2)-OMe·HCl, H-D-Phe-AMC·HCl, H-D-Phe-NH2·HCl, H-D-Phe-OBzl·HCl, H-D-Phe-OH, H-D-Phe-OMe·HCl, H-D-Phe-OtBu·HCl, H-D-Phe-pNA, H-D-Phg(4-Cl)-OH, H-D-Phg(4-Cl)-OH·HCl, H-D-Phg-AMC·HCl, H-D-Phg-NH2, H-D-Phg-OH, H-D-Phg-OMe·HCl, H-D-Phg-OtBu·HCl, H-D-Pra-OH, H-D-Pro-NH2, H-D-Pro-NH2·HCl, H-D-Pro-OBzl·HCl, H-D-Pro-OH, H-D-Pro-OMe·HCl, H-D-Pro-OtBu, H-D-Pro-OtBu·HCl, H-D-Pyr-OEt, H-D-Ser(Bzl)-OH, H-D-Ser(Bzl)-OH-HCI, H-D-Ser(tBu)-OBzl·HCl, H-D-Ser(tBu)-OH, H-D-Ser(tBu)-OMe·HCl, H-D-Ser(tBu)-OtBu·HCl, H-D-Ser-OBzl·HCl, H-D-Ser-OH, H-D-Ser-OMe·HCl, H-D-Thr(Me)-OH, H-D-Thr(tBu)-OH, H-D-Thr(tBu)-OMe·HCl, H-D-Thr-OBzl, H-D-Thr-OBzl·HCl, H-D-Thr-OH, H-D-Thr-OMe·HCl, H-D-Tic-OH, H-D-Tle-OH, H-D-Tle-OMe·HCl, H-D-Trp(Boc)-OH, H-D-Trp-OBzl·HCl, H-D-Trp-OEt·HCl, H-D-Trp-OH, H-D-Trp-OMe·HCl, H-D-Tyr(3,5-DiBr)-OH2·H2O, H-D-Tyr(3-Cl)-OH, H-D-Tyr(3-I)-OH, H-D-Tyr(Bzl)-OH, H-D-Tyr(tBu)-OH, H-D-Tyr(tBu)-OtBu·HCl, H-D-Tyr-NH2, H-D-Tyr-NH2·HCl, H-D-Tyr-OEt·HCl, H-D-Tyr-OH, H-D-Tyr-OMe, H-D-Tyr-OMe·HCl, H-D-Tyr-OtBu, H-D-Val-OBzl·TosOH, H-D-Val-OEt·HCl, H-D-Val-OH, H-D-Val-OMe·HCl, H-D-Val-OtBu·HCl, H-gamma-Glu-Glu-OH, H-Gln(Trt)-OH·H2O, H-Gln-OBzl, H-Gln-OH, H-Gln-OMe·HCl, H-Gln-OtBu·HCl, H-Gln-pNA, H-Glu(Gly-him)-OH, H-Glu(OAll)-OAll, H-Glu(OBzl)-NCA, H-Glu(OBzl)-OBzl·HCl, H-Glu(OBzl)-OBzl-TosOH, H-Glu(OBzl)-OH, H-Glu(OBzl)-OH·HCl, H-Glu(OBzl)-OtBu·HCl, H-Glu(OcHex)-OBzl·HCl, H-Glu(OcHex)-OH, H-Glu(OEt)-OEt·HCl, H-Glu(OEt)-OH, H-Glu(OMe)-OH, H-Glu(OMe)-OMe·HCl, H-Glu(OMe)-OtBu·HCl, H-Glu(OtBu)-NH2·HCl, H-Glu(OtBu)-OBzl·HCl, H-Glu(OtBu)-OH, H-Glu(OtBu)-OMe·HCl, H-Glu(OtBu)-OtBu·HCl, H-Glu-Gly-OH, H-Glu-OBzl, H-Glu-OBzl·HCl, H-Glu-OEt, H-Glu-OH, H-Glu-OMe, H-Glu-OtBu, H-Glu-OtBu·HCl, H-Glu-pNA, H-Gly-Ala-Gly-OH·HCl, H-Gly-AMC·HCl, H-Gly-Asn-OH, H-Gly-Asp-OH, H-Gly-Gly-Ala-OH HC1, H-Gly-Gly-Gly-OH, H-Gly-Gly-OMe·HCl, H-Gly-Gly-Phe-OH, H-Gly-Hyp-OH, H-Gly-Met-OH, H-Gly-NH2·AcOH, H-Gly-NH2·HCl, H-Gly-OBzl HCl, H-Gly-OBzl TosOH, H-Gly-OEt·HCl, H-Gly-OH, H-Gly-Oipr·HCl, H-Gly-OMe·HCl, H-Gly-OtBu·AcOH, H-Gly-OtBu·HCl, H-Gly-Phe-OH, H-Gly-pNA HCl, H-Gly-Trp-OH, H-Gly-Val-OH, H-Gly-Val-OH·HCl, H-His(1-Me)-OH, H-His(1-Me)-OH·2HCl, H-His(1-Me)-OMe·HCl, H-His(Trt)-OH, H-His(Trt)-OMe HCl, H-His-NH2·2HCl, H-His-OH, H-His-OMe·2HCl, H-HoArg-OH, H-HoArg-OH HC1, H-HoArg-OMe·2HCl, H-HoPhe-OET·HCl, H-HoPhe-OH, H-HoPhe-OMe·HCl, H-HoPro-OH, H-HoSer-OH, H-HoTyr-OH·HBr, H-Hyp(Bzl)-OH·HCl, H-Hyp(tBu)-OH, H-Hyp(tBu)-OtBu·HCl, H-Hyp-OBzl, H-Hyp-OBzl·HCl, H-Hyp-OEt·HCl, H-Hyp-OH, H-Hyp-OMe·HCl, H-Ile-NH2·HCl, H-Ile-OAll·TosOH, H-Ile-OEt·HCl, H-Ile-OH, H-Ile-OMe·HCl, H-Ile-OtBu-HCl, H-Leu-Ala-OH, H-Leu-CMK·HCl, H-Leu-Gly-OH, H-Leu-Leu-OH-HCI, H-Leu-Leu-OMe·HCl, H-Leu-NH2·HCl, H-Leu-OAll·TosOH, H-Leu-OBzl·TosOH, H-Leu-OEt·HCl, H-Leu-OH, H-Leu-OMe·HCl, H-Leu-OtBu, H-Leu-OtBu·HCl, H-Leu-pNA·HCl, H-Lys(2-Cl-Z)-OH, H-Lys(Ac)-OH, H-Lys(Ac)-OH·HCl, H-Lys(Alloc)-OH, H-Lys(Biotinyl)-OH, H-Lys(Boc)-NH2, H-Lys(Boc)-OBzl·HCl, H-Lys(Boc)-OBzl·TosOH, H-Lys(Boc)-OH, H-Lys(Boc)-OMe·HCl, H-Lys(Boc)-OtBu·HCl, H-Lys(Butyryl)-OH, H-Lys(Caproyl)-OH·HCl, H-Lys(Crotonyl)-OH, H-Lys(Dnp)-OH·HCl, H-Lys(Fmoc)-OH, H-Lys(Fmoc)-OH-HCI, H-Lys(Fmoc)-OMe·HCl, H-Lys(FrucTosyl)-OH, H-Lys(Propionyl)-OH, H-Lys(Suc)-OH·HCl, H-Lys(Tfa)-NCA, H-Lys(Tfa)-OH, H-Lys(Z)-NH2·HCl, H-Lys(Z)-OBzl HCl, H-Lys(Z)-OBzl·TosOH, H-Lys(Z)-OH, H-Lys(Z)-OMe·HCl, H-Lys(Z)-OtBu·HCl, H-Lysinol(Z)·HCl, H-Lys-OBzl HCl-TosOHTosOH, H-Lys-OEt·2HCl, H-Lys-OH·2HCl, H-Lys-OH HCl, H-Lys-OMe·2HCl, H-Met(O)-OH, H-Met-NH2·HCl, H-Met-OAll·TosOH, H-Met-OEt HCl, H-Met-OH, H-Met-OiPr HCl, H-Met-OMe HCl, H-Met-OtBu·HCl, H-Nle-NH2·HCl, H-Nle-OBzl HCl, H-Nle-OBzl·TosOH, H-Nle-OH, H-Nle-OMe·HCl, H-Nle-OtBu HCl, H-N-Me-Aib-NH2, H-N-Me-Ala-OH, H-N-Me-Ala-OH HCl, H-N-Me-Ala-OMe HCl, H-N-Me-D-Ala-OH·HCl, H-N-Me-Ile-OH, H-N-Me-Leu-OBzl·TosOH, H-N-Me-Phe-OH·HCl, H-N-Me-Pro-OH, H-N-Me-Ser-OH, H-N-Me-Ser-OH HCl, H-N-Me-Val-OH·HCl, H-Nva-OEt HCl, H-Nva-OMe HCl, H-Nva-OtBu HCl, H-Om(2-Cl-Z)-OH, H-Om(Boc)-OBzl-HCl, H-Om(Boc)-OMe·HCl, H-Om(Tfa)-OH, H-Orn(Z)-OH, HOrn(Z)-OMe·HCl, H-Om(Z)-OtBu·HCl, H-Om-AMC·HCl, H-Om-OH·HCl, H-Orn-OMe·2HCl, H-Phe(2,4-DiCI)-OH, H-Phe(2,4-Dime)-OH, H-Phe(2,5-DiCl)-OH, H-Phe(2,6-DiCl)-OH, H-Phe(2-Br)-OH, H-Phe(2-Cl)-OH, H-Phe(2-F)-OH, H-Phe(2-Me)-OH, H-Phe(3,4-DiCl)-OH, H-Phe(3,4-DiCl)-OMe·Cl, H-Phe(3-Br)-OH, H-Phe(3-Cl)-OH, H-Phe(3-Cl)-OH·HCl, H-Phe(3-CN)-OH, H-Phe(4-Br)-OH, H-Phe(4-Br)-OH-HCI, H-Phe(4-Br)-OMe·HCl, H-Phe(4-CF3)-OH, H-Phe(4-Cl)-OH, H-Phe(4-Cl)-OH·HCl, H-Phe(4-CN)-OH, H-Phe(4-F)-OH, H-Phe(4-I)-OH, H-Phe(4-Me)-OH, H-Phe(4-Me)-OH·HCl, H-Phe(4-NH2)-OH, H-Phe(4-NH2)-OH·HCl, H-Phe(4-NO2)-OEt HCl, H-Phe(4-NO2)-OH, H-Phe(4-NO2)-OH H2O, H-Phe(4-NO2)-OMe HCl, H-Phe-Ala-OH, H-Phe-Gly-OH, H-Phe-Leu-OH, H-Phe-NH2, H-Phe-NH2·HCl, H-Phe-NHNH2, H-Phe-OAll·TosOH, H-Phe-OBzl·HCl, H-Phe-OEt·HCl, H-Phe-OH, H-Phe-OMe·HCl, H-Phe-OtBu HCl, H-Phe-Phe-OH, H-Phe-pNA, H-Phg(4-Cl)-OH, H-Phg(4-OH)-OEt, H-Phg(4-OH)-OH, H-Phg-AMC HCl, H-Phg-NH2·HCl, H-Phg-OH, H-Phg-OtBu·HCl, H-Pra-OH, H-Pra-OMe·HCl, H-Pro-Gly-OH, H-Pro-Hyp-OH, H-Pro-NH2, H-Pro-NHEt·HCl, H-Pro-NMe2, H-Pro-OBzl·HCl, H-Pro-OH, H-Pro-Oipr·HCl, H-Pro-OMe HCl, H-Pro-OtBu, H-Pro-pNA HCl, H-Pyr-OEt, H-Pyr-OEt HCl, H-Pyr-OH, H-Pyr-OtBu, H-Sar-NH2·HCl, H-Sar-OEt·HCl, H-Sar-OMe HCl, H-Sar-OtBu·HCl, H-Ser(Ac)-OH, H-Ser(Bzl)-OBzl HCl, H-Ser(Bzl)-OH, H-Ser(Bzl)-OH HCl, H-Ser(Bzl)-OMe HCl, H-Ser(tBu)-NH2·HCl, H-Ser(tBu)-OBzl·HCl, H-Ser(tBu)-OH, H-Ser(tBu)-OMe·HCl, H-Ser-NH2·HCl, H-Ser-NHMe, H-Ser-OBzl HCl, H-Ser-OEt HCl, H-Ser-OH, H-Ser-OMe·HCl, H-Ser-OtBu·HCl, H-Thr(Bzl)-OBzl·HCl, H-Thr(Bzl)-OBzl·oxalate, H-Thr(Bzl)-OH-HCI, H-Thr(Me)-OH, H-Thr(tBu)-NH2·HCl, H-Thr(tBu)-OH, H-Thr(tBu)-OMe HCl, H-Thr(tBu)-OtBu, H-Thr(tBu)-OtBu·AcOH, H-Thr(tBu)-OtBu·HCl, H-Thr-OBzl, H-Thr-OBzl·HCl, H-Thr-OBzl·oxalate, H-Thr-OH, H-Thr-OMe, H-Thr-OMe·HCl, H-Thr-OtBu, H-Thr-OtBu·HCl, H-Tle-OH, H-Tle-OMe·HCl, H-Tle-OtBu·HCl, H-Trp(Boc)-OH, H-Trp-AMC·2HCl, H-Trp-NH2·HCl, H-Trp-OBzl·HCl, H-Trp-OEt·HCl, H-Trp-OH, H-Trp-OMe·HCl, H-Tyr(3,5-DiI)-OH, H-Tyr(3,5-DiNO2)-OH, H-Tyr(35-DiBr)-OH·2H2O, H-Tyr(35-DiCl)-OH, H-Tyr(3-Cl)-OH, H-Tyr(3-I)-OH, H-Tyr(3-NH2)-OH·2HCl, H-Tyr(3-NO2)-OH, H-Tyr(3-NO24-SO3H)-OH, H-Tyr(Ac)-OH, H-Tyr(Bzl)-OBzl·HCl, H-Tyr(Bzl)-OH, H-Tyr(Bzl)-OMe, H-Tyr(Bzl)-OMe·HCl, H-Tyr(H2PO3)-OH, H-Tyr(Me)-OH, H-Tyr(Propargyl)-OH, H-Tyr(tBu)-NH2, H-Tyr(tBu)-OH, H-Tyr(tBu)-OMe·HCl, H-Tyr(tBu)-OtBu·HCl, H-Tyr(Tos)-OH, H-Tyr-NH2, H-Tyr-NH2·HCl, H-Tyr-OBzl, H-Tyr-OBzl·HCl, H-Tyr-OBz·TosOH, H-Tyr-OEt·HCl, H-Tyr-OH, H-Tyr-OMe, H-Tyr-OMe·HO, H-Tyr-OtBu, H-Tyr-pNA, H-Val-Ala-OH, H-Val-Ala-OH·HCl, H-Val-NH2·HCl, H-Val-OBzl·HCl, H-Val-OBzl·TosOH, H-Val-OEt HCl, H-Val-OH, H-Val-Oipr·HCl, H-Val-OMe·HCl, H-Val-OtBu·HCl, H-Val-pNA, H-Val-Trp-OH, H-β-Ala-NH2·HCl, H-β-Ala-OBzl·TosOH, H-β-Ala-OEt·HCl, H-β-Ala-OH, H-p-Ala-OMe·HCl, H-β-Ala-OtBu·HCl, H-β-HoAla-OH·HCl, H-p-HoAsp·HCl, H-β-HoGln-OH·HCl, H-β-HoGlu-OH·HCl, H-β-HoIle-OH·HCl, H-β-HoLeu-OH·HCl, H-β-HoPhe-OH, H-β-HoVal-OH, H-γ-Abu-OBzl·TosOH, H-γ-Abu-OMe·HCl, H-γ-Abu-OtBu·HCl, Ivdde-Lys(Boc)-OH, L-Alaninol, L-Cysteinol(Bzl), L-Cysteinol(pMeBzl), L-Homoserine lactone, L-Isoleucinol, L-Leucinol(oil), L-Methioninol, L-Norvalinol, L-Phenylalaninol, L-Phenylglycinol, L-Prolinol, L-Serinol(Bzl), L-Threoninol, L-Threoninol(Bzl), L-Threoninol(Bzl) HCl, L-Tryptophanol, L-Tyrosinol, L-Tyrosinol·HCl, L-Valinol, Moc-Val-OH, Mpa(Acm), Mpa(Bzl), Mpa(MMt)-OH, Mpa(Trt), Mpa(Trt)-OSu, N-Boc-cis-4-hydroxy-D-Proline, N-Formyl-Leu-OH, NH2-NTA(Me)3·HBr, N-Phthaloyl-Phenylalanine, Pal-Glu(OtBu)-OH, Pal-Glu-OtBu, Pbf-NH2, PhC3H6-Lys(Boc)-OH, Pht-Dopa-OH, Tfa-Gly-OH, Thioanisole, Tos-Ala-OH, Tos-Arg-OH, Tos-Arg-OMe HCl, Tos-D-Pro-OH, Tos-D-Val-OH, Tos-Gly-OMe, Tos-Lys(Boc)-OH, Tos-Phe-OH, Tos-Pro-OH, Tos-Val-OH, Trans-4-hydroxy-L-prolinol·hydrochloride, Trt-Cys(Trt)-OH·DBA, Trt-Cys(Trt)-OSu, Trt-D-Cys(Trt)-OH DBA, Trt-D-Ser-OH, Trt-Gly-OH, Trt-Ser-OH, Trt-Ser-OMe, Trt-Thr-OH DBA, Z(2-Br)-OSu, Z(4-NO2)-OSu, Z-Abu-OH, Z-Aib-OH, Z-Ala-Ala-OH, Z-Ala-Gly-OH, Z-Ala-NH2, Z-Ala-OH, Z-Ala-OMe, Z-Ala-OSu, Z-Ala-Trp-OH, Z-Arg(Mbs)-OH . DCHA, Z-Arg(Mtr)-OH·CHA, Z-Arg(NO2)-OH, Z-Arg(Pbf)-OH·CHA, Z-Arg(Pbf)-OH DCHA, Z-Arg(Z)2-OH, Z-Arg-OH, Z-Arg-OH·HBr, Z-Arg-OH·HCl, Z-Asn(Trt)-OH, Z-Asn-OH, Z-Asn-ONp, Z-Asp(OBzl)-OH, Z-Asp(OBzl)-OSu, Z-Asp(OMe)-OH, Z-Asp(OMe)-OtBu, Z-Asp(OtBu)-OBzl, Z-Asp(OtBu)-OH DCHA, Z-Asp(OtBu)-OH·H2O, Z-Asp(OtBu)-OMe, Z-Asp(OtBu)-OSu, Z-Asp-OBzl, Z-Asp-OH, Z-Asp-OMe, Z-Asp-OMPe, Z-Asp-OtBu, Z-Asp-OtBu·DCHA, Z-Cha-OH, Z-Cha-OH DCHA, Z-Chg-OH, Z-Cys(pMeOBzI)-OH, Z-Cys(Trt)-OH, Z-Cys(Z)-OH, Z-D-2-Nal-OH, Z-D-Abu-OH, Z-D-Ala-Gly-OH, Z-D-Ala-NH2, Z-D-Alaninol, Z-D-Ala-OH, Z-Dap(Boc)-OH, Z-Dap(Fmoc)-OH, Z-Dap-OH, Z-D-Arg(Mtr)-OH-CHA, Z-D-Arg(Pbf)-OH CHA, Z-D-Arg-OH, Z-D-Arg-OH·HCl, Z-D-Asn(Trt)-OH, Z-D-Asn-OH, Z-D-Asp(OtBu)-OH·H2O, Z-D-Asp-OH, Z-D-Asp-OMe, Z-D-Cha-OH, Z-D-Chg-OH, Z-D-Dap(Boc)-OH, Z-D-Dap(Boc)-ol, Z-D-Dap-OH, Z-D-Gln-OH, Z-D-Glu(OBzl)-OH, Z-D-Glu(OtBu)-OH, Z-D-Glu-OBzl, Z-D-Glu-OEt, Z-D-Glu-OH, Z-D-Glu-OMe, Z-D-His-OH, Z-DL-Ala-OH, Z-DL-Asn-OH, Z-DL-Asp-OH, Z-D-Leu-OH, Z-D-Leu-OH DCHA, Z-DL-Glu-OtBu, Z-DL-His-OH, Z-DL-Met-OH, Z-DL-Nva-OH, Z-DL-Phe(4-Cl)-OH, Z-DL-Val-OH, Z-D-Lys(Boc)-OH, Z-D-Lys(Boc)-OH·DCHA, Z-D-Lys(Boc)-OSu, Z-D-Lys-OH, Z-D-Met-OH, Z-D-N-Me-Val-OH, Z-D-Nva-OH, Z-D-Orn-OH, Z-D-Phe(4-F)-OH, Z-D-Phenylalaninol, Z-D-Phe-OH, Z-D-Phg-OH, Z-D-Pro-OH, Z-D-Pyr-OH, Z-D-Ser(tBu)-OH, Z-D-Ser(tBu)-OMe, Z-D-Ser-OH, Z-D-Ser-OMe, Z-D-Thr-OH, Z-D-Thr-OMe, Z-D-Trp(Boc)-OH, Z-D-Trp(Boc)-OH DCHA, Z-D-Trp-OH, Z-D-Trp-OSu, Z-D-Tyr(Bzl)-OH, Z-D-Tyr(tBu)-OH-DCHA, Z-D-Tyr-OH, Z-D-Val-OH, Z-Gln(Trt)-OH, Z-Gln-OH, Z-Gln-OMe, Z-Gln-ONp, Z-Glu(OBzl)-OH, Z-Glu(OBzl)-OH·DCHA, Z-Glu(OSu)-OBzl, Z-Glu(OtBu)-OBzl, Z-Glu(OtBu)-OH, Z-Glu(OtBu)-OH·DCHA, Z-Glu(OtBu)-OMe, Z-Glu(OtBu)-OSu, Z-Glu-OBzl, Z-Glu-OBzl·DCHA, Z-Glu-OH, Z-Glu-OMe, Z-Glu-OtBu, Z-Glycinol, Z-Gly-NH2, Z-Gly-OH, Z-Gly-OMe, Z-Gly-OSu, Z-Gly-Phe-NH2, Z-Gly-Pro-OH, Z-His(Dnp)-OH, Z-His(Trt)-OH, Z-His(Z)-OH·EtOH, Z-His-OH, Z-His-OMe, Z-HoArg(NO2)-OH, Z-HoArg-OH, Z-HoSer-OH, Z-Hyp(tBu)-OMe, Z-Hyp-OH, Z-Hyp-OMe, Z-Ile-OH, Z-Ile-OSu, Z-L-2-Nal-OH, Z-Leu-Leu-OH, Z-Leu-OH, Z-Leu-OH·DCHA, Z-Lys(Boc)(Isopropyl)-OH DCHA, Z-Lys(Boc)-OH, Z-Lys(Boc)-ONp, Z-Lys(Boc)-OSu, Z-Lys(For)-OH, Z-Lys(Tfa)-OH, Z-Lys(Z)-OH, Z-Lys(Z)-OSu, Z-Lys-OH, Z-Lys-OMe·HCl, Z-Met-OH, Z-Met-OMe, Z-N-Me-Ala-OH, Z-N-Me-Glu(OtBu)-OH, Z-N-Me-Ile-OH, Z-N-Me-Phe-OH, Z-N-Me-Ser-OH, Z-N-Me-Val-OH, Z-Nva-OH, Z-Om(Alloc)-OH·DCHA, Z-Orn(Boc)-OH, Z-Om(Z)-OH DCHA, Z-Orn-OH, Z-Om-OH·HCl, Z-Phe(4-F)-OH, Z-Phe-NH2, Z-Phenylalaninol, Z-Phe-OH, Z-Phe-OMe, Z-Phe-OSu, Z-Phg-OH, Z-Pra-OH, Z-Prolinol, Z-Pro-NH2, Z-Pro-OH, Z-Pro-OSu, Z-Pyr-OH, Z-Pyr-OSu, Z-Pyr-OtBu, Z-Sar-NH2, Z-Sar-OH, Z-Ser(Bzl)-OH, Z-Ser(TBDMS)-OH, Z-Ser(tBu)-NH2, Z-Ser(tBu)-OH, Z-Ser(tBu)-OMe, Z-Ser(Tos)-OMe, Z-Ser(Trt)-OH, Z-Ser-NH2, Z-Ser-NHNH2, Z-Ser-OBzl, Z-Ser-OH, Z-Ser-OMe, Z-Thr(Me)-OH, Z-Thr(tBu)-OH, Z-Thr(tBu)-OH DCHA, Z-Threoninol, Z-Thr-NH2, Z-Thr-OBzl, Z-Thr-OH, Z-Tbr-OMe, Z-Tic-OH, Z-Tle-OH, Z-Tle-OH·DCHA, Z-Trp(Boc)-OH, Z-Trp(Boc)-OH·DCHA, Z-Trp-OBzl, Z-Trp-OH, Z-Trp-OMe, Z-Tyr(Bzl)-OH, Z-Tyr(tBu)-OH, Z-Tyr(tBu)-OH·DCHA, Z-Tyr(tBu)-OMe, Z-Tyr-OH, Z-Tyr-OMe, Z-Tyr-OtBu·H2O, Z-Tyr-Tyr-OH, Z-Val-Ala-OH, Z-Val-NH2, Z-Val-OEt, Z-Val-OH, Z-Val-OSu, Z-Val-Ser-OH, Z-β-Ala-OH, Z-β-Ala-OSu, Z-γ-Abu-OH or Z-ε-Acp-OH.

In the present disclosure, the "pyrrolysine (Pyl; O)" is an amino acid that may be represented by the formula C₁₂H₂₁N₃O₃ and is used in some methanogenic archaea.

In the present disclosure, the "theanine (gamma-glutamylethylamide)" may be represented by the formula C₇H₁₄N₂O₃ and exists in two isomeric forms (L-theanine and D-theanine). L-theanine is an amino acid found in the leaves of Gyokuro.

In the present disclosure, the "gamma-glutamylmethylamide (GMA)" is an amino acid that may be represented by the formula C₆H₁₂N₂O₃.

In the present disclosure, the "beta-aminobutyric acid (BABA)" and "gamma-aminobutyric acid (GABA)" are amino acid analogs that may be represented by the formula C₄H₉NO₂, and are isomers of each other.

In the present disclosure, the "monosaccharide" is the most basic unit of carbohydrate that is not decomposed into a simpler compound by hydrolysis, and may be glucose, fructose or lactose, or an isomer thereof. However, the monosaccharide may include, without limitation, any monosaccharide that may form a polysaccharide by an O-glycosidic bond.

In the present disclosure, the term "disaccharide" refers to a combination of two monosaccharides, such as sucrose, lactose, and maltose, and the term "oligosaccharide" refers to a combination of 2 to 10 monosaccharides, and the term "polysaccharide" refers to a combination of many monosaccharides. These terms may be used interchangeably and may include, without limitation, any polymer in which monosaccharides are linked together by an O-glycosidic bond.

In the present disclosure, two adjacent M and M among the plurality of M may be linked together by a pH-specifically or catalyst-specifically cleavable bond to form a polymer represented by, for example, "MM...M". The linkage may be achieved by a disulfide bond, an esterification reaction, a peptide coupling reaction, a Claisen condensation reaction, an aldol condensation reaction, or a glycosidic coupling reaction, but is not limited thereto. In the present disclosure, for the linkage, each M unit compound may have two or more functional groups therein.

In the present disclosure, the "disulfide bond" is a covalent bond formed between thiol groups (-SH), is represented by the formula R-S-S-R, and is also called a disulfide bridge. For example, the disulfide bond may be formed between cysteine units, but may include, without limitation, any disulfide bond that is formed between units having a thiol group.

In the present disclosure, the "ester reaction" is a generic term for a reaction in which an alcohol or phenol reacts with an organic acid or an inorganic acid and condenses with the loss of water.

In the present disclosure, the "peptide bond" or "amide linkage" is a covalent bond (-CO-NH-) formed between a carboxyl group (-COOH) and an amino group (NH₂-) by a chemical reaction. During the reaction, a dehydration reaction occurs in which a water molecule is formed. Through this process, the peptide has an N-terminus with an amino group and a C-terminus with a carboxyl group, which may indicate the directionality of the peptide.

In the present disclosure, M may be represented by the following Formula 2, but is not limited thereto:

[Formula 2] (X₁X₂...Xₘ)

wherein

m is an integer ranging from 1 to 100000, preferably from 2 to 50000, more preferably from 2 to 10000; even more preferably from 2 to 5000, even more preferably from 2 to 1000, even more preferably 2 to 100;

X₁ to Xₘ are each independently a unit, non-limiting examples of which include amino acids, amino acid analogs, peptides, peptide analogs, monosaccharides or oligosaccharides.

In the present disclosure, when X₁ to Xₘ in Formula 2 are each independently an amino acid, an amino acid analog, a peptide or a peptide analog, X₁ may be an N-terminus and Xₘ may be a C-terminus, or the Xₘ may be an N-terminus and X₁ may be a C-terminus.

In the present disclosure, m in Formula 2 may be an integer ranging from 1 to 100, preferably from 2 to 100, more preferably from 2 to 50, even more preferably from 3 to 15. In this case, in detection and analysis, the retention time in chromatography may be prevented from excessively decreasing or excessively increasing, thus enabling rapid detection, and easy and accurate detection or measurement may be achieved even by a method such as mass spectrometry. On the other hand, when m exceeds 100, the retention time during detection and analysis by chromatography may excessively increase, and thus an excessive amount of time may be taken for detection.

In the present disclosure, the "retention time (RT)" refers to the time from when a sample is added in chromatography to when the peak of the corresponding component appears.

In one example of the present disclosure, X₁ or Xₘ may be isoleucine, lysine, serine, arginine or threonine, preferably lysine or arginine, but may include, without limitation, any amino acid or amino acid analog that specifically reacts with a catalyst that cleaves the bond between the adjacent M and M among the plurality of M forming a polymer.

In another example of the present disclosure, X₂ to Xₘ₋₁ may be each independently any one selected from the group consisting of glycine, alanine, valine, leucine, isoleucine, threonine, serine, cysteine, aspartic acid, asparagine, glutamic acid, glutamine, phenylalanine, tyrosine, tryptophan and proline, but may include, without limitation, any amino acid or amino acid analog that does not react with a catalyst that cleaves the bond between adjacent M and M among the plurality of M forming a polymer.

In the present disclosure, the bond between adjacent M and M among the plurality of M forming a polymer may be cleaved by a catalyst, wherein the catalyst may be an enzyme or a synthetic catalyst.

In the present disclosure, the enzyme may be peptidase, preferably endopeptidase, or lactase, but is not limited thereto.

In the present disclosure, the "peptidase (protease or proteinase)" is an enzyme that catalyzes the hydrolysis of a peptide bond. An enzyme that acts on the N-terminus or C-terminus of a peptide chain to liberate amino acids in the order of binding is referred to as exopeptidase, and an enzyme that acts on a peptide bond inside a peptide chain is referred to as endopeptidase. The peptidase may be used to specifically hydrolyze only the peptide bond of a specific amino acid.

In the present disclosure, the peptidase may be at least one selected from the group consisting of trypsin, chymotrypsin, thrombin, plasmin, subtilisin, thermolysin, pepsin, and glutamyl endopeptidase. Preferably, the peptidase may be at least one selected from the group consisting of trypsin, chymotrypsin, subtilisin, thermolysin, and glutamyl endopeptidase, but is not limited thereto.

In the present disclosure, using the synthetic catalyst, an efficient cleavage reaction may be performed without being restricted by conditions such as pH or temperature.

In the present disclosure, the synthetic catalyst may be, but is not limited to, an artificial metalloprotease, an organic artificial protease, or a reducing agent that cleaves a disulfide bond.

In the present disclosure, examples of the artificial metalloprotease include, but are not limited to, water-soluble catalysts comprising copper (II), cobalt (III), iron (III), palladium (II), cerium (IV) or the like as the catalyst center, or catalysts comprising a copper (II) complex compound attached to a support.

In the present disclosure, examples of the organic artificial protease include, but are not limited to, those comprising a functional group attached to a silica support or a polystyrene support.

In the present disclosure, the reducing agent that cleaves a disulfide bond may be glutathione, thioglycolic acid, or cysteamine, but may include, without limitation, any reducing agent that may reduce the disulfide bond between adjacent M and M to a thiol group.

In the present disclosure, the first binding moiety is a substance capable of detecting or quantifying the analyte by direct or indirect binding to the analyte, and may include, without limitation, any substance that is capable of binding specifically and non-specifically to the analyte.

In the present disclosure, the first binding moiety may comprise at least one selected from the group consisting of a compound, a probe, an antisense nucleotide, an antibody, an oligopeptide, a ligand, PNA (peptide nucleic acid) and an aptamer, which bind specifically to the analyte, but is not limited thereto.

In the present disclosure, the "probe" refers to a substance which is capable of binding specifically to the analyte to be detected in a sample and may specifically identify the presence of the analyte in the sample through the binding. The kind of the probe is not specifically limited, as long as it is a substance that is generally used in the art. Preferably, the probe may be PNA (peptide nucleic acid), LNA (locked nucleic acid), a peptide, a polypeptide, a protein, RNA or DNA. More preferably, the probe is PNA. More specifically, the probe may comprise a biomaterial derived from an organism, an analogue thereof, or a material produced *ex vivo,* and examples thereof include enzymes, proteins, antibodies, microorganisms, animal/plant cells and organs, neural cells, DNA, and RNA. Examples of the DNA include cDNA, genomic DNA, and oligonucleotides, examples of the RNA include genomic RNA, mRNA, and oligonucleotides, and examples of the protein include antibodies, antigens, enzymes, and peptides.

In the present disclosure, the "locked nucleic acid (LNA)" refers to a nucleic acid analog comprising a 2'-O or 4'-C methylene bridge [J Weiler, J Hunziker and J Hall Gene Therapy (2006) 13, 496.502]. LNA nucleosides include common nucleic acid bases of DNA and RNA, and can form base pairs according to the Watson-Crick base pairing rule. However, due to 'locking' of the molecule attributable to the methylene bridge, the LNA fails to form an ideal shape in the Watson-Crick bond. When the LNA is incorporated in a DNA or RNA oligonucleotide, it can more rapidly pair with a complementary nucleotide chain, thus increasing the stability of the double strand.

In the present disclosure, the "antisense" refers to an oligomer having a sequence of nucleotide bases and a subunit-to-subunit backbone that allows the antisense oligomer to hybridize to a target sequence in an RNA by Watson-Crick base pairing, to form an RNA:oligomer heteroduplex within the target sequence, typically with an mRNA. The oligomer may have exact sequence complementarity to the target sequence or near complementarity.

In the present disclosure, when information on the sequence of the gene of the analyte is known, any person skilled in the art may easily design the primer, probe or antisense nucleotide that binds specifically to the gene, based on this information.

In the present disclosure, the "antibody (Ab)" refers to a substance that binds specifically to an antigen, causing an antigen-antibody reaction. With regard to the purposes of the present disclosure, the antibody refers to an antibody that binds specifically to the analyte.

In the present disclosure, examples of the antibody include all polyclonal antibodies, monoclonal antibodies, and recombinant antibodies. The antibody may be easily produced using techniques well known in the art. For example, the polyclonal antibody may be produced by a method well known in the art, which comprises a process of injecting the protein antigen into an animal, collecting blood from the animal, and isolating serum comprising the antibody. This polyclonal antibody may be produced from any animal species such as goats, rabbits, sheep, monkeys, horses, pigs, cattle, or dogs. In addition, the monoclonal antibody may be produced using a hybridoma method (see Kohler and Milstein (1976) European Journal of Immunology 6:511-519) well known in the art, or phage antibody library technology (see Clackson et al, Nature, 352:624-628, 1991; Marks et al, J. Mol. Biol., 222:58, 1-597, 1991). The antibody produced by the above method may be isolated and purified using methods such as gel electrophoresis, dialysis, salt precipitation, ion exchange chromatography, and affinity chromatography. In addition, the antibodies of the present disclosure include functional fragments of antibody molecules as well as complete forms having two full-length light chains and two full-length heavy chains. The expression "functional fragments of antibody molecules" refers to fragments retaining at least an antigen-binding function, and examples of the functional fragments include Fab, F(ab'), F(ab')2, and Fv.

In the present disclosure, the "peptide nucleic acid (PNA)" refers to an artificially synthesized polymer similar to DNA or RNA, and was first introduced by professors Nielsen, Egholm, Berg and Buchardt (at the University of Copenhagen, Denmark) in 1991. DNA has a phosphate-ribose backbone, whereas PNA has a backbone composed of repeating units of N-(2-aminoethyl)-glycine linked by peptide bonds. Thanks to this structure, PNA has a significantly increased binding affinity for DNA or RNA and a significantly increased stability, and thus is used in molecular biology, diagnostic analysis, and antisense therapy. PNA is disclosed in detail in Nielsen PE, Egholm M, Berg RH, Buchardt O (December 1991). "Sequence-selective recognition of DNA by strand displacement with a thymine-substituted polyamide". Science 254 (5037): 1497-1500.

In the present disclosure, the "aptamer" is an oligonucleic acid or peptide molecule, and general contents of the aptamer are disclosed in detail in Bock LC et al., Nature 355(6360):5646(1992); Hoppe-Seyler F, Butz K "Peptide aptamers: powerful new tools for molecular medicine". J Mol Med. 78(8):42630(2000); Cohen BA, Colas P, Brent R. "An artificial cell-cycle inhibitor isolated from a combinatorial library". Proc Natl Acad Sci USA. 95(24): 142727(1998).

In the present disclosure, the first binding moiety may comprise, but is not limited to, at least one compound selected from the group consisting of the following Chemical Formulas 1 to 5, which may bind non-specifically to the analyte:

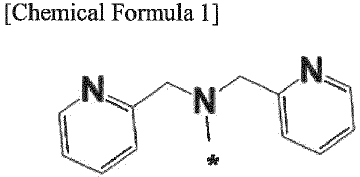

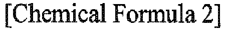

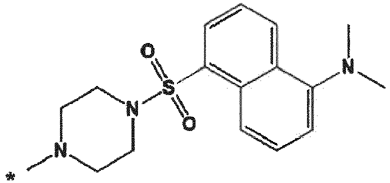

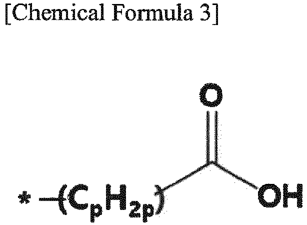

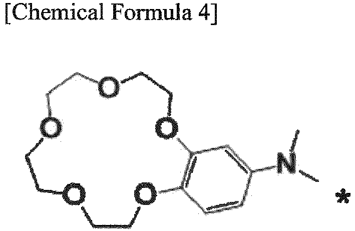

[Chemical Formula 5] **S=C=N- ***

wherein
p is an integer ranging from 7 to 20, and
* is a portion linked to [M]ₙ or L₁.

In the first binding moiety of the present disclosure, the compound represented by Chemical Formula 1, 2 or 4 may indirectly bind to the analyte through copper ions (Cu²⁺), zinc ions (Zn²⁺) or cobalt ions (Co²⁺).

In the present disclosure, any one residue of the plurality of M forming the polymer represented by Formula 2 may be linked directly or through a linker to the first binding moiety.

In the present disclosure, the "linker" refers to one that cross-links one compound with another compound, wherein the cross-linking may be achieved either by a chemical bond such as a covalent bond or by a physical bond such as an ionic bond. In the cross-linking process, a protecting group may be introduced.

In the present disclosure, the linker may comprise any one or more selected from among the following Chemical Formulas 6 to 8, but may include, without limitation, any linker that is used in the technology of producing small-molecule drug conjugates (SMDC) such as antibody-drug conjugates (ADCs) or ligand-drug conjugates (LDCs):

[Chemical Formula 6] *-C_{q}H_{2q}-*

[Chemical Formula 7] *-C_{q}H_{2q}COO-*

[Chemical Formula 8] *-H₂NCOC_{q}H_{2q}S-*

wherein
q is an integer ranging from 1 to 5; and
* signifies a portion linked to [M]ₙ or L₁.

In the present disclosure, the "small-molecule drug conjugate (SMDC)" is composed of three modules, including a targeting means such as a ligand or antibody, a linker, and a loaded drug, and is a technology used for drug delivery.

In the present disclosure, the complex compound represented by Formula 1 may further comprise a spacer between [M]ₙ and the linker (L₁) or between the linker (Li) and the first binding moiety (Ni).

In the present disclosure, the "spacer" is also referred to as a stretcher, provides linkage between the first binding moiety and the linker or between the linker and the polymer, and ensures a space between the first binding moiety and the polymer, and is cleavable by a catalyst, and may be made of an amino acid or an oligopeptide, but is not limited thereto.

In the present disclosure, the complex compound represented by Formula 1 may be represented by any one of the following Chemical Formulas 9 to 13, but is not limited thereto:

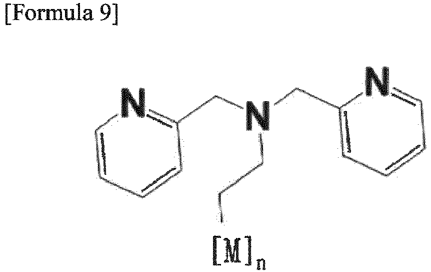

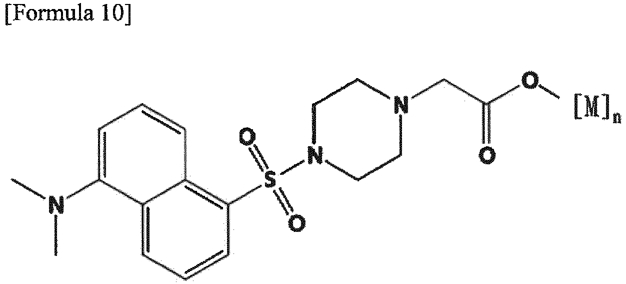

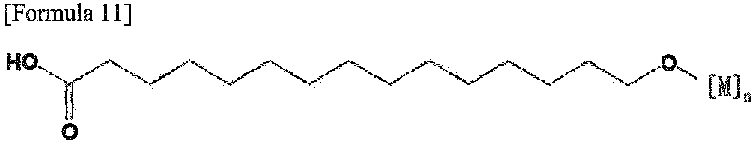

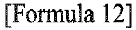

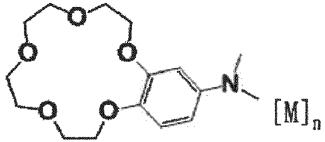

[Formula 13] **S=C=N-**[M]ₙ

wherein n and M are as defined in Formula 1 above.

In the present disclosure, the composition for detecting or measuring an analyte may comprise one complex compound represented by Formula 1, or may comprise two or more different complex compounds represented by Formula 1. In the latter case, at least one of the polymer, the linker and the first binding moiety may be different between the different complex compounds. In particular, the sequence "(X₁X₂...Xₘ)" represented by Formula 2 above may differ between the different complex compounds, or the polymerization number of M, that is, n in Formula 1, may differ between the different complex compounds.

Also, in the present disclosure, the composition for detecting or measuring an analyte may be composed of two or more compositions comprising different complex compounds represented by Formula 1. In this case, there is an advantage in that it is possible to perform analysis of multiple analytes, multiple subjects or multiple samples through only one analysis process by using different complex compounds for multiple analytes, respectively, or using compositions comprising different complex compounds for samples obtained from multiple subjects, respectively, or using compositions comprising different complex compounds for multiple samples obtained from a single subject, respectively.

According to another embodiment of the present disclosure, the present disclosure is directed to a kit for detecting or measuring an analyte, the kit comprising the composition for detecting or measuring an analyte according to the present disclosure.

In the present disclosure, the kit may be a protein chip kit, a rapid kit, or a multiple-reaction monitoring (MRM) kit, but is not limited thereto.

In the present disclosure, the kit may further comprise one or more other components, solutions or devices suitable for analysis methods, such as a second binding moiety, an immobilization support, a carrier, biotin, a washing solution or a reaction solution.

In the present disclosure, the kit may further comprise a second binding moiety that binds specifically to the analyte, has high affinity for the analyte, and has little cross-reactivity with other biomarkers.

In the present disclosure, the second binding moiety may comprise at least one selected from the group consisting of a probe, an antisense nucleotide, an antibody, an oligopeptide, a ligand, PNA (peptide nucleic acid) and an aptamer, which bind specifically to the analyte, but is not limited thereto.

In addition, in the present disclosure, the kit may comprise two or more different second binding moieties. In particular, when the composition for detecting or measuring an analyte comprises two or more different complex compounds represented by Formula 1, the kit may comprise two or more different second binding moieties so that the different second binding moieties correspond to the different complex compounds, respectively.

In addition, in the present disclosure, the second binding moiety may be bound to an immobilization support, a carrier or biotin.

In the present disclosure, the material of the immobilization support may be any one or more selected from among nitrocellulose, PVDF, polyvinyl resin, polystyrene resin, glass, silicone and a metal, and the immobilization support may be in the form of a membrane, a substrate, a plate, a well plate, a multi-well plate, a filter, a cartridge, a column or a porous body. However, the immobilization support may include, without limitation, any immobilization support that immobilizes the second binding moiety in two dimensions.

In the present disclosure, the carrier may be any material that has a three-dimensional structure and immobilizes the second binding moiety in three dimensions. Preferably, the carrier may be, but is not limited to, a material, for example, magnetic particles, which may be easily separated or recovered by weight, electric charge or magnetism. In the present disclosure, the magnetic particles are not particularly limited in kind, but may be made of one or more materials selected from the group consisting of iron, cobalt, nickel, and oxides or alloys thereof. Examples of the magnetic particles may include iron oxide (Fe₂O₃ or Fe₃O₄), ferrite (a form in which one Fe in Fe₃O₄ is replaced with another magnetism-related atom; e.g., CoFe₂O₄ or MₙFe₂O₄), and/or an alloy (alloyed with a noble metal to overcome the oxidation problem caused by magnetic atoms and to increase conductivity and stability; e.g., FePt, CoPt, etc.). Specific examples of the magnetic particles include, but are not limited to, maghemite (γ-Fe₂O₃), magnetite (Fe₃O₄), cobalt ferrite (CoFe₂O₄), manganese ferrite (MnFe₂C₄), an iron-platinum alloy (FePt alloy), an iron-cobalt alloy (FeCo alloy), a cobalt-nickel alloy (CoNi alloy), or a cobalt-platinum alloy (CoPt alloy).

In the present disclosure, the biotin may be bound to a streptavidin or avidin protein bound to the immobilization support or carrier.

In the present disclosure, the washing solution may include a phosphate buffered saline, NaCl, or a nonionic surfactant. Preferably, the washing solution may be, but is not limited to, a phosphate-buffered saline with Tween 20 (PBST), which is composed of 0.02 M phosphate buffered saline, 0.13 M NaCl and 0.05% Tween 20. The nonionic surfactant may be selected from the group consisting of digitoninum, Triton X-100, Triton X-114, Tween-20 and Tween-80, but is not limited thereto.

In the present disclosure, the reaction solution may comprise, but is not limited to, at least one metal salt selected from the group consisting of CuCl₂, Cu(NO₃)₂, CoCl₂, Co(NO₃)₂, Zn(NO₃)₂ and ZnCl₂, which react with the analyte.

In one example of the present disclosure, the second binding moiety may be a capture antibody. In this case, after the antigen-antibody reaction between the second binding moiety and the analyte, the immobilization support may be washed 3 to 6 times with the washing solution. Here, as the reaction stop solution, a sulfuric acid solution (H₂SO₄) may preferably be used. The washing solution that is used in this case may be any one or more non-ionic surfactants selected from among digitoninum, Triton X-100, Triton X-114, Tween-20 and Tween-80, but is not limited thereto.

According to still another embodiment of the present disclosure, the present disclosure is directed to a method for analyzing an analyte, the method comprising: a reaction step of allowing the analyte to react with the composition for detecting or measuring an analyte according to the present disclosure; and a detection step of detecting or measuring M in the complex compound of the composition.

In the present disclosure, the analyte may be a substance that is present in a biological sample isolated from a subject of interest. For example, the analyte may comprise any one or more selected from the group consisting of proteins, lipoproteins, glycoproteins, DNA, and RNA. However, the analyte may comprise, without limitation, any biomolecule in which organic substances such as amino acids, nucleotides, monosaccharides or lipids are contained as monomers.

In the present disclosure, the "subject" may be one from which the biological sample comprising or expected to comprise the analyte is isolated. If the analyte present in a trace amount in the biological sample can be analyzed, it may be applied to early diagnosis of various diseases, prediction of prognosis of the diseases, and prediction of the responsiveness of the diseases to drugs.

In the present disclosure, the "biological sample" refers to any material, biological fluid, tissue or cells obtained from or derived from a subject. Examples of the biological sample may include whole blood, leukocytes, peripheral blood mononuclear cells, buffy coat, plasma, serum, sputum, tears, mucus, nasal washes, nasal aspirate, breath, urine, semen, saliva, peritoneal washings, ascites, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extract, or cerebrospinal fluid. Preferably, the biological sample may be whole blood, plasma, or serum.

In the present disclosure, before the reaction step is performed, an immobilization step of immobilizing the analyte by bringing the analyte into contact with the second binding moiety may be performed first.

In the present disclosure, the second binding moiety may comprise, but is not limited to, at least one selected from the group consisting of a probe, an antisense nucleotide, an antibody, an oligopeptide, a ligand, PNA (peptide nucleic acid) and an aptamer, which bind specifically to the analyte.

In the present disclosure, the second binding moiety may bind to an immobilization support, a carrier or biotin to form a second binding moiety-immobilization support conjugate or a second binding moiety-carrier conjugate.

In the present disclosure, the material of the immobilization support may be any one or more selected from among nitrocellulose, PVDF, polyvinyl resin, polystyrene resin, glass, silicone and a metal, and the immobilization support may be in the form of a membrane, a substrate, a plate, a well plate, a multi-well plate, a filter, a cartridge, a column or a porous body. However, the immobilization support may include, without limitation, any immobilization support that immobilizes the second binding moiety in two dimensions.

In the present disclosure, the carrier may be any material that has a three-dimensional structure and immobilizes the second binding moiety in three dimensions. Preferably, the carrier may be, but is not limited to, a material, for example, magnetic particles, which may be easily separated or recovered by weight, electric charge or magnetism. In the present disclosure, the magnetic particles are not particularly limited in kind, but may be made of one or more materials selected from the group consisting of iron, cobalt, nickel, and oxides or alloys thereof. Examples of the magnetic particles may include iron oxide (Fe₂O₃ or Fe₃O₄), ferrite (a form in which one Fe in Fe₃O₄ is replaced with another magnetism-related atom; e.g., CoFe₂O₄ or MnFe₂O₄), and/or an alloy (alloyed with a noble metal to overcome the oxidation problem caused by magnetic atoms and to increase conductivity and stability; e.g., FePt, CoPt, etc.). Specific examples of the magnetic particles include, but are not limited to, maghemite (γ-Fe₂O₃), magnetite (Fe₃O₄), cobalt ferrite (CoFe₂O₄), manganese ferrite (MnFe₂O₄), an iron-platinum alloy (FePt alloy), an iron-cobalt alloy (FeCo alloy), a cobalt-nickel alloy (CoNi alloy), or a cobalt-platinum alloy (CoPt alloy).

In the present disclosure, the biotin may bind to a streptavidin or avidin protein bound to the immobilization support or carrier to form a second binding moiety-immobilization support conjugate or a second binding moiety-carrier conjugate.

In the present disclosure, the method may, if necessary, further comprise, subsequent to the immobilization step, a first separation step of separating an analyte-second binding moiety conjugate, analyte-second binding moiety-immobilization support conjugate or analyte-second binding moiety-carrier conjugate formed by immobilization of the analyte.

In the present disclosure, in the first separation step, depending on depending on the properties of the second binding moiety or on the immobilization support, carrier or biotin to which the second binding moiety is bound, the analyte-second binding moiety conjugate, the analyte-second binding moiety-immobilization support conjugate or the analyte-second binding moiety-carrier conjugate may be separated by weight, charge, or magnetism.

In the present disclosure, the method may, if necessary, further comprise, subsequent to the first separation step, a first washing step of washing the analyte-second binding moiety conjugate, the analyte-second binding moiety-immobilization support conjugate or the analyte-second binding moiety-carrier conjugate with a washing solution.

In the present disclosure, portions of the biological sample, which are not immobilized without forming the conjugate, may be removed through the first washing step.

In the present disclosure, the washing solution that is used in the first washing step may include a phosphate buffer solution, NaCl, or a nonionic surfactant. Preferably, the washing solution may be, but is not limited to, a phosphate-buffered saline with Tween 20 (PBST), which is composed of 0.02 M phosphate buffered saline, 0.13 M NaCl and 0.05% Tween 20. The nonionic surfactant may be selected from the group consisting of digitoninum, Triton X-100, Triton X-114, Tween-20 and Tween-80, but is not limited thereto.

In the present disclosure, after the first washing step, a reaction step of allowing the analyte to react with the composition for detecting or measuring an analyte according to the present disclosure may be performed.

In the present disclosure, the composition for detecting or measuring an analyte according to the present disclosure, which is used in the reaction step, may comprise one complex compound represented by Formula 1, or may comprise two or more different complex compounds represented by Formula 1. In the latter case, at least one of M, the linker and the first binding moiety may be different the different complex compounds. In particular, the unit M sequence expressed as "(X₁X₂...Xₘ)" may differ between the different complex compounds, or the polymerization number of M, that is, n in Formula 1, may differ between the different complex compounds. In this case, there is an advantage in that it is possible to perform analysis of multiple analytes, multiple subjects or multiple samples through only one analysis process by using different complex compounds for multiple analytes, respectively, or using different complex compounds for samples obtained from multiple subjects, respectively, or using different complex compounds for multiple samples obtained from a single subject, respectively.

In the present disclosure, a metal salt may be additionally added during the reaction step, so that the first binding moiety can bind indirectly to the analyte through the metal ion of the metal salt. Preferably, the analyte may be first treated with the metal salt before treatment with the composition of the present disclosure.

In the present disclosure, the metal salt may be, but is not limited to, at least one selected from the group consisting of CuCl₂, Cu(NO₃)₂, CoCl₂, Co(NO₃)₂, Zn(NO₃)₂ and ZnCl₂.

In the present disclosure, the method may further comprise a second separation step of separating an [M]ₙ-L₁-N₁-analyte conjugate, [M]ₙ-L₁-N₁-analyte-second binding moiety conjugate, [M]ₙ-L₁-N₁-analyte-second binding moiety-immobilization support conjugate or [M]ₙ-L₁-N₁-analyte-second binding moiety-carrier conjugate formed as a result of the reaction step.

In the present disclosure, in the second separation step, depending on the properties of the second binding moiety or on the immobilization support, carrier or biotin to which the second binding moiety is bound, the [M]ₙ-L₁-N₁-analyte-second binding moiety-immobilization support conjugate or the [M]ₙ-L₁-N₁-analyte-second binding moiety-carrier conjugate may be separated by weight, charge or magnetism.

In the present disclosure, the method may, if necessary, further comprise, subsequent to the second separation step, a second washing step of washing the [M]ₙ-L₁-Ni-analyte-second binding moiety conjugate, the [M]ₙ-L₁-N₁-analyte-second binding moiety-immobilization support conjugate or the [M]ₙ-L₁-N₁-analyte-second binding moiety-carrier conjugate with a washing solution.

In the present disclosure, portions of the reaction composition, which are not immobilized without forming the conjugate, may be removed through the second washing step.

In the present disclosure, the washing solution that is used in the second washing step may include a phosphate buffer solution, NaCl or a non-ionic surfactant. Preferably, the washing solution may be, but is not limited to, a phosphate-buffered saline with Tween 20 (PBST), which is composed of 0.02 M phosphate buffered saline, 0.13 M NaCl and 0.05% Tween 20. The nonionic surfactant may be selected from the group consisting of digitoninum, Triton X-100, Triton X-114, Tween-20 and Tween-80, but is not limited thereto.

In the present disclosure, the method may further comprise a cleavage step of cleaving the M unit from the [M]ₙ-L₁-N₁-analyte conjugate, the [M]ₙ-L₁-N₁-analyte-second binding moiety conjugate, the [M]ₙ-L₁-N₁-analyte-second binding moiety-immobilization support conjugate or the [M]ₙ-L₁-N₁-analyte-second binding moiety-carrier conjugate.

The cleavage step in the present disclosure may be performed using a catalyst that specifically cleaves the bond between the adjacent M and M, wherein the catalyst may be an enzyme or a synthetic catalyst.

In the present disclosure, the enzyme may be peptidase, preferably endopeptidase, or lactase, but is not limited thereto.

In the present disclosure, only peptide bonds between specific amino acids may be specifically hydrolyzed using the peptidase.

In the present disclosure, the peptidase may be at least one selected from the group consisting of trypsin, chymotrypsin, thrombin, plasmin, subtilisin, thermolysin, pepsin, and glutamyl endopeptidase. Preferably, the peptidase may be at least one selected from the group consisting of trypsin, chymotrypsin, subtilisin, thermolysin, and glutamyl endopeptidase, but is not limited thereto.

In the present disclosure, using the synthetic catalyst, an efficient cleavage reaction may be performed without being restricted by conditions such as pH or temperature.

In the present disclosure, the synthetic catalyst may be, but is not limited to, an artificial metalloprotease, an organic artificial protease, or a reducing agent that cleaves a disulfide bond.

In the present disclosure, examples of the artificial metalloprotease include, but are not limited to, water-soluble catalysts comprising copper (II), cobalt (III), iron (III), palladium (II), cerium (IV) or the like as the catalyst center, or catalysts comprising a copper (II) complex compound attached to a support.

In the present disclosure, examples of the organic artificial protease include, but are not limited to, those comprising a functional group attached to a silica support or a polystyrene support.

In the present disclosure, the reducing agent that cleaves a disulfide bond may be glutathione, thioglycolic acid, or cysteamine, but may include, without limitation, any reducing agent that may reduce the disulfide bond between the adjacent M and M to a thiol group.

In the present disclosure, the cleavage step may be followed by a detection step of detecting or measuring the cleaved M.

In the present disclosure, when M is a peptide, the detection step may, if necessary, comprise quantifying n peptide fragments (units M) obtained by cleaving and fragmenting the peptide polymer represented by "[M]ₙ". In that case, the quantification sensitivity may be increased n times compared to the case in which the peptide polymer is quantified.

In the present disclosure, when M is a monosaccharide, an oligosaccharide or a polysaccharide, the detection step may, if necessary, comprise quantifying n monosaccharides, oligosaccharides or polysaccharides (units M) obtained by cleaving and fragmenting the oligosaccharide or polysaccharide polymer represented by "[M]ₙ" by lactase or under an acidic condition. In that case, the quantification sensitivity may be increased n times compared to the case in which the polymer is quantified.

In the present disclosure, a method that is used for the detection, quantification or comparative analysis of M in the detection step may comprise, but is not limited to, at least one selected from the group consisting of protein chip assay, immunoassay, ligand binding assay, MALDI-TOF (Matrix Assisted Laser Desorption/Ionization Time of Flight Mass Spectrometry) assay, SELDI-TOF (Surface Enhanced Laser Desorption/Ionization Time of Flight Mass Spectrometry) assay, radioimmunoassay, radioimmunodiffusion, Ouchterlony immunodiffusion, rocket immunoelectrophoresis, immunohistochemical staining, complement fixation assay, two-dimensional electrophoresis assay, liquid chromatography-mass spectrometry (LC-MS), LC-MS/MS (liquid chromatography-mass spectrometry/mass spectrometry), Western blotting, and multiple-reaction monitoring (MRM).

In the present disclosure, the multiple-reaction monitoring method may be performed using mass spectrometry, preferably triple-quadrupole mass spectrometry.

In the present disclosure, the multiple-reaction monitoring (MRM) method using mass spectrometry is an analysis technique capable of monitoring a change in concentration of a specific analyte by selectively isolating, detecting and quantifying the specific analyte. MRM is a method that can quantitatively and accurately measure multiple substances such as trace amounts of biomarkers present in a biological sample. In MRM, mother ions among the ion fragments generated in an ionization source are selectively transmitted to a collision tube by a first mass filter Q1. Then, the mother ions arriving at the collision tube collide with an internal collision gas, are fragmented to generate daughter ions which are then sent to a second mass filter Q2, where only characteristic ions are transmitted to a detection unit. Thus, MRM is an analysis method with high selectivity and sensitivity that can detect only information on a component of interest. The MRM method has advantages in that it is easy to simultaneously measure multiple small molecules, and it is possible to confirm the relative concentration difference of protein diagnostic marker candidates between a normal person and a patient without using an antibody. In addition, since the MRM method has excellent sensitivity and selectivity, it has been introduced for the analysis of complex proteins and peptides in blood, particularly in proteomic analysis using a mass spectrometer (Anderson L. et al., Mol CellProteomics, 5: 375-88, 2006; DeSouza, L. V. et al., Anal. Chem., 81: 3462-70, 2009).

According to the method of the present disclosure, instead of the complex protein in blood, the polymer represented by "[M]ₙ" or n units M cleaved therefrom are analyzed as analytes using the MRM method. Thus, the method of the present disclosure may not only have a significant effect on the speed, ease and accuracy of analysis, but also allow simultaneous analysis of multiple biological samples or multiple analytes.

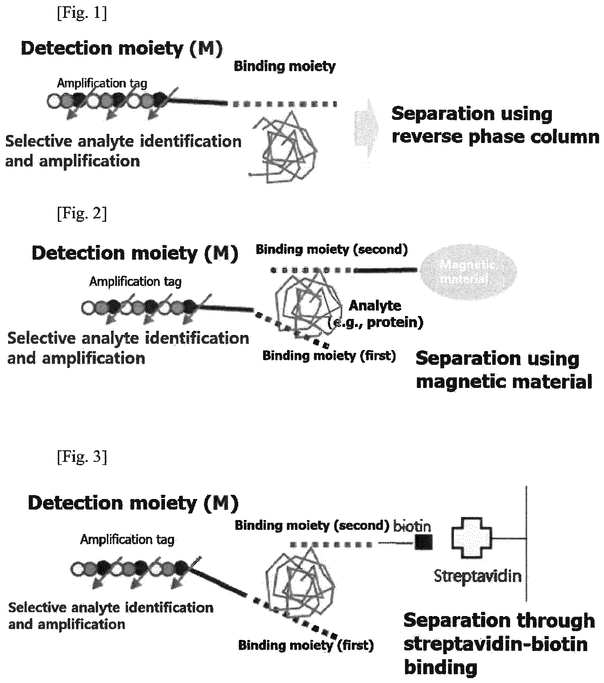

FIG. 1 is a schematic view showing a method for analyzing an analyte according to one example of the present disclosure. As shown therein, it is possible to quantitatively analyze an analyte with high sensitivity through the amplification effect resulting from the repetition of substances having the same mass to-charge ratio by 1) bringing a second binding moiety into contact with the analyte, and then immobilizing the analyte using a column such as a reversed-phase column or an ion exchange column, and then 2) removing impurities by washing, 3) allowing a conjugate of a repeatable peptide fragment, which is an amplification tag, and a first binding moiety capable of non-specifically binding to the analyte, to react with the immobilized analyte, and then 4) cleaving the peptide repeats contained in the conjugate into unit fragments by an enzyme, followed by mass spectrometry.

FIG. 2 is a schematic view showing a method for analyzing an analyte according to another example of the present disclosure. As shown therein, it is possible to quantitatively analyze an analyte with high sensitivity through the amplification effect resulting from the repetition of substances having the same mass to-charge ratio by 1) bringing the analyte into contact with a second binding moiety linked to magnetic particles, and then immobilizing the analyte by adjusting the magnetic force, and then 2) removing impurities by washing, 3) allowing a conjugate of a repeatable peptide fragment, which is an amplification tag, and a first binding moiety capable of non-specifically binding to the analyte, to react with the immobilized analyte, and then 4) cleaving the peptide repeats contained in the conjugate into unit fragments by an enzyme, followed by mass spectrometry.

FIG. 3 is a schematic view showing a method for analyzing an analyte according to still another example of the present disclosure. As shown therein, it is possible to quantitatively analyze an analyte with high sensitivity through the amplification effect resulting from the repetition of substances having the same mass to-charge ratio by 1) bringing a second binding moiety linked to biotin into contact with the analyte, and then immobilizing the analyte by reaction with an immobilization support (container) immobilized with streptavidin, and then 2) removing impurities by washing, 3) allowing a conjugate of a repeatable peptide fragment, which is an amplification tag, and a first binding moiety capable of non-specifically binding to the analyte, to react with the immobilized analyte, and then 4) cleaving the peptide repeats contained in the conjugate into unit fragments by an enzyme, followed by mass spectrometry.

According to another aspect of the present disclosure, there is provided a method for diagnosing the onset of a disease in the subject using the composition for detecting or measuring an analyte by mass spectrometry of the present disclosure; or the kit for detecting or measuring an analyte comprising the composition for detecting or measuring an analyte of the present disclosure.

According to a specific embodiment of the present invention, the method for diagnosing of the present disclosure comprising the following steps:

a reaction step of allowing the analyte to react with the composition for detecting or measuring an analyte comprising a complex compound represented by Formula I of the present disclosure or the kit for detecting or measuring an analyte by mass spectrometry of the present disclosure; or the kit for detecting or measuring an analyte comprising the composition for detecting or measuring an analyte of the present disclosure; and

a detection step of detecting or measuring the repeatable unit compound M present in the complex compound included in the composition or kit.

According to a specific embodiment of the present invention, the analyte is the blood of a subject to be diagnosed. More specifically, the analyte is a peptide present in the blood of a subject to be diagnosed.

According to a specific embodiment of the present disclosure, the disease which can be diagnosed by the method for diagnosing of the present disclosure is cancer.

Since the composition for detecting or measuring, kit for detecting or measuring and detection step in the present disclosure have already been described above, descriptions thereof are omitted to avoid excessive redundancy.

### Advantageous Effects

According to the present disclosure, it is possible to quantify an analyte with excellent selectivity and sensitivity, and to produce an amplification effect. Furthermore, it is possible to process various analytes simultaneously or process a large amount of a sample, and thus the present disclosure has excellent analysis efficiency and performance.

In addition, according to the present disclosure, it is possible to control the retention time during detection of various analytes in a sample. Thus, it is possible to increase the ease of analysis by adjusting the analysis time or suitably allocating the retention time between samples.

### Brief Description of Drawings

FIGS. 1 to 3 are schematic views showing methods for analyzing an analyte according to examples of the present disclosure.
FIG. 4 shows a process for producing a detection sensor according to an embodiment of the present disclosure in Preparation Example 1.
FIG. 5 shows the results of confirming coupling by the Kaiser test according to an embodiment of the present disclosure in Preparation Example 1.
FIG. 6 shows a process for producing a detection sensor according to an embodiment of the present disclosure in Preparation Example 2.
FIG. 7 shows a process for producing a detection sensor according to an embodiment of the present disclosure in Preparation Example 3.
FIG. 8 shows an aptamer-MNP conjugate according to an embodiment of the present disclosure, produced in Preparation Example 4.
FIG. 9 shows a process for producing an aptamer-MNP conjugate according to an embodiment of the present disclosure in Preparation Example 4.
FIGS. 10 and 11 show the results of mass spectrometry of peptide units according to an embodiment of the present disclosure, produced in Preparation Example 6.
FIG. 12 shows units according to an embodiment of the present disclosure, synthesized in Preparation Example 7.
FIG. 13 shows M according to an embodiment of the present disclosure, synthesized in Preparation Example 7.
FIG. 14 shows M according to an embodiment of the present disclosure and units cleaved therefrom, obtained in Preparation Example 8.
FIG. 15 shows the results of mass spectrometry of peptides according to an embodiment of the present disclosure, produced in Experimental Example 1.
FIG. 16 shows the results of confirming the amplification effect of peptides according to an embodiment of the present disclosure in Experimental Example 2.
FIG. 17 shows the results of confirming the amplification effect of peptides according to an embodiment of the present disclosure in Experimental Example 2.
FIG. 18 shows the results of confirming the amplification effect of peptides according to an embodiment of the present disclosure on improvement in the sensitivity of detection during mass spectrometry in Experimental Example 2.
FIG. 19 shows the increased detection sensitivity in mass spectrometry at low repeats (FIG. 19a) and high repeats (e.g. over 100 repeats, FIG. 19b) when the peptides are amplified as in Experimental Example 3.
FIG. 20 shows a quantification method according to an embodiment of the present disclosure in Experimental Example 4.
FIG. 21 shows a magnetic field treatment method according to an embodiment of the present disclosure in Experimental Example 4.
FIG. 22 shows an [M]ₙ-L₁-N₁-analyte-second binding moiety-carrier conjugate according to an embodiment of the present disclosure in Experimental Example 4.
FIG. 23 shows the results of quantifying the expression levels of proteins 1 to 4 according to an embodiment of the present disclosure in Experimental Example 5.
FIG. 24 shows the structure of a complex compound according to an embodiment of the present disclosure, produced in Experimental Example 6.
FIG. 25 shows a method for mass spectrometry after cleavage into SLVPR fragments in a complex compound according to an embodiment of the present disclosure in Experimental Example 6.
FIG. 26 shows a method for fluorescence analysis using a complex compound according to an embodiment of the present disclosure in Experimental Example 6.
FIG. 27 graphically shows the change in sensitivity as a function of the concentration of an analyte in mass spectrometry performed using a complex compound according to an embodiment of the present disclosure in Experimental Example 6.
FIG. 28 graphically shows the change in sensitivity as a function of the concentration of an analyte in fluorescence analysis performed using a complex compound according to an embodiment of the present disclosure in Experimental Example 6.

### Mode for Invention

Hereinafter, the present disclosure will be described in detail with reference to examples. However, the following examples merely illustrate the present disclosure, and the scope of the present disclosure is not limited by the following examples.

### Examples

The meanings of the abbreviations used in the following Examples of the present disclosure are shown in Table 1 below.

**[Table 1]**

| Abbreviation | Meaning |
|---|---|
| A.A | Amino acid |
| ACN | Acetonitrile |
| AC2O | Acetic anhydride |
| Boc | Tert-butyloxycarbonyl |
| Wang resins | Wang resins |
| CuCl₂ | Copper chloride |
| DIC | N,N'-diisopropylcarbodiimide |
| DMAP | Dimethylaminopyridine |
| DMF | N,N'-dimethylformamide |
| DIPEA | Diisopropylethylamine |
| HOBt | N-hydroxybenzotriazole |
| HNA | 9-9-hydroxynonanoic acid |
| Fmoc | 9-fluorenylmethoxycarbonyl |
| MeOH | Methanol |
| TFA | Trifluoroacetic acid |
| PEG | Polyethylene glycol |

### [Preparation Example 1] Production of Detection Sensor of Chemical Formula 9

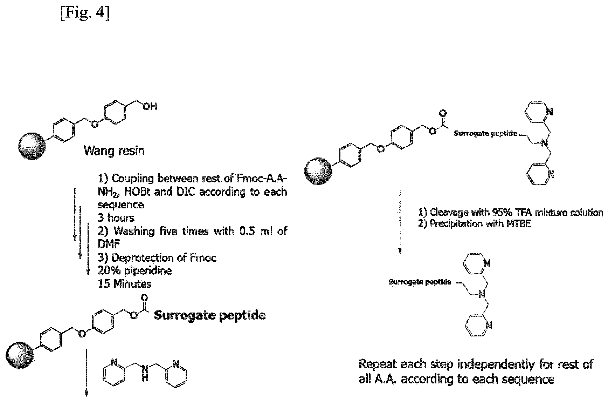

FIG. 4 shows a process of synthesizing a polymer (surrogate peptide), which is used to synthesize the complex compound represented by the following Chemical Formula 9 according to the present disclosure, and a process of linking the complex compound to a first binding moiety.

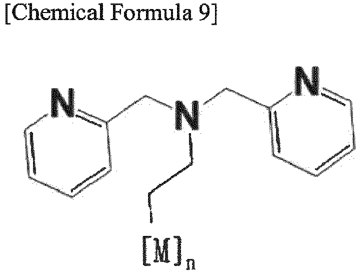

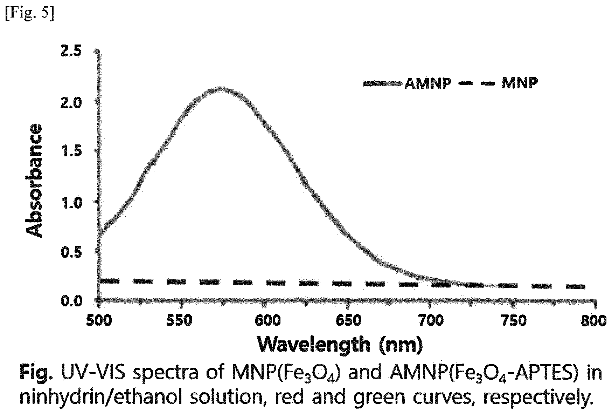

As shown in FIG. 4, for solid phase peptide synthesis, using Wang resin and EDCI synthesis, Fmoc-A.A-OH, HOBt and DIC were dissolved in DMF, and the solution was added to a reaction vessel and stirred. Capping of the unreacted sites of the resin was performed using AC2O. Deprotection of Fmoc was performed with piperidine. Similarly, Fmoc-A.A-OH, HOBt and DIC were dissolved in DMF, and the reaction solution was added to the reaction vessel and then stirred. Thereafter, the completion of coupling was monitored through the Kaiser test as shown in FIG. 5. Coupling of the rest of the amino acids in the sequence was performed using DIC/HOBt. Peptidyl resin was dried and taken for total cleavage. Peptidyl resin was treated with TFA at room temperature. After filtration, a solid was isolated from the filtrate using MTBE.

### [Preparation Example 2] Production of Detection Sensor of Chemical Formula 10

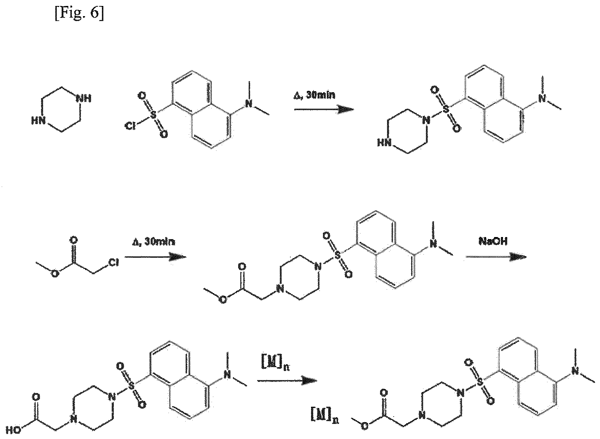

FIG. 6 shows a process for synthesizing a detection sensor complex compound represented by the following Chemical Formula 10 according to the present disclosure.

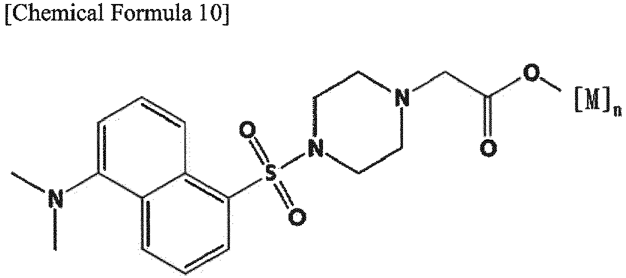

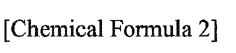

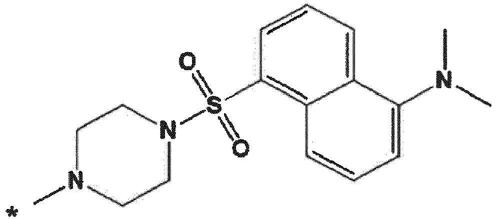

As shown in FIG. 6, chloroacetic acid was added to the * site of Chemical Formula 2, and then a peptide polymer was linked thereto as shown in Chemical Formula 10 above.

### [Preparation Example 3] Production of Detection Sensor of Chemical Formula 11

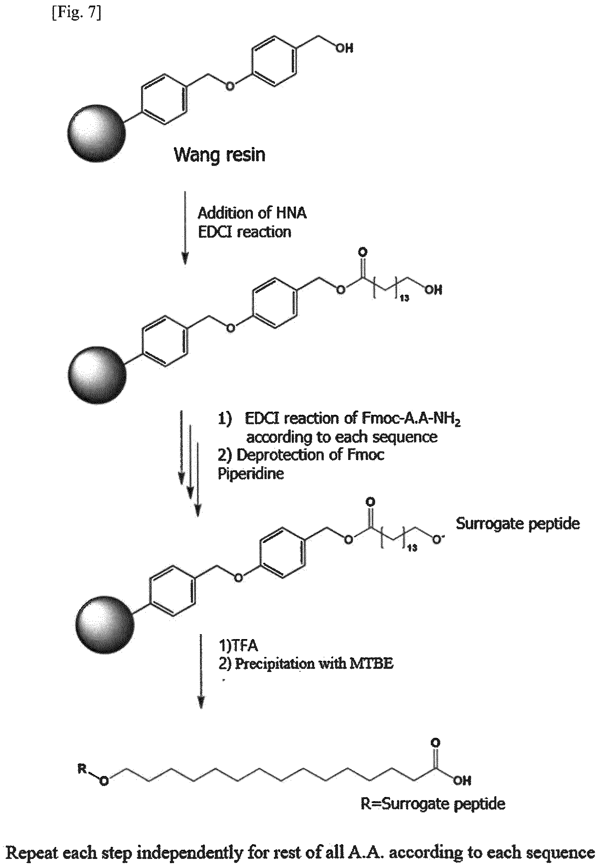

FIG. 7 shows a process for synthesizing a detection sensor complex compound represented by the following Chemical Formula 11 according to the present disclosure.

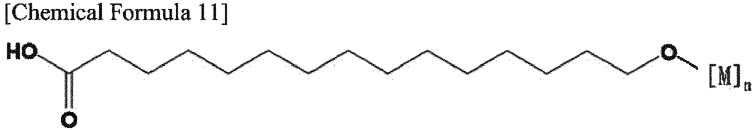

As shown in FIG. 7, for solid phase peptide synthesis, Wang resin was placed in a solid phase peptide synthesis vessel. HNA was dissolved in DMF, and using EDCI synthesis, HOBt and DIC were dissolved in DMF and added to the reaction vessel, followed by stirring. Capping of the unreacted sites of the resin was performed using AC2O. Deprotection of Fmoc was performed with piperidine. Similarly, Fmoc-A.A-OH, HOBt and DIC were dissolved in DMF, and the reaction solution was added to the reaction vessel and then stirred. Coupling of the rest of the amino acids in the sequence was performed using DIC/HOBt. Peptidyl resin was dried and taken for total cleavage. Peptidyl resin was treated with TFA at room temperature. After filtration, a solid was isolated from the filtrate using MTBE.

### [Preparation Example 4] Production of Aptamer-MNP Conjugate

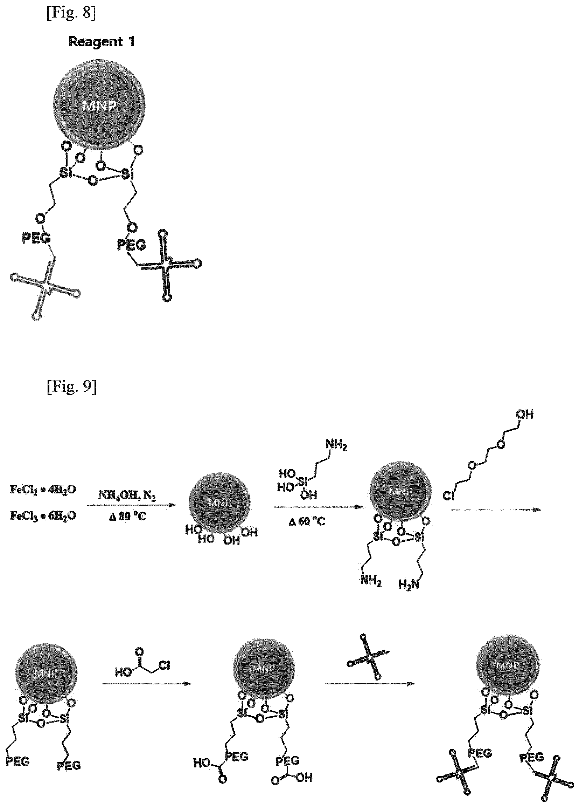

FIG. 9 shows a process for producing an aptamer-MNP conjugate (a second binding moiety-carrier conjugate) shown in FIG. 8.

As shown in FIG. 9, FeCl₂.4H₂O and FeCl₃.6H₂O were washed by repeated heating and cooling in water and dried. MNPs were dispersed using a sonicator. APTES was added slowly to the MNPs and then reacted, followed by drying in a vacuum oven. The completion of coupling was monitored through the Kaiser test. Chloroacetic acid was added to and reacted with the compound, followed by drying in a vacuum oven. Thereafter, an apatamer was linked thereto.

### [Preparation Example 5] (1) Production of Peptides, Represented by M and Measurement of Retention Time

In order to confirm the simultaneous detection ability of the detection sensor of the present disclosure, the retention time (RT) for the sequence of each peptide represented by M was measured, and the results of the measurement are shown in Tables 2 to 20 below.

**[Table 2]**

| SEQ ID | A.A sequence | RT(Min) |
|---|---|---|
| 1 | LNHEGK | 0.867 |
| 2 | AAATNPAR | 0.888 |
| 3 | SPEDEEK | 0.892 |
| 4 | EGGHNIK | 0.894 |
| 5 | NAGPTAR | 0.908 |
| 6 | FSNSGSR | 0.921 |
| 7 | NDSEPGSQR | 0.942 |
| 8 | TGVIHEK | 0.946 |
| 9 | LVHHNVTR | 0.957 |
| 10 | THHDGAITER | 0.965 |
| 11 | WTNQQK | 0.981 |
| 12 | VNDSGYK | 0.984 |
| 13 | VGSDTVR | 0.985 |
| 14 | VSQALR | 0.993 |

**[Table 3]**

| SEQ ID | Amino Acid sequence | RT(Min) |
|---|---|---|
| 15 | ENGTISR | 1.012 |
| 16 | SVDGPIR | 1.024 |
| 17 | FTEPSR | 1.024 |
| 18 | ETFGDSK | 1.024 |
| 19 | HSPGR | 1.063 |
| 20 | NGVHK | 1.068 |
| 21 | NNFGNGR | 1.072 |
| 22 | GDSTFESK | 1.077 |
| 23 | EEQEETSAIR | 1.083 |
| 24 | FQEGQEEER | 1.09 |
| 25 | ILDGGNK | 1.1 |
| 26 | TQTPK | 1.117 |
| 27 | VAHLTGK | 1.123 |
| 28 | VLVEQTK | 1.171 |
| 29 | FDGHR | 1.192 |
| 30 | YHEEFEK | 1.209 |
| 31 | SDFSNEER | 1.212 |
| 32 | ATAGFR | 1.246 |
| 33 | LHGTLPK | 1.278 |
| 34 | SGSGLVGR | 1.288 |
| 35 | AVLIPHHK | 1.289 |
| 36 | SQLANTEPTK | 1.291 |
| 37 | WQHQIK | 1.293 |
| 38 | LIAQASEK | 1.295 |
| 39 | VAQELEEK | 1.336 |
| 40 | EQAALVSK | 1.367 |
| 41 | YVPNSGQEDADR | 1.371 |
| 42 | SADSHGHPR | 1.382 |
| 43 | ISPDR | 1.393 |
| 44 | ASLAEETR | 1.42 |
| 45 | NGNFHPK | 1.441 |
| 46 | LYVVEK | 1.453 |
| 47 | FVTQAEGAK | 1.472 |

**[Table 4]**

| SEQ ID | A.A sequence | RT(Min) |
|---|---|---|
| 48 | GSQGAIPPPDK | 1.517 |
| 49 | IQGDLAGR | 1.525 |
| 50 | SVETIK | 1.536 |
| 51 | TIVAK | 1.543 |
| 52 | SHTALLR | 1.558 |
| 53 | SSDANLYR | 1.683 |
| 54 | ELVHTYK | 1.687 |
| 55 | GNVLR | 1.72 |
| 56 | DDVIK | 1.754 |
| 57 | EVFEDSDK | 1.776 |
| 58 | ILADATAK | 1.78 |
| 59 | IAGDQSTLQR | 1.834 |
| 60 | GEAGVIGER | 1.862 |
| 61 | ITQDAQLK | 1.863 |
| 62 | TQVEELSK | 1.872 |
| 63 | GGVASGFK | 1.889 |
| 64 | GAAFVSK | 1.896 |
| 65 | IQTQLQR | 1.9 |
| 66 | IQGDGAALQEK | 1.925 |
| 67 | YIGVGK | 1.929 |
| 68 | FPSTSESR | 1.936 |
| 69 | LNVEGTER | 1.94 |
| 70 | SSALQVSGSTR | 1.941 |
| 71 | EVFENTER | 1.954 |
| 72 | DGPEQLR | 1.975 |
| 73 | VAEAFR | 1.994 |

**[Table 5]**

| SEQ ID | A.A sequence | RT(Min) |
|---|---|---|
| 74 | QAFQGAVQK | 2 |
| 75 | SLGDLEK | 2.035 |
| 76 | EVATEGIR | 2.038 |
| 77 | VPPEDIK | 2.105 |
| 78 | ATVVYQGER | 2.158 |
| 79 | VGDVLK | 2.166 |
| 80 | LSSTTTTTGLR | 2.166 |
| 81 | QVFGEATK | 2.171 |
| 82 | SDIAPVAR | 2.184 |
| 83 | GISSTTVTGR | 2.197 |
| 84 | TAATAALAGR | 2.204 |
| 85 | FPDGR | 2.225 |
| 86 | QVPLQR | 2.249 |
| 87 | DADSINSSIDK | 2.32 |
| 88 | ELGYVEAK | 2.329 |
| 89 | VDPHFR | 2.36 |
| 90 | VILDGGDR | 2.373 |
| 91 | AGVGQSWK | 2.374 |
| 92 | TDQYWEK | 2.448 |
| 93 | LTWASHEK | 2.448 |
| 94 | VQDVIER | 2.489 |

**[Table 6]**

| SEQ ID | A.A sequence | RT(Min) |
|---|---|---|
| 95 | SGDFYTEK | 2.517 |
| 96 | DQVETALK | 2.527 |
| 97 | SSQAGIPVR | 2.545 |
| 98 | VYSTSVTGSR | 2.551 |
| 99 | STTPASNTVR | 2.556 |
| 100 | ADIIR | 2.576 |
| 101 | TTSDGGYSFK | 2.586 |
| 102 | VFQQVAQASK | 2.616 |
| 103 | ALVVK | 2.616 |
| 104 | EAFAAVSK | 2.623 |
| 105 | EDGSVDFQR | 2.662 |
| 106 | VTFEESAK | 2.691 |
| 107 | IPIQR | 2.691 |
| 108 | SSSISSFK | 2.699 |
| 109 | DLWQQAGTNK | 2.702 |
| 110 | LPGAHLQR | 2.714 |
| 111 | AHLTVVR | 2.718 |
| 112 | QYTDSTFR | 2.742 |
| 113 | DGHSESSTLIGR | 2.841 |
| 114 | QLTPYAQR | 2.887 |
| 115 | GSGLSLASGR | 2.896 |
| 116 | ELGFGSAK | 2.91 |
| 117 | VTVLGQPK | 2.926 |

**[Table 7]**

| SEQ ID | A.A sequence | RT(Min) |
|---|---|---|
| 118 | VAGWGR | 3.006 |
| 119 | QTWVK | 3.008 |
| 120 | TAGGGPDSELQPQDK | 3.03 |
| 121 | YSPGGTPTAIK | 3.049 |
| 122 | QHADAVHLISR | 3.059 |
| 123 | LEPQAA VVK | 3.078 |
| 124 | TLLTAAR | 3.096 |
| 125 | LTEATQLGK | 3.116 |
| 126 | SYFEK | 3.15 |
| 127 | AILSTYR | 3.252 |
| 128 | SPYGFR | 3.257 |
| 129 | VLIAHNQVR | 3.259 |
| 130 | WSYQLSSR | 3.278 |
| 131 | IPGSPEIR | 3.287 |
| 132 | AEQSLQAAIK | 3.294 |
| 133 | SVNAQVTDINSK | 3.3 |
| 134 | ASSFLGEK | 3.31 |
| 135 | DEQVPFSK | 3.336 |
| 136 | ESGVLLTDK | 3.425 |
| 137 | EGYLVK | 3.517 |
| 138 | SVEVLK | 3.559 |
| 139 | ALEQALEK | 3.567 |
| 140 | QWQTLK | 3.631 |
| 141 | DALSASVVK | 3.66 |
| 142 | VDPVNFK | 3.69 |
| 143 | DGSTIPIAK | 3.787 |
| 145 | TNDPGVLQAAR | 3.801 |
| 144 | FDDESAEEIR | 3.801 |
| 146 | EGTEASLQIR | 3.833 |
| 147 | GGVLIQR | 3.834 |
| 148 | ALNSVAYER | 3.904 |
| 149 | LTQGDYFTK | 3.91 |
| 150 | AGLSTVYK | 3.926 |
| 151 | LFDASDSSSYK | 3.971 |
| 152 | VGVNGFGR | 3.978 |
| 153 | GPGGVWAAK | 3.988 |

**[Table 8]**

| SEQ ID | A.A sequence | RT(Min) |
|---|---|---|
| 154 | YEYLEGGDR | 4.024 |
| 155 | YVSALTTPAR | 4.025 |
| 156 | SLLQPNK | 4.035 |
| 157 | GTPPGVYIK | 4.062 |
| 158 | FQASVATPR | 4.074 |
| 159 | QVFAVQR | 4.084 |
| 160 | EIFGQDAR | 4.111 |
| 161 | SVNPYLQGQR | 4.114 |
| 162 | GTFSTTVTGR | 4.115 |
| 163 | LLSEVR | 4.127 |
| 164 | EYFYTSGK | 4.132 |
| 165 | AILGATEVK | 4.136 |
| 166 | VLDEATLK | 4.16 |
| 167 | EQVDQGPDWER | 4.162 |
| 168 | DGPDTLLSK | 4.183 |
| 169 | GWSPTPR | 4.194 |
| 170 | QLYSALANK | 4.216 |
| 171 | VSISTLNK | 4.285 |
| 172 | DFVQPPTK | 4.299 |
| 173 | NAIEALGSK | 4.357 |
| 174 | EDGSLDFQR | 4.377 |
| 175 | DQLVLGR | 4.396 |
| 176 | NANTFISPQQR | 4.419 |
| 177 | SPQAFYR | 4.431 |
| 178 | SGIIIIAIHR | 4.448 |
| 179 | EGSDLSWER | 4.452 |
| 180 | AVEPQLQEEER | 4.486 |
| 181 | ISSAGASFGSR | 4.488 |

**[Table 9]**

| SEQ ID | A.A sequence | RT(Min) |
|---|---|---|
| 179 | EGSDLSWER | 4.452 |
| 180 | AVEPQLQEEER | 4.486 |
| 181 | ISSAGASFGSR | 4.488 |
| 182 | AWTYR | 4.511 |
| 183 | GGPFSDSYR | 4.515 |
| 184 | VTTNPNLR | 4.58 |
| 185 | DEVEDDYIK | 4.582 |
| 186 | PAPGSTAPPAHGVTSAPDTR | 4.594 |
| 187 | NQNTFLR | 4.602 |
| 188 | GLGDDTALNDAR | 4.61 |
| 189 | LSVIR | 4.64 |
| 190 | LIQGAPTIR | 4.645 |
| 191 | FPSGTLR | 4.66 |
| 192 | EDAVSAAFK | 4.707 |
| 193 | VAELEDEK | 4.739 |
| 194 | FVGGAENTAHPR | 4.742 |
| 195 | EVASNSELVQSSR | 4.751 |
| 196 | AAISGENAGLVR | 4.77 |
| 197 | TGLQEVEVK | 4.775 |
| 198 | TYLPAVDEK | 4.79 |
| 199 | GGLVDITR | 4.798 |
| 200 | INDISHTQSVSSK | 4.807 |
| 201 | FYQDLK | 4.815 |
| 202 | VEVLVER | 4.83 |
| 203 | LQAEAFQAR | 4.852 |
| 204 | AYTGFEQAAR | 4.886 |
| 205 | TGQIFNQSYSK | 4.951 |
| 206 | HSENFAWTENR | 4.956 |
| 207 | ELGFGSAR | 4.961 |
| 208 | AVIFK | 4.972 |
| 209 | GFWAGPSR | 4.977 |

**[Table 10]**

| SEQ ID | A.A sequence | RT(Min) |
|---|---|---|
| 210 | SNFVPTNVGSK | 5.003 |
| 211 | SLVGLGGTK | 5.005 |
| 212 | VSVYAVPDK | 5.007 |
| 213 | SDIAIDDVK | 5.032 |
| 214 | LGAETLPR | 5.033 |
| 215 | TEAESWYQTK | 5.036 |
| 216 | LVEIVHPSQEEDR | 5.037 |
| 217 | GSYYDSFK | 5.039 |
| 218 | GTYSTTVTGR | 5.044 |
| 219 | IVLVDNK | 5.078 |
| 220 | NPSDEDLLR | 5.099 |
| 221 | ETLDAQTFHTR | 5.118 |
| 222 | QLVEALDK | 5.126 |
| 223 | GEAAGAVQELAR | 5.135 |
| 224 | NFGGGNTAWEEK | 5.158 |
| 225 | GPLQLER | 5.183 |
| 226 | EDLTPFK | 5.19 |
| 227 | IQQNLDQLR | 5.226 |
| 228 | EALFGAR | 5.245 |
| 229 | YTSGFDELQR | 5.251 |
| 230 | LLQEIK | 5.265 |
| 231 | DLETSLEK | 5.273 |
| 232 | YLQSLER | 5.283 |
| 233 | FDPSLTQR | 5.284 |
| 234 | TYSVEYLDSSK | 5.318 |
| 235 | AGFAGDDAPR | 5.32 |
| 236 | TLTIQVK | 5.321 |
| 237 | SALTIQTLHTR | 5.327 |
| 238 | SYLPQTVR | 5.328 |
| 239 | YIFTATPAK | 5.368 |
| 240 | VTGVITQGAK | 5.379 |
| 241 | LPTDSELAPR | 5.425 |
| 242 | STDFFQSR | 5.426 |
| 243 | SLYNLGGSR | 5.435 |
| 244 | DTDLDGFPDEK | 5.436 |
| 245 | TFPISGAR | 5.447 |
| 246 | YLLEAK | 5.48 |
| 247 | ELLDYK | 5.486 |
| 248 | IDGVLIR | 5.487 |
| 249 | DISEVVTPR | 5.487 |

**[Table 11]**

| SEQ ID | A.A sequence | RT(Min) |
|---|---|---|
| 250 | TTGSGLLK | 5.512 |
| 251 | FDQNLDTK | 5.519 |
| 252 | LWEGSTSR | 5.53 |
| 253 | TNQVNSGGVLLR | 5.536 |
| 254 | LEGEPVALR | 5.546 |
| 255 | SAFSVAVTK | 5.552 |
| 256 | VDGSVDFYR | 5.554 |
| 257 | ETAALNSVR | 5.558 |
| 258 | ESGAEVYFR | 5.563 |
| 259 | FNDTEVLQR | 5.568 |
| 260 | IQALQQQADEAEDR | 5.59 |
| 261 | SLFTEGR | 5.591 |
| 262 | AYPTPLR | 5.609 |
| 263 | ATVFLEQR | 5.621 |
| 264 | EVGQLAETQR | 5.624 |
| 265 | LPVSLSSAK | 5.629 |
| 266 | QLYGDTGVLGR | 5.631 |
| 267 | AEIEYLEK | 5.654 |
| 268 | AAYLSTISK | 5.661 |
| 269 | LTQLNLDR | 5.663 |
| 270 | GQTLLAVAK | 5.675 |
| 271 | VLSFSSR | 5.676 |
| 272 | SLGFVSK | 5.683 |
| 273 | VAQVSITK | 5.695 |
| 274 | GDSVVYGLR | 5.7 |
| 275 | YLQGSSVQLR | 5.704 |
| 276 | DYWSTVK | 5.707 |
| 277 | SESETYTLSSK | 5.709 |
| 278 | ESLAAELR | 5.723 |
| 279 | SNFQQPYITNR | 5.73 |
| 280 | VLQGLPR | 5.745 |
| 281 | NWQDYGVR | 5.768 |
| 282 | DLFDR | 5.77 |
| 283 | ELVYETVR | 5.773 |
| 284 | SELVVEVK | 5.782 |
| 285 | YFQGIR | 5.786 |
| 286 | QINDYVAK | 5.789 |
| 287 | GNPESSFNDENLR | 5.79 |
| 288 | GYFGDEQQIR | 5.812 |
| 289 | VEDIPLAR | 5.817 |
| 290 | NDLISATK | 5.823 |
| 291 | QINDYVEK | 5.874 |
| 292 | EDTPNSVWEPAK | 5.874 |
| 293 | LPPLPPR | 5.916 |
| 294 | FVSTTYSGVTR | 5.917 |
| 295 | DISLSDYK | 5.939 |
| 296 | AAGASVVTELR | 5.948 |
| 297 | TFTPQPPGLER | 5.966 |
| 298 | IPALDPEK | 5.995 |

**[Table 12]**

| SEQ ID | A.A sequence | RT(Min) |
|---|---|---|
| 299 | VSSASDYNSSELK | 6.007 |
| 300 | YETELNLR | 6.063 |
| 301 | LVVVGAGGVGK | 6.086 |
| 302 | DFIYR | 6.11 |
| 303 | YLGEEYVK | 6.114 |
| 304 | LYTLVQR | 6.138 |
| 305 | GQVVYVFSK | 6.149 |
| 306 | LDVDQALNR | 6.151 |
| 307 | LESLLEEK | 6.155 |
| 308 | IIEGEPNLK | 6.158 |
| 309 | GVTSFGLENK | 6.161 |
| 310 | LTISESSISDR | 6.164 |
| 311 | VGDYGSLSGR | 6.167 |
| 312 | EPNAQEILQR | 6.172 |
| 313 | SFLDSGYR | 6.179 |
| 314 | SFHHEESLEELPETSGK | 6.182 |
| 315 | DQYYNIDVPSR | 6.186 |
| 316 | NIDVLEK | 6.187 |
| 317 | DLVQPINPR | 6.205 |
| 318 | INPASLDK | 6.213 |
| 319 | ADVNVLTK | 6.216 |
| 320 | AAGAPLATELR | 6.219 |
| 321 | QSIVPLR | 6.223 |
| 322 | GGSPPAPLPAHLSR | 6.229 |
| 323 | TEFTTALQR | 6.234 |
| 324 | SYVITTSR | 6.251 |
| 325 | DAVEDLESVGK | 6.256 |
| 326 | FLLYNR | 6.291 |
| 327 | QVIDVLETDK | 6.339 |
| 328 | TEEFEVTK | 6.36 |
| 329 | GLQAQGYGVR | 6.36 |
| 330 | ITDFGLAK | 6.366 |
| 331 | LEPESEFYR | 6.373 |
| 332 | ANSFLGEK | 6.379 |
| 333 | GNQWVGYDDVK | 6.394 |
| 334 | DADPDTFFAK | 6.421 |
| 335 | VVTITLDK | 6.423 |
| 336 | DFYVDENTTVR | 6.424 |
| 337 | YLVAPDGK | 6.43 |
| 338 | GSPILLGVSK | 6.431 |
| 339 | DLGSELVR | 6.431 |
| 340 | FSISNANIK | 6.466 |
| 341 | GLLPTSVSPR | 6.486 |
| 342 | YGLHVSPAYEGR | 6.491 |

**[Table 13]**

| SEQ ID | A.A sequence | RT(Min) |
|---|---|---|
| 344 | TFYLR | 6.529 |
| 343 | GPGLNLTSGQYR | 6.529 |
| 345 | YPDTLLGSSEK | 6.539 |
| 346 | LSEEEFGGFR | 6.555 |
| 347 | QEYEQLIAK | 6.556 |
| 348 | SLHVPGLNK | 6.563 |
| 349 | TVIEVDER | 6.564 |
| 350 | SLETSAFVK | 6.599 |
| 351 | SDDEVDDPAVELK | 6.63 |
| 352 | AALPEGLPEASR | 6.638 |
| 353 | QLDVEAALTK | 6.644 |
| 354 | LDSSEFLK | 6.661 |
| 355 | AVYEAVLR | 6.664 |
| 356 | VPTPQAIR | 6.667 |
| 358 | VTVNVLSPR | 6.669 |
| 357 | GWDWTSGVNK | 6.669 |
| 359 | FETEQALR | 6.698 |
| 360 | GQDTSEELLR | 6.704 |
| 361 | LSFSYGR | 6.719 |
| 362 | EVSFYYSEENK | 6.741 |
| 363 | APEGFAVR | 6.746 |
| 364 | DYPFQGK | 6.75 |
| 365 | LDGPLPSGVR | 6.767 |
| 366 | FSTQEEIQAR | 6.782 |
| 367 | AYQGVAAPFPK | 6.785 |
| 368 | ISPVEESEDVSNK | 6.788 |
| 369 | YSITFTGK | 6.809 |
| 370 | YQTWIK | 6.821 |
| 371 | QESFFVDER | 6.822 |
| 372 | QQDGELVGYR | 6.828 |
| 373 | FNVSSVEK | 6.834 |
| 374 | TDPGVFIGVK | 6.862 |
| 375 | AAGASVATELR | 6.863 |
| 376 | GEPGEGAYVYR | 6.865 |
| 377 | EAVILYAQPSER | 6.87 |
| 378 | GAVYVYFGSK | 6.878 |
| 379 | YQYAIDEYYR | 6.89 |
| 380 | TELLPGDR | 6.89 |
| 381 | DALEESLK | 6.899 |
| 382 | TEGDGVYTLNNEK | 6.904 |
| 383 | AFLGLQK | 6.913 |
| 384 | SPEAAGVQDPSLR | 6.916 |
| 386 | IQNILTEEPK | 6.923 |
| 385 | GNFVSPVK | 6.923 |
| 387 | DSEYPFK | 6.957 |
| 388 | ENYLLPEAK | 6.968 |
| 389 | DEGSYSLEEPK | 6.975 |
| 390 | VAQGIVSYGR | 6.991 |

**[Table 14]**

| SEQ ID | A.A sequence | RT(Min) |
|---|---|---|
| 391 | EQDQVWVR | 7.005 |
| 392 | SVPLPTLK | 7.01 |
| 393 | GNETLHYETFGK | 7.02 |
| 394 | NTQIDNSWGSEER | 7.028 |
| 395 | LLELTGPK | 7.031 |
| 396 | IQELQLAASR | 7.039 |
| 397 | LAAADGAVAGEVR | 7.047 |
| 398 | TAVNALWGK | 7.072 |
| 399 | VGAHAGEYGAEALER | 7.076 |
| 400 | LPGGLEPK | 7.093 |
| 402 | LPGGYGLPYTTGK | 7.103 |
| 401 | LAILYR | 7.103 |
| 403 | QLAEEYLYR | 7.134 |
| 404 | DITSDTSGDFR | 7.147 |
| 405 | AGGSIPIPQK | 7.155 |
| 406 | QNSLLWR | 7.159 |
| 407 | LPASFDAR | 7.161 |
| 408 | QIGEFIVTR | 7.172 |
| 409 | VIDEEWQR | 7.18 |
| 410 | ESDTSYVSLK | 7.205 |
| 411 | SDALQLGLGK | 7.221 |
| 412 | DVAVIAESIR | 7.221 |
| 413 | DSLSINATNIK | 7.243 |
| 414 | LAYYGFTK | 7.251 |
| 415 | GVQINIK | 7.273 |
| 416 | ALLAFQESK | 7.28 |
| 417 | SVIAPSLEQYK | 7.301 |
| 418 | GTHSLPPRPAAVPVPLR | 7.304 |
| 419 | YEELQVTVGR | 7.307 |
| 420 | SQASPSEDEETFELR | 7.311 |
| 421 | YTELPYGR | 7.322 |
| 422 | DFIDIESK | 7.323 |
| 423 | LTPEELER | 7.348 |
| 424 | VTWQNLR | 7.356 |
| 425 | VLDELTLSK | 7.378 |
| 426 | GIDPDLLK | 7.384 |
| 427 | AFVFPK | 7.385 |
| 428 | TAAIVNSIR | 7.386 |
| 429 | NGSQAFVHWQEPR | 7.387 |
| 430 | ELLETVVNR | 7.392 |
| 431 | DLNETLLR | 7.405 |
| 432 | QDGSVDFFR | 7.418 |
| 433 | TSNFNAAISLK | 7.419 |
| 434 | EATLELLGR | 7.42 |
| 435 | AEIYALNR | 7.435 |
| 436 | ALLEAPLK | 7.441 |
| 437 | IELPTTVK | 7.46 |
| 438 | VLFSGSLR | 7.461 |

**[Table 15]**

| SEQ ID | A.A sequence | RT(Min) |
|---|---|---|
| 439 | EVEQVYLR | 7.51 |
| 440 | LPGIFDDVHGSHGR | 7.516 |
| 441 | GTPLPTYEEAK | 7.522 |
| 442 | TVPDPLAVK | 7.528 |
| 443 | LQQQLWSK | 7.531 |
| 444 | SQLEESISQLR | 7.532 |
| 445 | QELTTEFR | 7.563 |
| 446 | LYDVLR | 7.567 |
| 447 | TVLFGVQPK | 7.568 |
| 448 | LSWGYSGSAGR | 7.584 |
| 449 | LFAYPDTHR | 7.6 |
| 450 | ISISTSGGSFR | 7.602 |
| 451 | LSPEYYDLAR | 7.604 |
| 452 | ALPSHLGLHPER | 7.628 |
| 453 | YEVVYPIR | 7.644 |
| 454 | ALFSTLK | 7.646 |
| 455 | IQILPR | 7.654 |
| 456 | SGPTWWGPQR | 7.661 |
| 457 | GLQVALEEFHK | 7.665 |
| 459 | WGGLVALR | 7.676 |
| 458 | LGDGFEGFYK | 7.676 |
| 460 | VSPLTFGR | 7.678 |
| 461 | TATITVLPQQPR | 7.684 |
| 462 | SANTITSFVDR | 7.699 |
| 463 | NSWGENWGNK | 7.71 |
| 464 | VGDQPTLQLK | 7.727 |
| 465 | ELLEEVGQNGSR | 7.736 |
| 466 | NVIDPPIYAR | 7.74 |
| 467 | VLFYVDSEK | 7.741 |
| 468 | GSEIVAGLEK | 7.743 |
| 469 | GLVVLTPER | 7.744 |
| 470 | AVPEGFVIPR | 7.753 |
| 471 | GWSTDEANTYFK | 7.757 |
| 472 | GVAETPTYPWR | 7.763 |
| 473 | WSGDFTQGPQSAK | 7.769 |
| 474 | LLLGTGTDAR | 7.781 |
| 475 | GLSGIGAFR | 7.783 |
| 476 | ESESAPGDFSLSVK | 7.783 |
| 477 | DFIATLGK | 7.798 |
| 478 | SFISGGSTITGVGK | 7.799 |
| 479 | GVSPSASAWPEEK | 7.825 |
| 480 | DTNALPPTVFK | 7.825 |
| 481 | IFGSYDPR | 7.829 |
| 482 | SLTEILK | 7.834 |
| 483 | TLEPELGTLQAR | 7.844 |
| 484 | SGLSTGWTQLSK | 7.849 |
| 485 | EEADALYEALK | 7.856 |
| 486 | IAQYYYTFK | 7.872 |
| 487 | IEVAQFVK | 7.882 |
| 488 | AELAETIVYAR | 7.884 |
| 489 | FFQYDTWK | 7.899 |
| 490 | LYTDDEDDIYK | 7.924 |
| 491 | DNIYTSEWSQR | 7.937 |
| 492 | QLVLNVSK | 7.954 |
| 493 | ALDFAVGEYNK | 7.963 |

**[Table 16]**

| SEQ ID | A.A sequence | RT(Min) |
|---|---|---|
| 494 | YLGVTLSPR | 8.019 |
| 495 | TTTLPVEFK | 8.023 |
| 496 | TGIIDYGIR | 8.067 |
| 497 | EQPELEVQYQGR | 8.069 |
| 498 | SWSVYVGAR | 8.072 |
| 499 | WVQDYIK | 8.115 |
| 500 | GDLTIANLGTSEGR | 8.137 |
| 501 | DALSALAR | 8.148 |
| 502 | LALFPDK | 8.166 |
| 503 | SGLNIEDLEK | 8.176 |
| 504 | AQATPWTQTQAVR | 8.205 |
| 505 | SLDSPAALAER | 8.217 |
| 506 | YGGDPPWPR | 8.221 |
| 507 | QWAGLVEK | 8.239 |
| 508 | TSFPEDTVITYK | 8.24 |
| 509 | NYNLVESLK | 8.253 |
| 510 | LYIEYGIQR | 8.257 |
| 511 | VEPSVFLPASK | 8.288 |
| 512 | DGGVLSPILTR | 8.29 |
| 513 | VLDELTLTK | 8.298 |
| 514 | LDIGIINENQR | 8.308 |
| 515 | LPEPIVSTDSR | 8.313 |
| 516 | EENDDFASFR | 8.344 |
| 517 | TILFSYGTK | 8.369 |
| 518 | SQFEGFVK | 8.376 |
| 519 | LDPFFK | 8.377 |
| 520 | DSDLLSPSDFK | 8.383 |
| 521 | ALENLLPTK | 8.396 |
| 522 | TIELLGQEVSR | 8.399 |
| 523 | VGYPGPSGPLGAR | 8.435 |
| 524 | NFPSPVDAAFR | 8.448 |
| 525 | YISLLK | 8.45 |
| 526 | IPQEEFDGNQFQK | 8.466 |
| 527 | SAVTALWGK | 8.49 |
| 528 | LSILYPATTGR | 8.493 |
| 529 | LFLETAEK | 8.497 |
| 530 | QLEWGLER | 8.498 |

**[Table 17]**

| SEQ ID | A.A sequence | RT(Min) |
|---|---|---|
| 531 | IIVPLNNR | 8.503 |
| 532 | DDFLIYDR | 8.515 |
| 533 | AGYYYIYSK | 8.573 |
| 534 | TPASQGVILPIK | 8.599 |
| 535 | NTVLVWR | 8.605 |
| 536 | IVEELQSLSK | 8.612 |
| 537 | TFYNASWSSR | 8.634 |
| 538 | QEVWLANGAAESR | 8.643 |
| 539 | ISVPYEGVFR | 8.654 |
| 540 | IIDGVPVEITEK | 8.674 |
| 541 | FQLFGSPSGQK | 8.675 |
| 542 | ANVFVQLPR | 8.687 |
| 543 | AQWANPFDPSK | 8.698 |
| 544 | LAAWLAK | 8.737 |
| 545 | YYTVFDR | 8.76 |
| 546 | EFSEENPAQNLPK | 8.767 |
| 547 | FTGSSWIK | 8.818 |
| 548 | IWLDNVR | 8.829 |
| 549 | ETLLQDFR | 8.838 |
| 550 | LTFYGNWSEK | 8.849 |
| 551 | QLVPALGPPVR | 8.852 |
| 552 | ENYPLPWEK | 8.869 |
| 553 | LELQQLQAER | 8.879 |
| 554 | IVIEYVDR | 8.897 |
| 555 | IPVDLPEAR | 8.917 |
| 556 | DPTFIPAPIQAK | 8.918 |
| 557 | QQPLFVSGGDDYK | 8.93 |
| 558 | VLLPPDYSEDGAR | 8.937 |
| 559 | FVSFLGR | 8.948 |
| 560 | FSAEFDFR | 8.954 |
| 561 | DQEAPYLLR | 8.984 |
| 562 | AFLLTPR | 8.984 |
| 563 | WAFNWDTK | 8.989 |

**[Table 18]**

| SEQ ID | A.A sequence | RT(Min) |
|---|---|---|
| 564 | STDYGIFQINSR | 9.061 |
| 565 | DSPSVWAAVPGK | 9.076 |
| 566 | VEYITGPGVTTYK | 9.123 |
| 567 | LLPYVLEK | 9.131 |
| 568 | LVIIEGDLER | 9.17 |
| 569 | TVIYEIPR | 9.195 |
| 570 | GPPAALTLPR | 9.215 |
| 571 | TPLYIDFK | 9.246 |
| 572 | NLQEILHGAVR | 9.251 |
| 573 | LLDLGAGDGEVTK | 9.27 |
| 575 | YSSDYFQAPSDYR | 9.312 |
| 574 | DTSLFSDEFK | 9.312 |
| 576 | IPEGEAVTAAEFR | 9.316 |
| 577 | EGYYGYTGAFR | 9.322 |
| 578 | EGHFYYNISEVK | 9.322 |
| 579 | DSTYSLSSTLTLSK | 9.328 |
| 580 | NGSGPFLGNIPK | 9.429 |
| 581 | YGNLSNFLR | 9.441 |
| 582 | GNPTVEVDLYTAK | 9.489 |
| 583 | VYLPWSR | 9.49 |
| 584 | YLPLENLR | 9.502 |
| 585 | VYSGILNQSEIK | 9.525 |
| 586 | FPLTNAIK | 9.527 |
| 587 | VIEASFPAGVDSSPR | 9.529 |
| 588 | TWYPEVPK | 9.543 |
| 589 | QIFLPEPEQPSR | 9.584 |
| 590 | QELIQAEIQNGVK | 9.595 |
| 591 | GDLYFANVEEK | 9.684 |
| 592 | GWVTDGFSSLK | 9.711 |
| 593 | VDAETGDVFAIER | 9.715 |
| 594 | LFQIQFNR | 9.752 |
| 595 | AQDGGPVGTELFR | 9.758 |
| 596 | YGSQLAPETFYR | 9.77 |
| 597 | LSSPAVITDK | 9.818 |
| 598 | AYSLFSYNTQGR | 9.818 |
| 599 | LAILGGVEGQPAK | 9.824 |
| 600 | DWFLR | 9.83 |
| 601 | YSFTIELR | 9.849 |
| 602 | AADDTWEPFASGK | 9.876 |
| 603 | LPGIFDDVR | 9.894 |
| 604 | ELTLEDLK | 9.897 |
| 605 | ESFEESWTPNYK | 9.903 |
| 606 | TSVPPFNLR | 9.909 |
| 607 | DYPDEVLQFAR | 9.937 |
| 608 | WIQEYLEK | 9.943 |
| 609 | FGIILR | 9.944 |
| 610 | FEDGVLDPDYPR | 9.952 |
| 611 | ANLTVVLLR | 9.959 |
| 612 | GSVQYLPDLDDK | 9.966 |
| 613 | LSDLEAQWAPSPR | 9.971 |
| 614 | LPLEYSYGEYR | 9.987 |
| 615 | FNAPFDVGIK | 9.988 |

**[Table 19]**

| SEQ ID | A.A sequence | RT(Min) |
|---|---|---|
| 616 | YLYTDDAQQTEAHLEIR | 10.055 |
| 617 | FYTFLK | 10.159 |
| 618 | VPPPSDAPLPFDR | 10.215 |
| 619 | FLNVLSPR | 10.31 |
| 620 | QFYSVFDR | 10.319 |
| 621 | TFTLLDPK | 10.331 |
| 622 | NSSAAWDETLLEK | 10.467 |
| 623 | LALAFYGR | 10.524 |
| 624 | DYVSQFEGSALGK | 10.624 |
| 625 | DSSAAWDEDLLDK | 10.652 |
| 626 | SWSWNYYR | 10.694 |
| 628 | VDLFYLR | 10.814 |
| 627 | ITFSPPLPR | 10.814 |
| 629 | LLWQLNGR | 10.817 |
| 630 | DSSATWEQSLLEK | 10.839 |
| 631 | NPLNAGSWEWSDR | 10.935 |
| 632 | YSVFPTLR | 10.973 |
| 633 | VTAGISFAIPSDK | 10.994 |
| 634 | FLASVSTVLTSK | 11.022 |
| 635 | QSWGLENEALIVR | 11.09 |
| 636 | FLVSLALR | 11.108 |
| 637 | EYFWGLSK | 11.156 |
| 638 | TVDNFVALATGEK | 11.297 |
| 639 | ALAAVLEELR | 11.307 |
| 640 | VGYPELAEVLGR | 11.414 |
| 641 | FTPWWETK | 11.516 |
| 642 | TLAFPLTIR | 11.554 |
| 643 | LPPWNPQVFSSER | 11.974 |
| 644 | SYELPDGQVITISNEWFR | 12.048 |
| 645 | WVAVVFPLSYR | 12.064 |
| 646 | TVAGQDAVIVLLGTR | 12.068 |
| 647 | VLLVELPAFLR | 12.103 |

As shown in Tables 2 to 19 above, as a result of producing the peptides represented by M and then measuring the retention time (RT) for the sequence of each of the peptides, it was possible to produce various sequences for each retention time (RT).

| No | SEQ ID | A.A sequence | hydrophobicity | RT(Min) | No | SEQ ID | A.A sequence | hydrophobicity | RT(Min) |
|---|---|---|---|---|---|---|---|---|---|
| 1 | SEQ ID 648 | LVLK | 14.13 | 2.14 | 21 | SEQ ID 668 | KISVLAI | 24.86 | 9.74 |
| 2 | SEQ ID 649 | TLLK | 13.48 | 1.48 | 22 | SEQ ID 669 | KIATLAI | 21.3 | 8.33 |
| 3 | SEQ ID 650 | SLLK | 12.96 | 1.52 | 23 | SEQ ID 670 | KIASLAI | 21.3 | 8.01 |
| 4 | SEQ ID 651 | IVLK | 12.84 | 1.82 | 24 | SEQ ID 671 | KIASLSI | 20.93 | 7.84 |
| 5 | SEQ ID 652 | LTLK | 10.92 | 1.72 | 25 | SEQ ID 672 | KTTVLAI | 19.75 | 7.44 |
| 6 | SEQ ID 653 | LSLK | 10.54 | 1.83 | 26 | SEQ ID 673 | KSAVLAI | 19.63 | 7.11 |
| 7 | SEQ ID 654 | LALK | 9.88 | 1.62 | 27 | SEQ ID 674 | KIAVSAI | 18.53 | 7.1 |
| 8 | SEQ ID 655 | ITLK | 9.64 | 1.55 | 28 | SEQ ID 675 | KSSVLAI | 18.59 | 6.92 |
| 9 | SEQ ID 656 | ISLK | 9.25 | 1.53 | 29 | SEQ ID 676 | KTTTLAI | 15.41 | 5.97 |
| 10 | SEQ ID 657 | TVLK | 8.22 | 1.08 | 30 | SEQ ID 677 | KIAVLTT | 19.23 | 5.91 |
| 11 | SEQ ID 658 | SVLK | 7.91 | 1.1 | 31 | SEQ ID 678 | KIAVSSI | 16.65 | 5.84 |
| 12 | SEQ ID 659 | VTLK | 7.49 | 1.17 | 32 | SEQ ID 679 | KIAVLAS | 17.13 | 5.27 |
| 13 | SEQ ID 660 | VSLK | 7.25 | 1.18 | 33 | SEQ ID 680 | KSASLSI | 14.41 | 5.12 |
| 14 | SEQ ID 661 | VLTK | 6.43 | 0.99 | 34 | SEQ ID 681 | KSSSLAI | 13.05 | 4.58 |
| 15 | SEQ ID 662 | TALK | 5.65 | 0.98 | 35 | SEQ ID 682 | KIAVTAT | 10.98 | 2.35 |
| 16 | SEQ ID 663 | SALK | 5.34 | 0.96 | 36 | SEQ ID 683 | KIAVTTT | 11.22 | 2.19 |
| 17 | SEQ ID 664 | KIAVLAI | 25.59 | 9.95 | 37 | SEQ ID 684 | KIAVSAS | 8.81 | 1.59 |
| 18 | SEQ ID 665 | KITVLAI | 24.86 | 9.84 | 38 | SEQ ID 685 | KIAVSSS | 7.91 | 1.27 |
| 19 | SEQ ID 666 | KIAVLTI | 25.39 | 9.83 | 39 | SEQ ID 686 | KTAVTAT | 5.65 | 1.03 |
| 20 | SEQ ID 667 | KIAVLSI | 25.39 | 9.76 | 40 | SEQ ID 687 | KSAVSAS | 7.61 | 0.96 |

In addition, as shown in Table 20 above, as a result of additionally producing the peptides represented by M and then measuring the hydrophobicity and retention time (RT) for the sequence of each of the peptides, it was possible to produce various sequences showing a retention time (RT) of 30 seconds to 20 minutes. In order to quantify the same biomarker in multiple samples using the peptide, a binding moiety that recognizes the analyte, such as a detection moiety composed of a different sequence for each sample, may be provided, so that multiple samples may be pooled into one and quantified simultaneously.

### [Preparation Example 6] (2) Production of Peptides Represented by M and Measurement of Retention Time

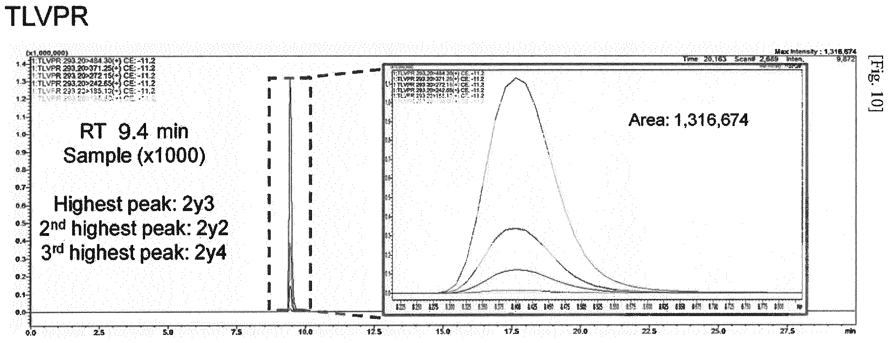

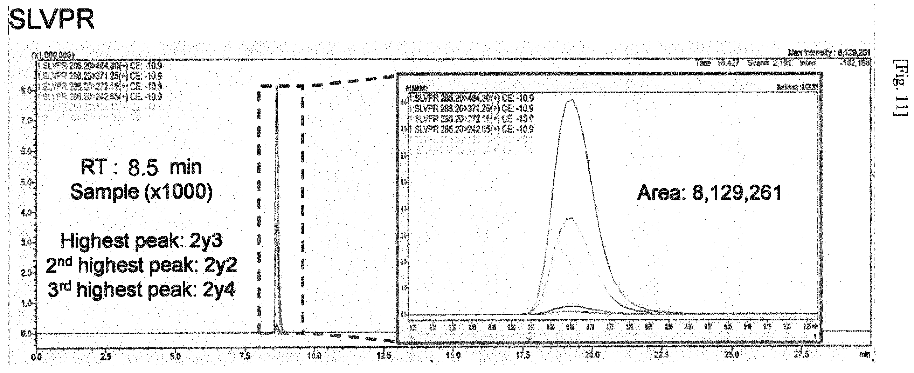

In order to confirm the simultaneous detection ability of the detection sensor of the present disclosure as described in Preparation Example 5 above, the peptide (TLVPR) represented by SEQ ID NO: 688 and the peptide (SLVPR) represented by SEQ ID NO: 669 were synthesized, and then the retention time (RT) for each of the sequences of these peptides was measured. The results of the measurement are shown in Table 21 below. In addition, these compounds were prepared at a concentration of 1.5 µg/ml, and then the peak intensity of each peptide fragment was determined through the mass-to-charge ratio of each peptide fragment in a mass spectrometer, and the results are shown in FIGS. 10 and 11.

**[Table 21]**

| No | SEQ ID | A.A sequence | RT(Min) |
|---|---|---|---|
| 1 | SEQ ID 688 | TLVPR | 9.4 |
| 2 | SEQ ID 689 | SLVPR | 8.5 |

### [Preparation Example 7] (1) Synthesis of Units and M

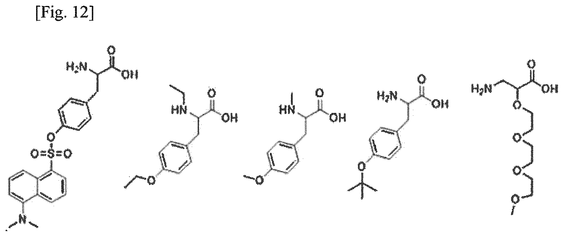

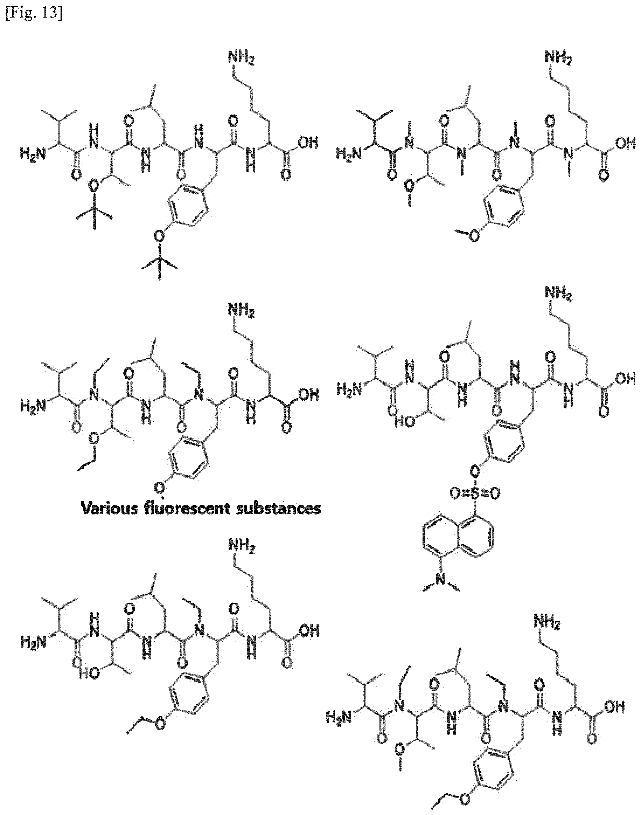

FIG. 12 shows the kinds of exemplary amino acids or amino acid analogs that may correspond to X₁ to Xₘ in Formula 2 of the present disclosure. In addition, FIG. 13 shows examples of M that may be obtained by polymerizing these amino acids or amino acid analogs.

### [Preparation Example 8] (2) Synthesis of Units and M

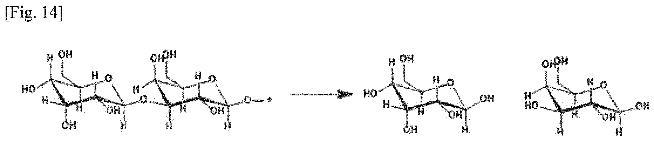

As shown in FIG. 14, a disaccharide that may be M of the present disclosure was prepared. The disaccharide M was degraded by lactase or under an acidic condition into two monosaccharides that are isomers of each other, and thus the sensitivity in mass spectrometry thereof was doubled.

### [Experimental Example 1] Experiment for Simultaneous Detection of Four Peptides represented by M

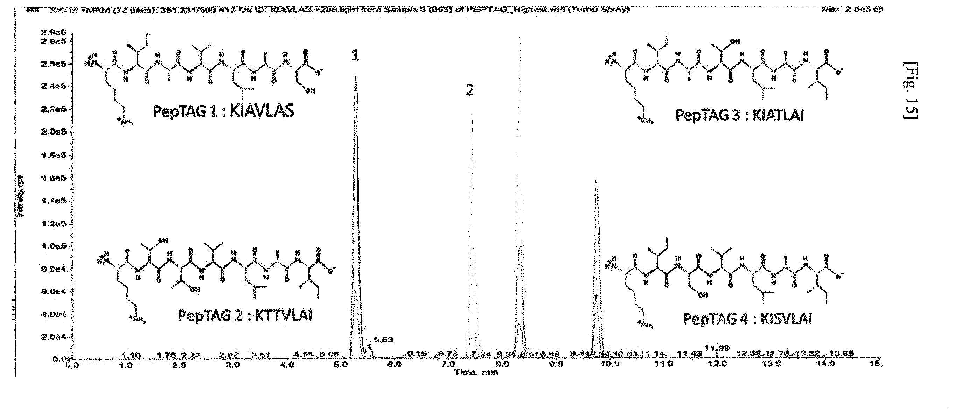

In order to confirm the ability to simultaneously detect the peptides represented by M according to the present disclosure, the peptides having the sequences shown in Table 22 below were detected by mass spectrometry MRM, and the results are shown in FIG. 15.

**[Table 22]**

| No | SEQ ID | A.A sequence |
|---|---|---|
| 1 | SEQ ID 679 | KIAVLAS |
| 2 | SEQ ID 672 | KTTVLAI |
| 3 | SEQ ID 669 | KIATLAI |
| 4 | SEQ ID 668 | KISVLAI |

As shown in FIG. 15, it was confirmed that it was possible to simultaneously detect the peptides having the four sequences shown in Table 22 above.

### [Experimental Example 2] (1) Examination of Sensitivity to Peptides Represented by M

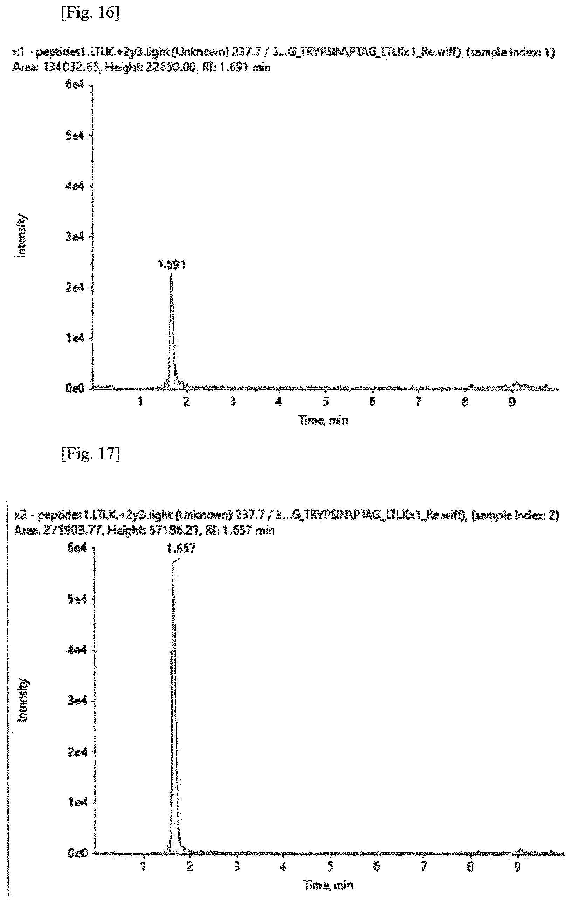

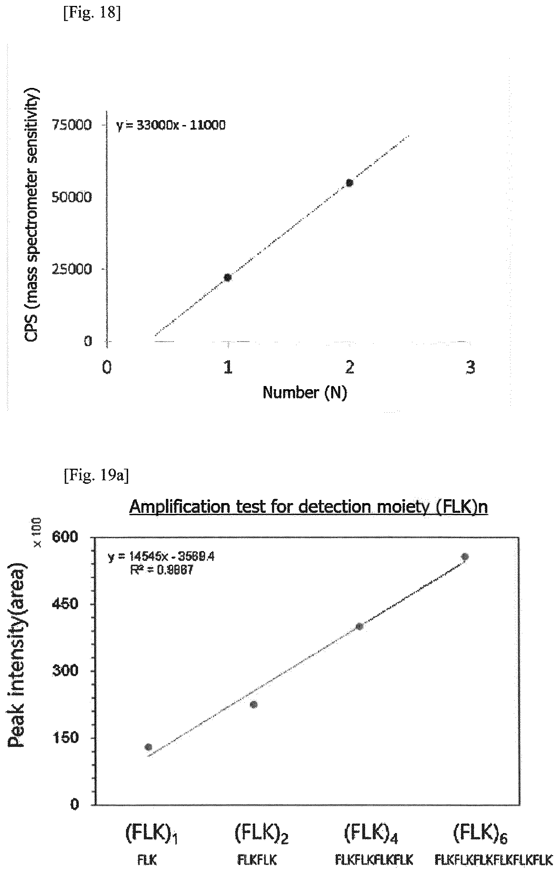

In order to confirm the amplification effect resulting from the repetition of the peptide sequence of the present disclosure, each of the peptide (LTLK) of SEQ ID NO: 652 in Table 20 above and the polymer (LTLKLTLK) composed of two repeats of the peptide was trypsinized, and then the intensity of the peak thereof was measured using a mass spectrometer. The results of the measurement are shown in FIGS. 16 and 17. The mass spectrometer sensitivity (CPS) as a function of the polymerization number was calculated and the results are shown in FIG 18.

As shown in FIGS. 16 and 17, compared to the intensity of the peak of the peptide (LTLK) of SEQ ID NO: 652, the intensity of the peak of the polymer (LTLKLTLK) composed of two repeats of the peptide was doubled. In addition, as shown in FIG. 18, it could be confirmed that when the peptide was repeated twice, the sensitivity was exactly doubled, suggesting that when the peptide is polymerized, the sensitivity increases as much as the polymerization number.

### [Experimental Example 3] (2) Examination of Sensitivity to Peptides Represented by M

In order to confirm the amplification effect resulting from the repetition of the peptide sequence of the present disclosure, the peptide fragment (FLK) of SEQ ID NO: 690 or a peptide composed of 2, 4 or 6 repeats of this fragment was produced. Then, each of these compounds was prepared at a concentration of 1 pM, and trypsin was added in an amount of 1:20 to 100 (w/w) with respect to the compound, followed by cleavage into FLK fragments at 37°C. The peptide fragments were dried completely and resuspended, and the mass-to-charge ratio of the FLK peptide fragment was input using a mass spectrometer (MRM mode). The area of the chromatogram was calculated, and the change in the peak intensity as a function of the polymerization number of the peptide fragment was measured, and the results of the measurement are shown in FIG. 19a.

As shown in FIG. 19a, it could be confirmed that, when the peptide fragment represented by FLK of SEQ ID NO: 690 is polymerized to form a polymer composed of repeats of the peptide fragment, the detection sensitivity increases in proportion to the polymerization number.

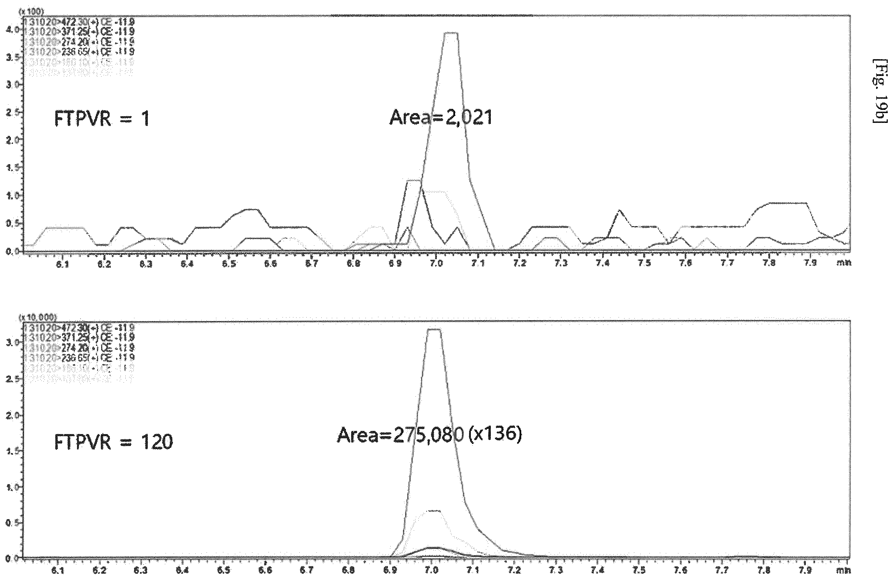

In addition, in order to measure the extent of increase in detection sensitivity when the peptide sequence of the present disclosure is repeated 100 times or more, a peptide consisting of a 120-repeat fragment of FTPVR was synthesized and the intensity changes of the peaks were measured using the same method as described above. As a result, the 120-repeat polymer showed an amplification factor of approximately 136 times compared to the FTPVR monomer, as seen in FIG. 19b, and it was found that the detection sensitivity of the target peptide of the present disclosure increased almost linearly or even more with respect to the number of repeats.

### [Experimental Example 4] (1) Evaluation of Diagnostic Ability of Detection Sensor

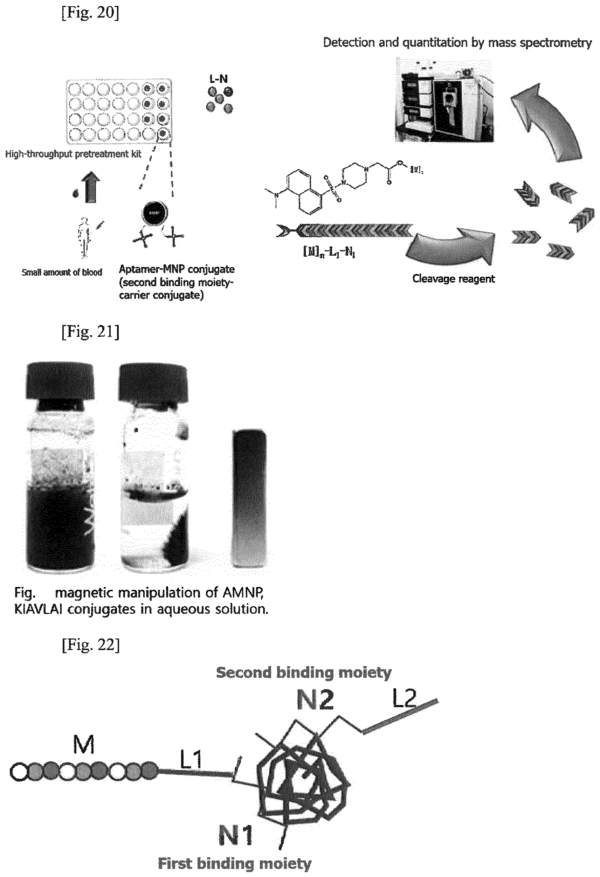

In order to evaluate the diagnostic ability of the detection sensor of the present disclosure, a protein detection test was performed as shown in FIG. 20. First, a target protein for cancer diagnosis was selected, and then an aptamer specific to the target protein was prepared, and an aptamer-MNP conjugate was produced in the same manner as in Preparation Example 4. Thereafter, each well was treated with the produced aptamer-MNP conjugate, and each well was treated and reacted with the blood isolated from a person in need of diagnosis. After the reaction was completed, each well was treated with a magnetic field, and a photograph of the blood after treatment is shown in FIG. 21.

As shown in FIG. 21, impurities other than the target protein that specifically binds to each aptamer could be removed from each well. Thereafter, reaction with each of proteins 1 to 4 through CuCl₂ treatment, removal of the remaining CuCl₂, treatment of each well with the complex compound represented by Chemical Formula 10, and removal of the remaining complex compound were sequentially performed, so that only the [M]ₙ-L₁-N₁-analyte-second binding moiety-carrier conjugate shown in FIG. 22 remained in each well. Then, each well was trypsinized, followed by filtration to obtain peptides.

### [Experimental Example 5] (2) Evaluation of Diagnostic Ability of Detection Sensor - Simultaneous Measurement of Multiple Samples

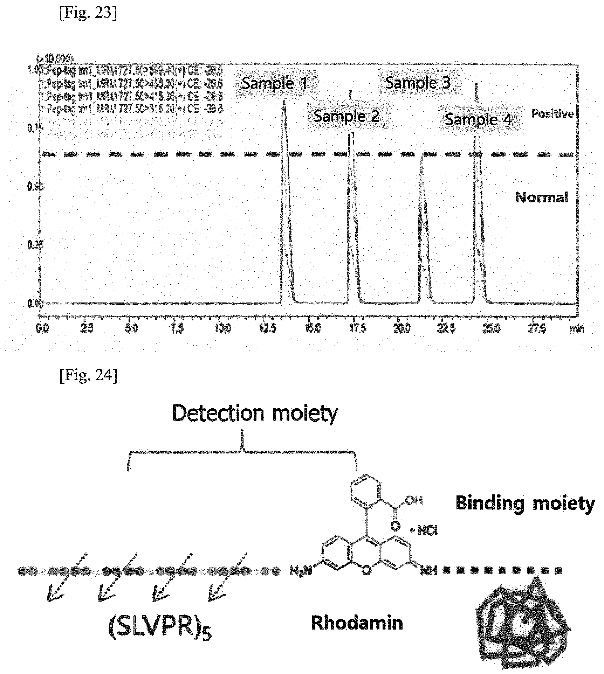

In order to confirm the diagnostic ability of the detection sensor of the present disclosure, simultaneous quantification of multiple samples was performed in the same manner as in Experimental Example 5, and the results are shown in FIG. 23.

Protein (albumin) present in human samples was selected. Accordingly, an aptamer specific to the protein was prepared, and an aptamer-MNP conjugate was produced in the same manner as in Preparation Example 4. Next, as in Experimental Example 5, each of wells 1 to 4 was treated with the produced aptamer-MNP conjugate, and then each well was treated and reacted with the blood isolated from a person in need of diagnosis. After the reaction was completed, each well was treated with a magnetic field as shown in FIG. 21. As a result, impurities other than the protein that bind specifically to the aptamer could be removed from each well. Thereafter, reaction with each of proteins 1 to 4 through CuCl₂ treatment, removal of the remaining CuCh, treatment of each well with the complex compound represented by Chemical Formula 10, and removal of the remaining complex compound were sequentially performed, so that only the [M]ₙ-L₁-N₁-analyte-second binding moiety-carrier conjugate shown in FIG. 23 remained in each well. M having different sequences were applied to the samples, respectively. Then, each well was trypsinized, followed by filtration to obtain peptides. As a result of analyzing the obtained peptides by a mass spectrometer, the polymer of the detection sensor treated into well 1 was composed of a peptide having a retention time (RT) of 14 minutes, the polymer of the detection sensor treated into well 2 was composed of a peptide having a retention time (RT) of 17.5 minutes, the polymer of the detection sensor treated into well 3 was composed of a peptide having a retention time (RT) of 21.5 minutes, and the polymer of the detection sensor treated into well 4 was composed of a peptide having a retention time (RT) of 24.5 minutes. It could be seen that, for samples 1, 2 and 4, the expression levels of the proteins exceeded the normal reference value, but for sample 3, the expression level of the protein was normal, suggesting that the detection sensor has excellent ability to simultaneously detect an analyte in biological samples with high sensitivity even in simultaneous measurement of multiple samples.

### [Experimental Example 6] (2) Evaluation of Diagnostic Ability of Detection Sensor

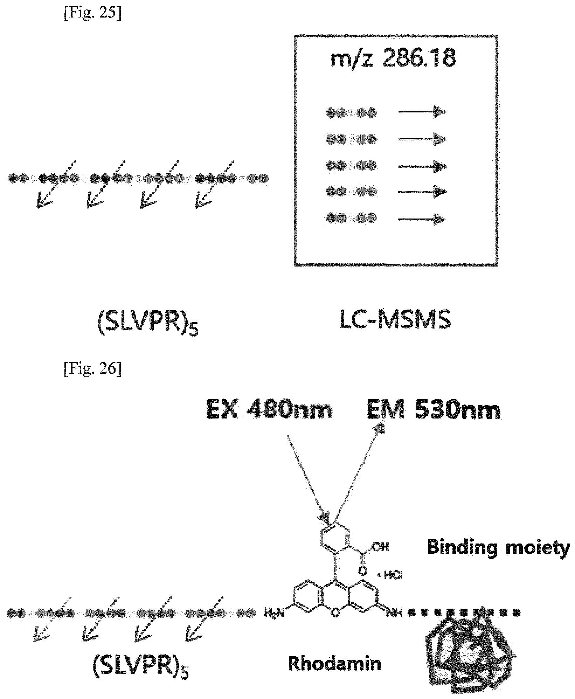

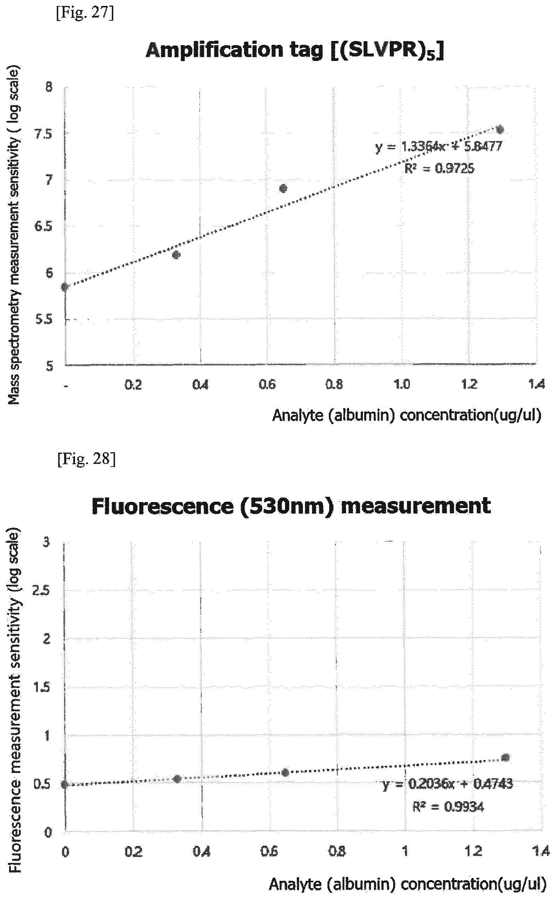

In order to evaluate the diagnostic ability of the detection sensor of the present disclosure, albumin was prepared as an analyte and then prepared at concentrations of 0, 0.33 µg/µl, 0.65 µg/µl and 1.3 µg/µl. Thereafter, for the detection of albumin, a complex compound consisting of an albumin-specific peptide (CB3GA)-rhodamine-(SLVPR (SEQ ID NO: 689))₅ having the structure shown in FIG. 24 was produced. Thereafter, the complex compound was allowed to react with albumin in a ratio of 3 to 6 equivalents, and then an unreacted portion of the compound was removed. Thereafter, as shown in FIG. 25, the (SLVPR)₅ peptide compound was cleaved into SLVPR fragments by treatment with trypsin, and the change in sensitivity as a function of the concentration of the analyte was measured using a mass spectrometer, and the results are shown in FIG. 27. Meanwhile, for comparison of the diagnostic ability of the detection sensor of the present disclosure, the fluorescence intensity of rhodamine was measured as shown in FIG. 26 before trypsin treatment, and the results are shown in FIG. 28.

As shown in FIGS. 27 and 28, it could be confirmed that, when the peptide polymer ((SLVPR)s) composed of 5 repeats of the SLVPR peptide fragment was used for albumin detection, the amplification effect could be produced as the peptide polymer was cleaved into 5 SLVPR peptide fragments due to trypsin treatment, and in particular, the sensitivity increased more than 6.5 times compared to that in the fluorescence measurement method.

As described above, it was confirmed through the Examples of the present disclosure that the detection sensor of the present disclosure could detect the analyte with high sensitivity through amplification, and simultaneous detection was also possible through the production of peptides having various sequences.

## Claims

1. A composition for detecting or measuring analytes by mass spectrometry comprising a complex compound represented by Formula 1:
[Formula 1] [M]ₙ-L₁-N₁
wherein
n is an integer ranging from 2 to 100000;
M is a repeatable unit compound selected from the group consisting of amino acids, amino acid analogs, peptides, peptide analogs, monosaccharides, oligosaccharides and polysaccharides;
L₁ is either a direct bond between M and N₁ or a linker;
N₁ is a first binding moiety that binds to the analyte; and
the bond between adjacent M and M is cleaved by a catalyst, so that the M is detected or measured when the analytes are detected or measured.

2. The composition of claim 1, wherein M has a mass-to-charge ratio (m/z) of 30 to 3,000.

3. The composition of claim 1, wherein M is represented by Formula 2:
[Formula 2] (X₁X₂...Xₘ)
wherein
m is an integer ranging from 1 to 100000; and
X₁ to Xₘ are each independently selected from the group consisting of an amino acid, amino acid analog, peptide, peptide analog, monosaccharide, oligosaccharide and polysaccharide.

4. The composition of claim 3, wherein X₁ or Xₘ is isoleucine, lysine, serine, arginine or threonine.

5. The composition of claim 1, wherein the first binding moiety comprises at least one selected from the group consisting of a probe, an antisense nucleotide, an antibody, an oligopeptide, a ligand, PNA (peptide nucleic acid) and an aptamer, which bind to the analyte.

6. The composition of claim 1, wherein the first binding moiety comprises at least one selected from the group consisting of Chemical Formulas 1 to 5: 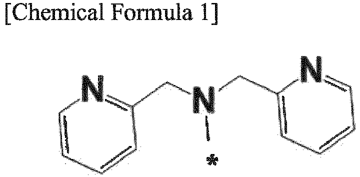 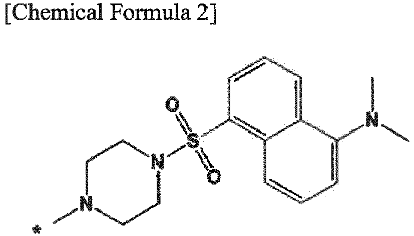 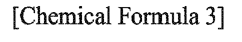 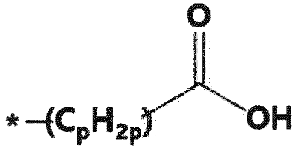 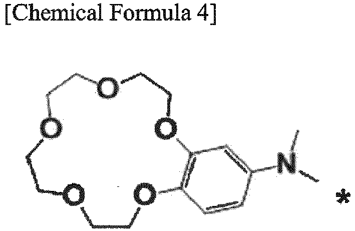
[Chemical Formula 5] **S=C=N- ***
wherein
p is an integer ranging from 7 to 20, and
* is a portion linked to [M]ₙ or L₁.

7. The composition of claim 1, wherein the linker comprises at least one selected from the group consisting of Chemical Formulas 6 to 8:
[Chemical Formula 6] *-C_{q}H_{2q}-*
[Chemical Formula 7] *-C_{q}H_{2q}COO-*
[Chemical Formula 8] *-H₂NCOC_{q}H_{2q}S-*
wherein
q is an integer ranging from 1 to 5; and
* is a linking portion.

8. The composition of claim 1, comprising two or more different complex compounds represented by Formula 1.

9. A kit for detecting an analyte comprising the composition of any one of claims 1 to 8.

10. The kit of claim 9, wherein the kit further comprises a second binding moiety, an immobilization support, a carrier, biotin, a washing solution or a reaction solution.

11. The kit of claim 10, wherein the kit comprises two or more different second binding moieties.

12. The kit of claim 10, wherein the second binding moiety comprises at least one selected from the group consisting of probe, antisense nucleotide, an antibody, an oligopeptide, a ligand, PNA (peptide nucleic acid) and an aptamer.

13. The kit of claim 10, wherein the reaction solution comprises at least one metal salt selected from the group consisting of CuCl₂, Cu(NO₃)₂, CoCl₂, Co(NO₃)₂, Zn(NO₃)₂ and ZnCl₂.

14. A method for analyzing an analyte, the method comprising a reaction step of allowing the analyte to react with the composition for detecting or measuring an analyte comprising the complex compound represented by Formula 1; and
a detection step of detecting or measuring M in the complex compound of the composition:
[Formula 1] [M]ₙ-L₁-N₁
wherein
n is an integer ranging from 2 to 100000;
M is a repeatable unit compound selected from the group consisting of amino acids, amino acid analogs, peptides, peptide analogs, monosaccharides, oligosaccharides and polysaccharides;
L₁ is either a direct bond between M and N₁ or a linker;
N₁ is a first binding moiety that binds to the analyte.

15. The method of claim 14, wherein the analyte is present in a biological sample isolated from a subject of interest.

16. The method of claim 15, further comprising immbolization step of immobilizing the analyte by bringing the analyte into contact with a second binding moiety.

17. The method of claim 16, wherein the second binding moiety comprises at least one selected from consisting of a probe, an antisense nucleotide, an antibody, an oligopeptide, a ligand, PNA (peptide nucleic acid) and an aptamer, which bind specifically to the analyte.

18. The method of claim 17, wherein the second binding moiety is bound to an immobilization support, a carrier or biotin to form a second binding moiety-immobilization support conjugate or second binding moiety-carrier conjugate.

19. The method of claim 14, further comprising a cleavage step of cleaving [M]ₙ in the complex compound into units M, after the reaction step.

20. The method of claim 19, wherein the cleaving [M]ₙ into the units M in the cleavage step is performed by an enzyme or a synthetic catalyst.

21. The method of claim 20, wherein [M]ₙ in the complex compound is cleaved into n of units M in the cleavage step, so that detection or measurement sensitivity of M increases.

22. The method of claim 14, further comprising treating with a metal salt in the reacting step.
